# EUROPEAN PATENT SPECIFICATION

(11) **EP 2 766 494 B1**
(45) Date of publication and mention of the grant of the patent: **22.11.2017**
(21) Application number: 12773106.5
(22) Date of filing: 10.10.2012
(51) Int. Cl.: C12Q 1/68

(54) **FISH SELECTION FOR IMPROVED TRAITS BASED ON THE DETECTION OF SEQUENCE VARIATIONS IN GENES**
FISCHAUSWAHL FÜR VERBESSERTEN MERKMALE AUF BASIS DER ERKENNUNG VON SEQUENZVARIATIONEN IN GENEN
SÉLECTION DE POISSONS POUR LEURS TRAITS AMÉLIORÉS EN SE BASANT SUR LA DÉTECTION DE VARIATIONS DE SÉQUENCES DANS LES GÈNES

(30) Priority: 10.10.2011 GB 201117448
(43) Date of publication of application: 20.08.2014
(73) Proprietor: University Court of The University of St Andrews, St Andrews Fife KY16 9AJ (GB)
(72) Inventor: JOHNSTON, Ian Alistair, St Andrews KY16 8LB (GB); ASHTON, Thomas James, St Andrews KY16 8LB (GB)
(74) Representative: Gibbs, Richard
(86) International application number: PCT/GB2012/052509
(87) International publication number: WO 2013/054107

(56) References cited:
- WO-A1-03/018845
- WO-A1-2009/064481
- WO-A1-2010/115275
- consortium Genomics Research on All Salmon (cGRASP): "cGRASP microarray", UVic - University of Victoria , 15 August 2009 (2009-08-15), XP002689345, Retrieved from the Internet: URL:http://web.uvic.ca/grasp/microarray/ar ray.html [retrieved on 2012-12-14]
- BAHUAUD D ET AL: "Fillet texture and protease activities in different families of farmed Atlantic salmon (Salmo salar L.)", AQUACULTURE, ELSEVIER, AMSTERDAM, NL, vol. 310, no. 1-2, 22 December 2010 (2010-12-22), pages 213-220, XP027536746, ISSN: 0044-8486 [retrieved on 2010-10-15]
- SALEM M ET AL: "Characterization of calpastatin gene in fish: Its potential role in muscle growth and fillet quality", COMPARATIVE BIOCHEMISTRY AND PHYSIOLOGY. PART B, BIOCHEMISTRYAND MOLECULAR BIOLOGY,, vol. 141, no. 4, 1 August 2005 (2005-08-01), pages 488-497, XP027701574, ELSEVIER,OXFORD, GB ISSN: 1096-4959 [retrieved on 2005-08-01]
- HAYES ET AL: "An extensive resource of single nucleotide polymorphism markers associated with Atlantic salmon (Salmo salar) expressed sequences", AQUACULTURE, vol. 265, no. 1-4, 5 April 2007 (2007-04-05), pages 82-90, XP022021603, ELSEVIER, AMSTERDAM, NL ISSN: 0044-8486, DOI: 10.1016/J.AQUACULTURE.2007.01.037
- W J TAO ET AL: "Associations between single nucleotide polymorphisms in candidate genes and growth rate in Arctic charr (Salvelinus alpinus L.)", HEREDITY, vol. 91, no. 1, 1 July 2003 (2003-07-01), pages 60-69, XP055047919, ISSN: 0018-067X, DOI: 10.1038/sj.hdy.6800281
- CHISTIAKOV D A ET AL: "Microsatellites and their genomic distribution, evolution, function and applications: A review with special reference to fish genetics", AQUACULTURE, vol. 255, no. 1-4, 31 May 2006 (2006-05-31), pages 1-29, XP027903121, ELSEVIER, AMSTERDAM, NL ISSN: 0044-8486 [retrieved on 2006-05-31]

## Description

### FIELD OF THE INVENTION

The present disclosure relates to genetic markers associated with flesh quality in fish. Accordingly, the disclosure provides nucleotide sequences as well as associated proteins/peptides to be exploited in methods of assessing fish flesh quality and identifying fish exhibiting improved or superior flesh quality, for breeding or sale.

### BACKGROUND OF THE INVENTION

Flavour, nutritional value, appearance and texture are the key components of flesh quality affecting consumer demand for farmed Atlantic salmon (*Salmo salar* L.) (Anderson 2000; Haard 1992; Robb *et al.* 2002; Sargent *et al.* 2001). In addition, gaping - the formation of tears and holes in the flesh (Lavéty *et al.* 1988) results in factory downgrading due to limited onward processability and reduces the visual appeal of a fillet, reducing saleability (Michie 2001).

Flesh softening of fish and other meats is influenced by degradation of muscle and connective tissue proteins by endogenous proteolytic enzymes including the calpain and cathepsin families (Bahuaud *et al.* 2010; Cheret *et al.* 2007; Delbarre-Ladrat *et al.* 2004; Godiksen *et al.* 2009). The cathepsins and calpains perform a very wide range of functions in vivo (Brix *et al.* 2008; Goll *et al.* 2003), including the degradation of muscle structural proteins and components of the connective tissue matrix during protein turnover. It is likely that degradation of connective tissue components by proteolytic enzymes could also influence gaping.

Fillet yield is a very important determinant of profitability in the salmon processing industry since processors buy whole salmon and sell edible flesh and its derivatives. Due to its direct link to profit, fillet yield is commonly monitored in the commercial environment and is defined as the quantity of saleable flesh expressed as a percentage of the whole gutted mass (Einen *et al.* 1998). There are a wide range of potential mechanisms influencing fillet yield in animals. The myogenic regulatory factor (MRF) family of transcription factors play key roles in the development of skeletal muscle (Yun and Wold 1996) and have been implicated in allelic influence over beef cattle carcass traits (Bhuiyan *et al.* 2009). Myostatin (MSTN) and its antagonist follistatin (FSTN) are also of considerable interest in muscle mass regulation as MSTN directly inhibits the proliferation of myogenic progenitor cells and its deficiency can lead to considerable increase in fish muscle bulk (Lee *et al.* 2009; Medeiros *et al.* 2009).

Marker assisted selection is a molecular approach to farmed animal or plant breeding that allows the identification of individuals with superior commercial traits in a non-lethal manner by screening for genotypes with known advantageous phenotypic effects. This can involve the use of single (or very few) loci within candidate genes (e.g. Schenkel *et al.* 2006), but this is relatively uncommon in aquaculture, which still largely relies on complex and less targeted multi-locus genotypes (e.g. Houston *et al.* 2008). Measurement of the degree of flesh softening is commonly performed using a mechanical texture analyser fitted with one of a range of probes (Torrissen *et al.* 2001). The Warner Bratzler shear method has been shown to be the most effective method for measuring firmness while tensile strength analysis provides a useful measure of the potential of a fillet to experience gaping (Ashton *et al.* 2010).

WO 2009/064481 (Sandia Corp.) relates to the identification of genetic markers in patients with high risk B-precursor acute lymphoblastic leukemia and diagnostic, prognostic and related methods using these genetic markers. CAPN3 is disclosed as one such marker.

The Consortium Genomics Research on All Salmon Project (cGRASP) from the University of Victoria discloses the use of cGRASP microarrays to detect SNPs in salmon genes (http://web.uvic.ca/grasp/microarray/array.html). A number of arrays are described and the Agilent 44K array contains probes for detecting SNPs in the CAPN3 gene.

WO 2010/115275 (Genome Atlantic) describes single nucleotide polymorphism (SNP) markers and associated methods for the genetic analysis of Atlantic cod (Gadus morhua), in particular associated methods for desirable production traits in Atlantic cod.

Bahuaud et al ("Fillet texture and protease activities in different families of famred Atlantic Salmon (Salmo salar L.) ", Aquaculture, vol. 310, no. 1-2, 22 December 2010, pages 213 to 220) describe a study into the fillet texture of Atlantic salmon. This document indicates that although differences in calpain activity between textural groups of families were observed, calpain activity was not significantly correlated to fillet texture and also that no significant correlation between calpastatin and fillet firmness was detected.

Salem et al describe a study of the calpastatin gene in rainbow trout ("Characterization of calpastatin gene in fish: Its potential role in muscle growth and fillet quality", Comparative Biochemistry and Physiology, Part B, Biochemistry and Molecular Biology, vol. 141, no. 4, 1 August 2005, pages 488-497).

Hayes et al describe that Atlantic salmon is a potentially useful model organism for studying the consequences of genome duplication and the availability of large numbers of SNPs for Atlantic salmon would be useful for both mapping of genes affecting quantitative traits, as well as a tool to establish the extent of duplication in the salmon genome (*"*An extensive resource of single nucleotide polymorphisms markers associated with Atlantic salmon (Salmon solar) expressed sequences", Aquaculture, vol. 265, no 1-4, 5 April 2007, pages 82-90).

WO 03/018845 (University New Hampshire) describes methods of selecting fish for breeding and optimum growth in conditions of various salinity. Fish are selected for growth in specific salinity based on their prolactin 1 genotype. A simple sequence repeat polymorphism (microsatellite) in the tilapia prolactin (prl 1) promoter is associated with differences in prl 1 expression and differences in growth in "salt-challenged" or "salt-effected" fishes.

W J Tao et al have described an investigation into SNPs associated with growth traits in Arctic charr (*"*Associations between single nucleotide polymorphisms in candidate genes and growth rate in Arctic charr (Salvelinus alpinus L.) " Heredity, vol. 91, no. 1, 1 July 2003, pages 60-69).

Chistiakov et al have reviewed the importance of microsatellite genetic markers in the field of aquaculture *("*Microsatellites and their genomic distribution, evolution, function and applications: A preview with special reference to fish genetics", vol. 255, no. 1-4, 31 May 2006, pages 1 to 29).

The present invention sets out to provide assays and methods for identifying single nucleotide polymorphisms in candidate genes associated with phenotypic effects on texture and fillet yield in farmed Atlantic salmon.

### SUMMARY OF THE INVENTION

Aspects of the present invention are described in the appended claims.

The present disclosure is based on the identification of a number of genetic markers that segregate (or are associated) with flesh quality in fish. In particular, the inventors have determined that variations in the sequence of several genes are associated with flesh quality in fish.

Furthermore, the inventors have determined alleles and (additive) combinations thereof that can be exploited in breeding programs to produce broodstock exhibiting improved flesh quality.

Accordingly, this disclosure provides details of genetic markers which may find application in the production or selection of fish with improved flesh quality.

The term "flesh" is used throughout the specification and should be understood as encompassing for example, parts of a fish designated for human consumption. As such, the term "flesh" embraces tissues such as skin and/or muscle. The term "flesh" may include specific flesh cuts such as, for example, fillets.

The present disclosure provides genetic markers associated with flesh quality and, for example, any increase in fillet size or weight (otherwise referred to as fillet "yield") may be regarded as indicative of improved flesh quality. One of skill in this field will appreciate that fillet yield may be defined as the quantity of saleable flesh expressed as a percentage of the whole gutted mass (of the fish).

Other flesh characteristics which may be indicative of flesh quality include, for example, flesh texture where a soft or "mushy" flesh is regarded as indicative of poor quality flesh and a firm and/or resilient flesh texture or flesh which exhibits a degree of tensile strength, may be regarded as indicative of improved flesh quality. Flesh quality may be determined by analysing aspects of flesh nutrition, flavour and/or appearance. In this regard any improvement in the nutritional value of the flesh, the taste and/or the development of an appealing or aesthetic colouration within the flesh, may be regarded as a marker of improved flesh quality. Additionally and/or alternatively the level of tissue gaping (characterised by the formation of holes and tears in the flesh) may be exploited as a means to quantify and/or assess the level of flesh quality as flesh which exhibits excessive gaping may be regarded as poor quality flesh.

In view of the above, the disclosure provides variant gene sequences associated with improvements in one or more aspects of fish flesh quality, said aspects being selected from the group consisting of:
(i) fillet yield;
(ii) flesh texture;
(iii) flesh nutrition;
(iv) appearance;
(v) flavour;
(v) gaping;
(vi) flesh tensile strength; and
(vii) firmness.

The term "improvement" is a relative term and one of skill would appreciate that any "improvement" in the quality of the flesh of a fish may be assessed relative to the quality of the flesh of a control fish having a known, graded or predetermined flesh quality. By way of example, the flesh quality in any given fish may be assessed relative to an average "flesh quality" value as determined from an assessment of flesh quality across a population; in this way, it may be possible to identify those with superior characteristics. An improvement in the quality of the flesh of a fish may be assessed relative to an "average" quality of flesh as determined in an unselected population of fish

As used herein, the term "fish" relates to all fresh water and/or sea water fish (including fish which inhabit both sea and fresh water habitats). Additionally, the term "fish" includes both wild and farmed species. In particular, the term "fish" encompasses fish for human consumption. The "fish" encompassed within the scope of this invention are species within the family Salmonidae, including those within the genus *Salmo.* The invention provides variant (gene) sequences associated with improvements in one or more markers of flesh quality in *Salmo salar* (the Atlantic salmon).

One of skill will appreciate that the terms "variant sequence" or "variant gene sequence" as used herein may encompass nucleic acid or amino acid sequences which differ by one or more nucleic acid(s)/amino acid(s) from a corresponding wild type or reference nucleic acid/amino acid sequence. A reference or wild type sequence may be obtained from a fish which exhibits typical (or normal) tissue quality. In other cases, reference nucleic acid and/or amino acid sequences may be those submitted to recognised depositories and issued accession numbers. A number of deposited sequences are referred to as reference sequences in this specification. It should be understood that a variant nucleic acid or amino acid sequence may comprise one or more nucleic acid/amino acid additions, deletions, substitutions and/or inversions. The nucleic acid sequence variations described in this invention may take the form of one or more polymorphism(s), where, for example, one or more nucleotides of a sequence are substituted for nucleotides not present in the corresponding wild-type or reference sequence. One of skill will appreciate that polymorphisms occurring at a single nucleotide position may be referred to as "single nucleotide polymorphisms" or "SNPs" and that one or more SNPs may be present in any given sequence. A "variant" nucleic acid sequence may result from the occurrence of one or more mutations within the sequence. Again, as one of skill will appreciate, a mutated nucleic acid sequence may comprise one or more nucleotide inversions, additions, deletions and/or substitutions.

One of skill will appreciate that any variation in a nucleic acid and/or amino acid sequence may result in modulated nucleic acid/amino acid expression, function and/or activity. By way of example, where a nucleic acid sequence encodes a enzyme, a variation in the nucleic acid sequence may modulate (i.e. increase or decrease) the level of expression of the nucleic acid - this, in turn, may lead to a corresponding modulation in the level of expression of the translated protein. Additionally or alternatively, a nucleic acid sequence variation which alters the amino acid sequence of the translated protein, may modulate the function and/or activity (for example enzymatic activity) of the translated protein.

As such, the variant sequences described herein may exhibit modulated expression, function and/or activity - i.e. an increase or decrease in expression, function and/or activity relative to the expression, function and/or activity of a control sequence (for example a wild type or reference sequence)).

Each of the variant sequences and genetic loci identified as being associated with flesh quality in fish are discussed in more detail below.

The disclosure concerns the finding that variations in the calpain 1 gene (CAPN1a) are associated with flesh quality in fish (for example Atlantic salmon). As such, the invention relates to variant CAPN1a sequences which differ from reference CAPN1a sequences by one or more nucleotides of the nucleic acid sequence and/or by one or more amino acid residues of the translated protein sequence (i.e. that encoded by the CAPN1a nucleic acid sequence). An exemplary reference sequence is deposited under the accession number BT059271.

Typically, the variant CAPN1a nucleic acid sequences comprise one or more nucleotide variations within the coding regions and/or those regions which are untranslated (referred to as the "UTR"). The variant CAPN1a sequences described herein may comprise one or more nucleotide variations within the coding region of the CAPN1a gene.

The CAPN1a gene encodes a calpain 1 protease belonging to the calcium-dependent, non-lysosomal cysteine protease family. As such, and without wishing to be bound by theory, it is suggested that calpain 1 is associated with flesh quality in fish through its role in the degradation of muscle tissue and components of the connective tissue matrix. One of skill will appreciate that any increase in the level of protease activity exhibited by calpain 1 (and occurring as a result of the presence of one or more variations within the nucleic acid/amino acid sequence), may result in a decrease in flesh quality. In contrast, a decrease in the protease activity exhibited by calpain 1 in fish flesh, may result in improved flesh quality. Again, without wishing to be bound by any particular theory, it is suggested that variations in the CAPN1a amino acid sequence and/or a nucleic acid sequence encoding the same, may be associated with (or causative of) modulated protease activity which in turn leads to variations in flesh quality in fish.

The variant CAPN1a sequences described herein may comprise a SNP at position 220 of the nucleic acid sequence. The SNP at position 561 may comprise a nucleotide substitution where the cytosine (C) nucleobase present at this position in the wild-type or reference sequence is replaced with a thymine (T) nucleobase; this SNP may be designated 561 C>T. This SNP is a non-synonymous SNP as it also affects a change in the primary amino acid sequence encoded by the CAPN1a gene. Specifically, the 561 C>T SNP affects a change at residue 39 of the CAPN1a amino acid sequence where the serine residue present in the wild type or reference sequence, is substituted for a leucine residue; this amino acid modification may be designated as Ser39Leu.

In view of the above, this invention provides a variant CAPN1a nucleic acid sequence comprising a 561 C>T SNP and variant CAPN1a amino acid sequences comprising a Ser39Leu alteration (a result of the 561C>T SNP).

SEQ ID NOS: 1 and 2 below highlight the position (bold and underlined residue) of the 561C>T SNP in the CAPN1a nucleic acid sequence (SEQ ID NO: 1) and the Ser39Leu alteration in the amino acid sequence (SEQ ID NO: 2)
**SEQ ID NO: 1 (BT059271): CAPN1a 561 C>T Ser39Leu**
**SEQ ID NO: 2: (BT059271): CAPN1a Ser39Leu**

Additionally, the disclosure relates to variations in myoblast determination protein 1b (MYOD1b) which may be associated with flesh quality in fish (for example Atlantic salmon). As such, the invention provides variant MYOD1b sequences which differ from reference MYOD1b sequences by one or more nucleotides of the nucleic acid sequence (encoded by the *MYOD1b* gene) and/or by one or more amino acid residues of the translated protein sequence (i.e. that encoded by the *MYOD1b* nucleic acid sequence). An exemplary reference sequence is deposited under the accession number AJ557149 (It should be noted that the deposited sequence is referred to as MYOD2 - Macqueen & Johnston, PLos ONE, February 2008 (3) explain MYOD nomenclature and in Table 1, show that MYOD1b is the suggested designation for MYOD2 - the designation MYOD1b is used herein)

Typically, the variant MYOD1b nucleic acid sequences of this invention comprise one or more nucleotide variations within the coding regions and/or those regions which are untranslated (referred to as the "UTR"). The variant MYOD1b sequences described herein may comprise one or more nucleotide variations within the coding region of the *MYOD1b* gene.

The *MYOD1b* gene encodes a protein involved in muscle differentiation. As such, and without wishing to be bound by theory, it is suggested that any modulation in the activity or function of the MYOD1b protein, may have an effect upon muscle development in fish. This, in turn, may have an effect of flesh quality - in particular, the texture, appearance and/or tensile strength of the muscle component of fish flesh. As such, it is suggested that variations in the *MYOD1b* gene sequence and/or the translated MYOD1b protein, may have an effect upon the function and/or activity of the MYOD1b protein.

The variant MYOD1b sequences described herein may comprise a SNP at position 829 of the nucleic acid sequence. The SNP at position 829 may comprise a nucleotide substitution where the guanine (G) nucleobase present at this position in the wild-type or reference sequence is replaced with an adenine nucleobase; this SNP may be designated 829G>A. This SNP is a non-synonymous SNP as it also affects a change in the primary amino acid sequence encoded by the *MYOD1b* gene. Specifically, the 829G>A SNP affects a change at residue 243 of the MYOD1b amino acid sequence where the cysteine residue present at this position in the wild type or reference sequence, is substituted for a tyrosine residue; this amino acid modification may be designated as Cys243Tyr.

In view of the above, this invention provides a variant MYOD1b nucleic acid sequence comprising a 829G>A SNP and a variant MYOD1b amino acid sequence comprising a Cys243Tyr alteration (a result of 829G>A SNP).

SEQ ID NOS: 3 and 4 below highlight the 829G>A SNP in the MYOD1b nucleic acid sequence (SEQ ID NO: 3) and the Cys243Tyr alteration in the amino acid sequence (SEQ ID NO: 4)
**SEQ ID NO: 3 (AJ557149): MYOD1b 829 G>A**
**SEQ ID NO: 4 (AJ557149): (Cys243Tyr)**

This disclosure also relates to variant myogenic regulatory factor 5 (MYF5) which may be associated with flesh quality in fish (for example Atlantic salmon). As such, the invention provides variant MYF5 sequences which differ from reference MYF5 sequences by one or more nucleotides of the nucleic acid sequence (encoded by the *Myf5* gene) and/or by one or more amino acid residues of the translated protein sequence (i.e. that encoded by the *Myf5* nucleic acid sequence). An exemplary reference sequence is deposited under the accession number DQ452070.

Typically, the variant MYF5 nucleic acid sequences of this invention comprise one or more nucleotide variations within the coding regions and/or those regions which are untranslated (referred to as the "UTR. The variant MYF5 sequences described herein may comprise one or more nucleotide variations within the coding region of the *Myf5* gene.

The *Myf5* gene encodes a protein involved myogenic differentiation and myogenesis. As such, and without wishing to be bound by theory, it is suggested that any modulation in the activity or function of the MYF5 protein, may have an effect upon muscle development in fish. This, in turn, may have an effect of flesh quality - in particular, the texture, appearance and tensile strength of the muscle component of fish flesh. As such, it is suggested that variations in the *Myf5* gene sequence and/or the translated MYF5 protein, may have an effect upon the function and/or activity of the MYF5 protein.

The variant MYF5 sequences described herein may comprise a di-nucleotide SNP at positions 578 and 579 of the nucleic acid sequence. The SNPs at these positions may comprise nucleotide substitutions in which the adenine (A) and cytosine (C) nucleobases present at positions 578 and 579 respectively are replaced with guanine (G) and thymine (T) nucleobases; this SNP may be designated 578-9AC>GT. This SNP is a non-synonymous SNP as it also affects a change in the primary amino acid sequence encoded by the *Myf5* gene. Specifically, the 578-9 AC>GT SNP affects a change at residue 193 of the MYF5 amino acid sequence where the asparagine residue at this position in the wild type or reference sequence, is substituted for an serine residue; this amino acid modification may be designated as Asn193Ser.

In view of the above, this invention provides a variant MYF5 nucleic acid sequence comprising a 578-9 AC>GT SNP and a variant MYF5 amino acid sequence comprising an Asn193Ser alteration (a result of the 578-9 AC>GT SNP).

SEQ ID NOS: 5 and 6 below highlight the 578-9 AC>GT SNP in the MYF5 nucleic acid sequence (SEQ ID NO: 5) and the Asn193Ser alteration in the amino acid sequence (SEQ ID NO: 6)
**SEQ ID NO: 5 (DQ452070 coding region only): MYF5 578-9 AC>GT**
**SEQ ID NO: 6 (DQ452070 coding region only): MYF5 Asn193Ser**

The disclosure also concerns the finding that variations in the calpastatin 2 (CAST2) gene, are associated with improved tissue characteristics in fish. The invention relates to CAST2 variants, wherein the sequence variation(s) occur within the coding region of the gene.

The CAST2 gene encodes a member of the calcium-dependent cysteine protease inhibitors and, without wishing to be bound by theory, may be associated with tissue quality in fish through its role as a proteolytic enzyme inhibitor which influences the degradation of tissues, including, for example, muscle and connective tissues.

The variant CAST2 sequence provided by this invention may comprise a SNP at position 134 of the nucleotide sequence. The SNP at this position may comprise a nucleotide substitution in which the guanine (G) present at position 134 is substituted for a thymine (T) nucleobases. This SNP may be designated 134 G>T SNP. Additionally, this SNP is a non-synonymous SNP as it also affects a change in the primary amino acid sequence encoded by the *CAST2* gene. Specifically, the 134 G>T SNP affects a change at residue 45 of the encoded calpastatin 2 protein. A variant CAST2 sequence of this invention may comprise one or more SNPs which alters the Glycine residue present at residue 45 in a wild type CAST2 primary sequence, replacing it with a valine residue - a CAST2 gene comprising such polymorphisms may be designated "CAST2 Gly45Val".

SEQ ID NOS: 7 and 8 below highlight the 134 G>T SNP in the CAST2 nucleic acid sequence (SEQ ID NO: 7) and the Gly45Val alteration in the amino acid sequence (SEQ ID NO: 8)
**SEQ ID NO: 7:** (JX489501, **coding region only) CAST2 134 G>T**
**SEQ ID NO: 8:** (JX489501, **coding region only) CAST2 Gly45Val**

Additionally, this disclosure provides variant calpain 11 (CAPNs11) sequences which have been recognised as associated with flesh quality in fish - including the Atlantic Salmon. As such, the invention provides variant CAPN11 sequences which differ from reference CAPN11 sequences by one or more nucleotides of the nucleic acid sequence (encoded by the *Capn11* gene) and/or by one or more amino acid residues of the translated protein (CAPN11) sequence (encoded by the *Capn11* nucleic acid sequence). An exemplary reference sequence is deposited under the accession number CX355265.

Typically, the variant CAPN11 nucleic acid sequences of this invention comprise one or more nucleotide variations within the parts designated as non-coding regions or "untranslated regions" (UTR). The variant CAPN11 nucleic acid sequences of this invention may comprise one or more nucleotide variations within the non-coding (i.e. untranslated) regions of the *Capn11* gene.

The *Capn*11 gene encodes a calpain 11 protesase belonging to the calcium-dependent, non-lysosomal cysteine protease family. As such, and without wishing to be bound by theory, it is suggested that, like calpain 1 above, calpain 11 is associated with flesh quality in fish through its role in the degradation of muscle tissue and components of the connective tissue matrix. One of skill will appreciate that any increase in the level of protease activity exhibited by calpain 11 may result in a decrease in flesh quality (due to increased levels of protein degradation). In contrast, any decrease in calpain 11 activity, may result in increased fish flesh quality. Without wishing to be bound by theory, the inventors hypothesise that variations in the non-coding sequences of the *Capn11* gene may affect regulatory regions which impact upon the function of the gene. By way of example, the variant *Capn11* sequences described herein may have an effect on RNA stability.

The variant CAPN11 sequences described herein may comprise a 3' UTR nucleotide SNP at position 611 of the *Capn11* sequence. The SNP may comprise a nucleotide substitution in which the cytosine (C) present at position 611 is replaced an adenine (A) nucleobase; this SNP may be designated 611 C>A.

In view of the above, this invention provides a variant CAPN11 nucleic acid sequence comprising a 3' UTR SNP characterised as a 611 C>A substitution.

SEQ ID NO: 9 below highlights the 611 C>A SNP in the CAPN11 3' UTR nucleic acid sequence
**SEQ ID NO: 9 (CX355265 This is an EST): Capn11 611C>A in 3'UTR**

Additionally, this disclosure provides variant calpain 3 (CAPN3) sequences which have been recognised as associated with flesh quality in fish - including the Atlantic Salmon. As such, the invention provides variant CAPN3 sequences which differ from reference CAPN3 sequences by one or more nucleotides of the nucleic acid sequence (encoded by the *Capn3* gene) and/or by one or more amino acid residues of the translated protein (CAPN3) sequence (encoded by the *Capn3* nucleic acid sequence). An exemplary reference sequence is deposited under the accession number BT058958.

The variant CAPN3 sequence provided by this invention may comprise a SNP at position 1701 of the nucleotide sequence. The SNP at this position may comprise a nucleotide substitution in which the cytosine (C) present at position 1701 is substituted for a guanine (G) nucleobase. This SNP may be designated 1701 C>G SNP. Additionally, this SNP is a non-synonymous SNP as it also affects a change in the primary amino acid sequence encoded by the *CAPN3* gene. Specifically, the 1701 C>G SNP affects a change at residue 541 of the encoded calpain 3 protein. A variant CAPN3 sequence of this invention may comprises one or more SNPs which alter the threonine residue present at residue 541 in a wild type CAPN3 primary sequence, replacing it with an arginine residue - a CAPN3 gene comprising such polymorphisms may be designated "CAPN3 Thr541Argl".

The *Capn*3 gene encodes a calpain protesase belonging to the calcium-dependent, non-lysosomal cysteine protease family. As such, and without wishing to be bound by theory, it is suggested that, like calpain 1 above, calpain 3 is associated with flesh quality in fish through its role in the degradation of muscle tissue and components of the connective tissue matrix. One of skill will appreciate that any increase in the level of protease activity exhibited by calpain 3 may result in a decrease in flesh quality (due to increased levels of protein degradation). In contrast, any decrease in calpain 3 activity, may result in increased fish flesh quality. Without wishing to be bound by theory, the inventors hypothesise that variations in the non-coding sequences of the *Capn11* gene may affect regulatory regions which impact upon the function of the gene.

SEQ ID NOs: 10 & 11 below highlight the 1701 C>G SNP in the CAPN3 nucleic acid sequence (SEQ ID NO: 10) and the Thr541ARG alteration in the amino acid sequence (SEQ ID NO: 11)
**SEQ ID NO: 10 (BT058958) CAPN3 1701C>G**
**SEQ ID NO:11 (BT058958 coding region only) CAPN3 Thr451Arg**

The present invention further provides variants of the expressed sequence tag (EST) deposited on GenBank under accession number DY714774; variants of this sequence have been recognised as associated with flesh quality in fish - including the Atlantic Salmon.

The variant DY714774 sequence provided by this invention may comprise a SNP at position 251 of the nucleotide sequence. The SNP at this position may comprise a nucleotide substitution in which the cytosine (C) present at position 251 is substituted for a thymine (T) nucleobases. This SNP may be designated 251 C>T SNP. The identity of this EST sequence (DY714774) is currently unknown. This polymorphic locus was experimentally determined to be associated with flesh quality traits.

SEQ ID No:12 below highlights the 251C>T SNP in DY714774
**SEQ ID NO:12 (DY714774, unidentified gene, EST) DY714774 251C>T**
*Residue at position 251 may be "C"

Additionally, the invention provides variants of the *Salmo salar* genomic contig sequence deposited on GenBank under accession number AGKD01012360.; variants of these sequences have been recognised as associated with flesh quality in fish - including the Atlantic Salmon.

The variant AGKD01012360 sequence provided by this invention may comprise a SNP at position 520 of the nucleotide sequence. The SNP at this position may comprise a nucleotide substitution in which the cytosine (C) present at position 520 is substituted for an adenine (A) nucleobases. This SNP may be designated 520 C>A SNP. The identity of the sequence containing the SNP (AGKD01012360) is currently unknown. This polymorphic locus was experimentally determined to be associated with flesh quality traits.

SEQ ID NO:13 below highlights the 520C>A SNP in AGKD01012360
**SEQ ID NO 13 (AGKD01012360, unidentified genomic contig, 0-1000bp) AGKD01012360 520C>A**
*The C>A SNP at 520 shows an "A" residue in the reference sequence but the data presented herein shows that the "A" residue is the minor allele. Consequently, a AGKD01012360 reference sequence may have a "C" residue at position 520.

For convenience, the various variant gene sequences described in this invention shall be referred to as "flesh quality associated genes" whereas the proteins of this invention (i.e. proteins encoded by the (variant) flesh quality associated genes) shall be referred to as "flesh quality associated proteins".

According to an aspect of the invention there is provided a method of selecting fish from species within the family *Salmonidae* with one or more improved traits according to claim 1. A fourth aspect of this disclosure provides a method of selecting fish with superior commercial traits such as, for example, improved flesh quality, said method comprising the steps of identifying sequence variations within one or more of the nucleic acid or amino acid sequences encoding proteins selected from the group consisting of:
(i) CAPN1a;
(ii) MYOD1b;
(iii) MYF5;
(iv) CAST2;
(v) CAPN11;
(vi) CAPN3;
(vii) DY714774; and
(viii) AGKD01012360
wherein variations in the nucleic acid or amino acid sequences encoding any of (i)-(viii) above, are associated with improved flesh quality in fish.

The method may comprise identifying variants in the nucleic acid or amino acid sequence encoding one of (i)-(viii) - wherein, the identification of a variation in a nucleic acid or amino acid sequence encoding any of proteins (i)-(viii) above, is associated with improved flesh quality in fish. As such, the disclosure provides methods in which any combination of variant CAPN1a, MYOD1b, MYF5, CAST2, CAPN11, CAPN3, DY714774 and/or AGKD01012360 sequences may be used as a means to screen or select for fish with improved flesh quality.

The method provided by the fourth aspect of this disclosure may comprise or consist essentially/consist of, the step of detecting or identifying a variant of the CAPN1a nucleic acid and/or amino acid sequence and a variant of the MYOD1b nucleic acid and/or amino acid sequence, wherein the detection or identification of variant CAPN1a and MYOD1b sequences or variant CAPN1a and MYOD1b proteins, may indicate that the fish exhibit improved flesh quality. In particular, fish with variant CAPN1a and MYOD1b sequences may exhibit improvements in fillet yield. As stated above, it should be understood that improvements in flesh quality (such as improvements in fillet yield) may be assessed relative to a control flesh. A "control flesh" may be a flesh sample which is considered representative of an average flesh quality or may be represent the "average" quality of flesh in an unselected population of fish. Fish in an unselected population may lack one or more of the sequence variants described herein.

The method provided by the fourth aspect of this disclosure may comprise, or consist of, the step of identifying (in any order) (i) a variant of the CAPN1a nucleic acid and/or amino acid sequence (ii) a variant of the MYOD1b nucleic acid and/or amino acid sequence and (iii) a variant of the MYF5 nucleic acid and/or amino acid sequence, wherein the detection or identification of variant CAPN1a, MYOD1b and MYF5 sequences (i.e. variant nucleic acid and/or amino acid sequences), may indicate that the fish exhibit improved flesh quality. In particular, fish with variant CAPN1a, MYOD1b and/or MYF5 sequences may exhibit improvements in tensile strength. As stated above, it should be understood that improvements in flesh quality (such as improvements in fillet yield, tensile strength and the like) may be assessed relative to, for example, a population average.

It should be understood that each of the methods described herein may utilise samples obtained or derived from, or provided by a fish to be tested. A sample may comprise nucleic acid and/or peptides and/or proteins and assays for detecting and/or identifying variant nucleic acid and/or amino acid sequences may be performed thereon. A sample may take the form of a tissue biopsy (comprising cells and other material), a scraping or swab. Furthermore, the sample may comprise a fluid, for example a biological fluid, such as whole blood, serum, plasma, semen, urine, immunological fluids (lymph etc.), tissue and/or glandular secretions and/or saliva. The term "sample" may further include samples provided by or obtained from embryonic (for example unfertilised/fertilised) egg) material. When the methods described herein are applied to embryonic material, the methods may be used to screen embryos/developing embryos for beneficial (and commercially important) traits.

The methods described herein may comprise the step of identifying (in a sample as described above) one or more sequence variations selected from the groups consisting of:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a: Ser39Leu;
(ix) CAPN11611 C>A;
(x) CAPN3: 1701 C>G;
(xi) CAPN3: Thr541Arg;
(xii) DY714774 251 C>T; and
(xiii) AGKD01012360 520 C>A.
wherein the identification of one or more of the variations listed as (i)-(xiii) above indicates that the sample was provided by (or obtained from) a fish with improved flesh quality. One of skill will appreciate that variants (i), (iii), (v), (vii), (ix) and (x) are nucleic acid variants of the *Myf5, MYOD1b, CAST2, GAPN1a, CAPN11 and CAPN3* genes respectively. In contrast, variants (ii), (iv), (vi), (viii) and (xi) are amino acid variants of the Myf5, MYOD1b, CAST2, CAPN1a and CAPN3 proteins respectively.

The methods described herein may comprise, or consist essentially/consist of, the step of identifying (in a sample as described above) the sequence variations given as (i) ((a) and/or (b)) and (ii) ((a) and/or (b)) below:
(i)
   (a) MYOD1b: 829 G>A; or
   (b) MYOD1b: Cys243Tyr;
   and
(ii)
   (a) CAPN1a: 561C>T; or
   (b) CAPN1a: Ser39Leu;
wherein detection of nucleic acid/amino acid variations (i) and (ii) in a sample, indicates that the sample was provided by or obtained from, a fish with improved flesh quality in fish. The sample may be provided by (or obtained from) *Salmo salar* and the results can be used to identify *Salmo salar* individuals with improved flesh quality.

The methods described herein may comprise or consist essentially/consist of the step of identifying (in a sample as described above) the sequence variations given as (i) ((a) and/or (b)), (ii) ((a) and/or (b)) and (iii) ((a) and/or (b)) below:
(i)
   (a) MYOD1b: 829 G>A; or
   (b) MYOD1b: Cys243Tyr;
(ii)
   (a) CAPN1a: 561C>T; or
   (b) CAPN1a: Ser39Leu;
   and
(iii)
   (a) MYF5 578-9 AC>GT;
   (b) MYF5 Asn193Ser;
wherein detection of sequence variations (i), (ii) and (iii) in a sample, indicates that the sample was provided by or obtained from, a fish with improved flesh quality in fish. The sample may be provided by (or obtained from) *Salmo salar* and the results can be used to identify *Salmo salar* individuals with improved flesh quality.

According to aspects of the invention there are provided methods of selecting fish from species within the family *Salmonidae* having a greater yield of flesh according to claims 2 and 8. The disclosure further provides methods which may be used to select fish with improved yield (i.e. flesh yield), said method comprising the steps of identifying (in a sample as described above) one or more of the sequence variations given as (i), (ii), (iii), (iv) and (v):
(i)
   (a) CAST2 234 G>T
   (b) CAST2 Gly45Val
   and
   (a) MYOD1b: 829 G>A; or
   (b) MYOD1b: Cys243Tyr;
   or
(ii)
   (a) CAST2 234 G>T
   (b) CAST2 Gly45Val
   and
   (a) CAPN11 611 C>A
   or
(iii)
   (a) MYOD1b: 829 G>A; or
   (b) MYOD1b: Cys243Tyr;
   and
   (a) CAPN11 611 C>A
   or
(iv)
   (a) CAPN3 1701 C>G
   (b) CAPN3 Thr541Arg
   and
   (a) CAPN11 611 C>A
   or
(v)
   (a) MYOD1b: 829 G>A; or
   (b) MYOD1b: Cys243Tyr;
   and
   (a) CAPN3 1701 C>G
   (b) CAPN3 Thr541Arg
wherein detection of sequence variations (i), (ii), (iii), (iv) or and/or (v) in a sample, indicates that the sample was provided by or obtained from, a fish with (potential) improved yield. The sample may be provided by (or obtained from) *Salmo salar* and the results can be used to identify *Salmo salar* individuals with (potential) improved yield.

According to aspects of the invention there are provided methods of selecting fish from species within the family *Salmonidae* having flesh exhibiting improved tensile strength according to claims 5 and 9 The disclosure further provides methods which may be used to select fish with flesh exhibiting improved tensile strength (mJ), said method comprising the steps of identifying (in a sample as described above) one or more of the sequence variations given as (i) and (ii):
(i)
   (a) CAPN11 611 C>A
   and
   (a) DY714774 (TAG4083) 251 C>T
   or
(ii)
   (a) AGKD01012360 (TAG49325) 520 C>A
   and
   (a) DY714774 (TAG4083) 251 C>T
wherein detection of sequence variations (i) and/or (ii) in a sample, indicates that the sample was provided by or obtained from, a fish with flesh exhibiting improved tensile strength. The sample may be provided by (or obtained from) *Salmo salar* and the results can be used to identify *Salmo salar* individuals with (potential) flesh exhibiting improved tensile strength.

According to aspects of the invention there are provided methods of selecting fish from species within the family *Salmonidae* having flesh exhibiting improved firmness according to claims 6 and 10. The disclosure further provides methods which may be used to select fish with flesh exhibiting improved firmness (mJ), said method comprising the steps of identifying (in a sample as described above) the sequence variations:
(i)
   (a) AGKD01012360 (TAG49325) 520 C>A
   and
(ii)
   (a) CAPN3 1701 C>G
   (a) CAPN3 Thr541Arg
wherein detection of these sequence variations in a sample, indicates that the sample was provided by or obtained from, a fish with flesh exhibiting improved firmness. The sample may be provided by (or obtained from) *Salmo salar* and the results can be used to identify *Salmo salar* individuals with flesh exhibiting improved firmness.

Surprisingly, the inventors have shown that while variant Cast2, Capn11 and Myodlb gene/protein sequences are each individually (significantly) associated with fillet yield, all two-locus combinations of these genes were additively associated with fillet yield. All three loci could be used to create a three-locus genotype of even greater influence.

Moreover, the inventors have determined that Capnla, DY714774 (tag4083) and AGKD01012360 (tag49325) also appear to have an additive association with tensile strength. Without wishing to be bound by theory, it is possible that they may be able to exert a greater combined influence over this trait.

As stated above, it is emphasised that the sequences of nucleic acids and/or amino acids in any of the samples described herein may be compared to one or more reference or control sequences.

One of skill will appreciate that variations in nucleic acid and/or amino acid sequences can lead to variations in gene/protein expression, function and/or activity. For example, a variation in wild-type gene or protein sequence may be characterised as a partial or total loss-of function mutation, whereby the sequence variation (at least partially) abrogates or modulates the function, level of expression of general activity of a gene and/or protein. As such, in addition to providing variant nucleic acid and amino acid sequences, the present disclosure may further relate to associations between a level of expression, activity and/or function of one or more the flesh quality associated genes/proteins described herein, with flesh quality in fish.

According to an aspect of the invention there is provided a method of selecting fish from species within the family *Salmonidae* with improved traits according to claim 14. The disclosure may provide a method of selecting fish with superior commercial traits such as, for example, improved flesh quality, said method comprising the steps of identifying in a sample provided by a fish to be tested, a level of expression, function and/or activity of one or more of the proteins selected from the group consisting of:
(i) CAPN1a;
(ii) MYOD1b;
(iii) MYF5;
(ix) CAST2; and
(x) CAPN11
(xi) CAPN3
(xii) DY714774
(xiii) AGKD01012360
wherein any modulation in the level of protein expression, function and/or activity may indicate that the sample was provided by a fish exhibiting improved flesh quality. The level protein expression, function and/or activity may be compared to the level of expression, function and/or activity of the corresponding protein(s) identified in a reference or control sample (or control system). It should be understood that a reference or control sample may be provided by a fish with known (for example wild-type, typical or average) flesh quality. As with those methods which focus on identifying variant sequences associated with improved flesh quality, the method may comprise or consist essentially/consist of identifying levels of expression, function and/or activity of any combination (for example 1, 2, 3, 4, 5, 6, 7 or all 8) of the flesh quality associated proteins described herein.

The methods which may be used to detect variant nucleic acid and/or amino acid sequences are well known to one of skill in this field and may include, for example, polymerase chain reaction (PCR) based techniques utilising, for example genomic DNA as template or reverse transcriptase (RT)-PCR (see below) techniques in combination with real-time PCR (otherwise known as quantitative PCR). TaqMan assays using fluorescently labelled probes to bind different alleles may be exploited. One of skill will readily understand that the principal of the TaqMan system relies on the 5'-3' exonuclease activity of Taq polymerase to cleave a dual-labelled probe during hybridization to the complimentary target sequence and flourophore based detection. Other useful techniques may include restriction fragment length polymorphism analysis (RFLP) and/or hybridisation techniques using probes and/or primers designed to hybridise under conditions of high, medium and/or low stringency, to sequences associated with (for example adjacent to or spanning the loci harbouring the sequence variations described herein). Suitable probes and/or primers are described below. Further information on such techniques may be found in Molecular Cloning: A Laboratory Manual (Third Edition) By Sambrook, MacCallum & Russell, Pub. CSHL; ISBN 978-087969577-4 - incorporated herein by reference.

For example, PCR techniques useful in the detection of variant nucleic acid sequences may require the use of short oligonucleotide primers designed to hybridise to sequences proximal to (for example 3' and 5' (upstream or downstream)) of a nucleic acid sequence of interest - for example a sequence potentially harbouring a variant sequence. Once oligonucleotides have been hybridised to a nucleic acid sequence, the nucleic acid sequence between the primers is enzymatically amplified via the PCR. The amplified nucleic acid may then be sequenced to determine whether or not it comprises a variant sequence. Additionally, or alternatively, the amplified nucleic acid may be contacted with one or more restriction enzymes - this technique is particularly useful if a variant nucleic acid sequence is known either to remove a particular restriction site or create a restriction site. The presence or absence of a variant nucleic acid sequence may be detected via analysis of the resulting restriction fragment length polymorphism (RFLP) profile. When analysing RFLP profiles, the results may be compared to standard or control profiles obtained by contacting nucleic acid sequences obtained from fish exhibiting standard flesh quality (or harbouring wild type (reference) nuclei acid and/or amino acid sequences), with the same restriction enzymes.

In addition to the above, altered electophoretic mobility may be used to detect alterations in nucleic acid sequences. For example, small sequence deletions and insertions may be visualised by high resolution gel electrophoresis - nucleic acid sequences with different sequences migrating through agarose gels (denaturing or non-denaturing and/or gradient gels) at different speeds/rates.

In addition to identifying sequence variations associated with flesh quality in fish, the present disclosure may further encompass associations between the levels of expression of any of the genes/proteins described herein and flesh quality in fish. In this regard, a number of techniques may be used to determine levels of gene and/or protein expression.

One of skill will appreciate that relative levels of mRNA expression may be used as a means of determining a level of expression, function and/or activity of a particular gene (such as, for example, a gene associated with improved flesh quality). By way of example, any modulation (i.e. an increase or decrease) in the amount of mRNA encoding any of the genes described herein, may also be associated with improved flesh quality.

More specifically, real time-PCR may used to determine a level of gene expression. Typically, and in order to quantify the level of expression of a particular nucleic acid sequence, RT-PCR may be used to reverse transcribe the relevant mRNA to complementary DNA (cDNA). Preferably, the reverse transcriptase protocol may use primers designed to specifically amplify an mRNA sequence of interest (in this case mRNA derived from the genes associated with improved flesh quality). Thereafter, PCR may be used to amplify the cDNA generated by reverse transcription. Typically, the cDNA is amplified using primers designed to specifically hybridise with a certain sequence and the nucleotides used for PCR may be labelled with fluorescent or radiolabelled compounds.

One of skill in the art will be familiar with the technique of using labelled nucleotides to allow quantification of the amount of DNA produced during a PCR. Briefly, and by way of example, the amount of labelled amplified nucleic acid may be determined by monitoring the amount of incorporated labelled nucleotide during the cycling of the PCR.

Further information regarding the PCR based techniques described herein may be found in, for example, PCR Primer: A Laboratory Manual, Second Edition Edited by Carl W. Dieffenbach & Gabriela S. Dveksler: Cold Spring Harbour Laboratory Press and Molecular Cloning: A Laboratory Manual by Joseph Sambrook & David Russell: Cold Spring Harbour Laboratory Press.

Other techniques that may be used to determine a level of gene expression in a sample, include, for example, northern and/or Southern blot techniques. A northern blot may be used to determine the amount of a particular mRNA present in a sample and as such, could be used to determine the amount of expression of any of the "flesh quality" genes described herein. Briefly, total or messenger (m)RNA may be extracted from any of the samples described above using techniques known to the skilled artisan. The extracted RNA may then be subjected to electrophoresis. A nucleic acid probe, designed to hybridise (i.e. complementary to) an RNA sequence of interest - in this case the mRNA derived from any of the "flesh quality" genes described herein, may then be used to detect and quantify the amount of a particular mRNA present in a sample.

Additionally, or alternatively, a level of gene expression may be identified by way of microarray analysis. Such a method would involve the use of a DNA microarray which comprises nucleic acid derived from wild type or reference genes corresponding to the various "flesh quality" genes described herein. To identify a level of gene expression, one of skill in the art may extract the nucleic acid, preferably the mRNA, from a sample and subject it to an amplification protocol such as, RT-PCR to generate cDNA. Preferably, primers specific for a certain mRNA sequence - in this case a sequence derived from a mRNA of any of the flesh quality genes of this invention, may be used.

The amplified gene cDNA may be subjected to a further amplification step, optionally in the presence of labelled nucleotides (as described above). Thereafter, the optionally labelled amplified cDNA may be contacted with the microarray under conditions which permit binding with the DNA of the microarray. In this way, it may be possible to identify a level of gene expression (by qualitative/quantitative analysis of the "amount" of binding).

In addition, other techniques such as deep sequencing and/or pyrosequencing may be used to detect variant gene sequences such as, for example, the variant sequences described herein. Further information on these techniques may be found in "Applications of next-generation sequencing technologies in functional genomics", Olena Morozovaa and Marco A. Marra, Genomics Volume 92, Issue 5, November 2008, Pages 255-264 and "Pyrosequencing sheds light on DNA sequencing", Ronaghi, Genome Research, Vol. 11, 2001, pages 3-11.

Samples to be analysed may be probed for levels any of the proteins described herein. In this regard, one of skill would appreciate that immunological detection techniques such as, for example, enzyme linked immunosorbent assays (ELISAs) or Western blot and/or immunoblot techniques may be used. Such techniques may require the use of binding agents or antibodies, specific to, or selective for, one or more of the proteins described herein. Further information on such techniques may be found in Using Antibodies: A Laboratory Manual By Harlow & Lane, Pub. CSHL, ISBN 978-087969544-6 and Antibodies: A Laboratory Manual by Harlow & Lane, CSHL, ISBN 978-087969314-5 - both of which are incorporated herein by reference.

Binding agents (for example antibodies) having affinity/specificity/selectivity to/for any of the flesh quality associated proteins (or epitopes thereof), may be coated onto the surface of a suitable substrate (for example a microtitre plate). Thereafter, the coated substrate may be contacted with a sample to be tested for the presence or absence of a flesh quality associated protein. Binding between any flesh quality associated proteins present in the sample and the binding agents coated onto the surface of the substrate, may be detected by means of a secondary binding agent having specificity for a flesh quality associated protein. Secondary antibodies useful in the present invention may optionally be conjugated to moieties which permit them to be detected (referred to hereinafter as "detectable moieties"). For example, the secondary antibodies may be conjugated to an enzyme capable of reporting a level via a colourmetric chemiluminescent reaction. Such conjugated enzymes may include but are not limited to Horse Radish Peroxidase (HRP) and Alkaline Phosphatase (AlkP). Additionally, or alternatively, the secondary antibodies may be conjugated to a fluorescent molecule such as, for example a fluorophore, such as FITC, rhodamine or Texas Red. Other types of molecule which may be conjugated to binding agents include radiolabelled moieties.

Other techniques which exploit the use of agents capable of binding flesh quality associated proteins, for example antibodies, include, for example, techniques such as western blot or dot blot. A western blot may involve subjecting a sample to electrophoresis so as to separate or resolve the components, for example the proteinaceous components, of the sample. The resolved components/proteins may then be transferred to a substrate, such as nitrocellulose.

In order to identify any flesh quality associated proteins present in a sample, the substrate (for example nitrocellulose substrate) to which the resolved components and/or proteins have been transferred, may be contacted with a binding agent capable of binding flesh quality associated proteins under conditions which permit binding between any flesh quality associated proteins present in the sample (or transferred to the substrate) and the agents capable of binding the flesh quality associated proteins.

Advantageously, the agents capable of binding the flesh quality associated proteins may be conjugated to a detectable moiety.

Additionally, the substrate may be contacted with a further binding agent having affinity for the binding agent(s) capable of binding flesh quality associated proteins. Advantageously, the further binding agent may be conjugated to a detectable moiety.

Any of the samples described above may be used a source of flesh associated protein. Additionally or alternatively, the flesh quality associated protein may be isolated or purified from the sample, or produced in recombinant form.

In order to identify the levels of flesh quality associated protein present in a sample, one may compare the results of any of the immunological of molecular (i.e. PCR, RT-PCR) techniques described herein, with results obtained from the same procedures using control or reference samples obtained from subjects exhibiting wild-type (typical/normal) flesh quality. By way of example, a sample revealing either an increased or decreased level of flesh quality associated gene activity, expression or function and/or an increased or decreased level of flesh quality associated protein than that detected in a corresponding reference or control sample, may have been provided by (or obtained from) a fish exhibiting improved flesh quality.

The present invention also provides polynucleotide sequences comprising nucleotides which are complementary to nucleotide sequences (preferably contiguous sequences) adjacent to and/or comprising (or spanning), the variant sequences described herein. The polynucleotide sequences may be primer or probe sequences which may otherwise be referred to as oligonucleotides. The probe or primer oligonucleotides provided by this invention may comprise, for example 5-50, 6-40, 7-30, 8-20 nucleotides. Where the oligonucleotide has application as a probe, the
oligonucleotide may comprise a nucleotide complementary to a SNP (i.e. complementary to the minor allele). One of skill will appreciate that primer sequences comprising nucleotides complementary to sequences upstream and/or downstream of any of the SNPs described herein, may be used in PCR based techniques to amplify sections of nucleic acid comprising one or more SNP or SNP loci. Oligonucleotide sequences useful as probes or primers may be used to detect the presence or absence of certain SNP sequences in nucleic acid samples provided by subjects.

In a further aspect, the disclosure provides antibodies having affinity for or selective/specific for a polypeptide (or an epitope thereof) encoded by a variant sequence described herein. The antibodies described herein may have affinity for (or be selective/specific to/for) proteins (or epitopes thereof) comprising the following variations gene/protein sequences comprising the following variations: MYF5 Ser193Asn; MYOD1b: Cys243Tyr; CAST2: Gly45Val; or CAPN1a: Ser39Leu, CAPN3: Thr541Arg or proteins (or epitopes thereof) encoded by sequences comprising the following variations: MYF5 628 AC>GT; MYOD1b: 829 G>A; CAST2: 234G>T; CAPN1a: 220G>A , CAPN11 611C>A or CAPN3 1701 C>G.

Polyclonal and/or monoclonal antibodies are easily produced and/or purified using routine laboratory techniques. It should be understood that the term antibody also includes epitope binding fragments or derivatives such as, for example, single chain antibodies, diabodies, triabodies, minibodies and/or single domain antibodies. The term antibodies also encompasses Fab, (Fab)₂ and/or other epitope binding fragments.

A further aspect of the invention relates to kits for identifying and/or determining whether or not a variant flesh quality associated gene/protein is present or absent in a nucleic acid sample provided by a fish species from within the family *Salmonidae* according to claim 15. A kit according to this aspect of the invention may include one or more pairs of oligonucleotide primers useful for amplifying a nucleotide sequence of interest. For example, the nucleotide sequence of interest may comprise one or more nucleic acid sites known to harbour the specific variations provided by this invention. The kit may comprises a polymerizing agent, for example, a thermo-stable nucleic acid polymerase such as one disclosed in U.S. Pat. Nos. 4,889,818 or 6,077,664. Furthermore, the kit may comprises an elongation oligonucleotide that hybridizes to a sequence adjacent or proximal to a site potentially harbouring a variant sequence provided by this invention. Where the kit includes an elongation oligonucleotide, it may also include chain elongating nucleotides, such as dATP, dTTP, dGTP, dCTP, and dITP and/or analogs thereof. In addition, the kit provided by this aspect of this invention may optionally include terminating nucleotides such as, for example, ddATP, ddTTP, ddGTP, ddCTP. The kit may include one or more oligonucleotide primer pairs, a polymerizing agent, chain elongating nucleotides, at least one elongation oligonucleotide, and one or more chain terminating nucleotides. Kits may also optionally include reaction and/or storage buffers, vials or other storage/reaction vessels, microtiter plates and instructions for use.

In a further aspect, the invention provides a method for identifying broodstock fish from species within the family *Salmonidae* according to claim 11. One of skill will appreciate that broodstock fish may be used in breeding programs to generate progeny fish which exhibit one or more of the improved traits (for example improved flesh yield, firmness and the like) described herein. A method of selecting broodstock fish may comprise,, consist essentially/consist of, the steps of identifying one or more of the flesh quality associated genes/proteins described herein and/or modulated expression, function and/or activity thereof, wherein fish identified as harbouring one or more of the flesh quality genes/proteins described herein, or modulated expression, function and/or activity thereof may be used as broodstock. As such, the methods provided by this aspect of the invention may be used in order to select fish for use in commercial farming or the like. Fish broodstock selected using the methods described herein are more likely to exhibit favourable commercial traits.

In should be noted that any of the methods described herein may be used either in isolation or together. Moreover one or more of the methods provided by this invention may be combined with one or more existing fish selection protocols. By way of example, the methods provided by this invention may be combined with existing protocols which facilitate the selection of fish exhibiting improved growth rates and/or disease resistance. For example, the methods of selecting broodstock described herein may be further supplemented with methods for selecting fish which are likely to exhibit a high growth rate or a degree of disease resistance.

### DETAILED DESCRIPTION

The present invention will now be described in detail with reference to the following figures which show:

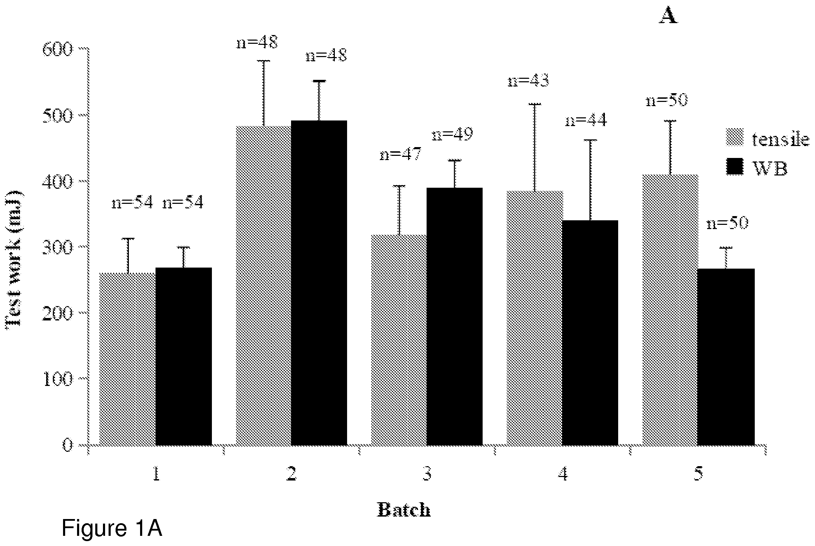

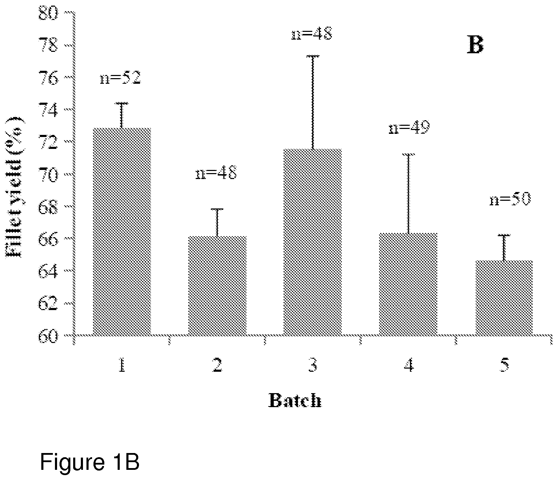

Figure 1: Mean values for individual fillet traits. Chart A shows tensile test and Warner Bratzler. Chart B shows fillet yield expressed as a percentage of the whole gutted weight. Error bars show standard deviation and numbers above the bars give the number of data-points after removal of outliers.

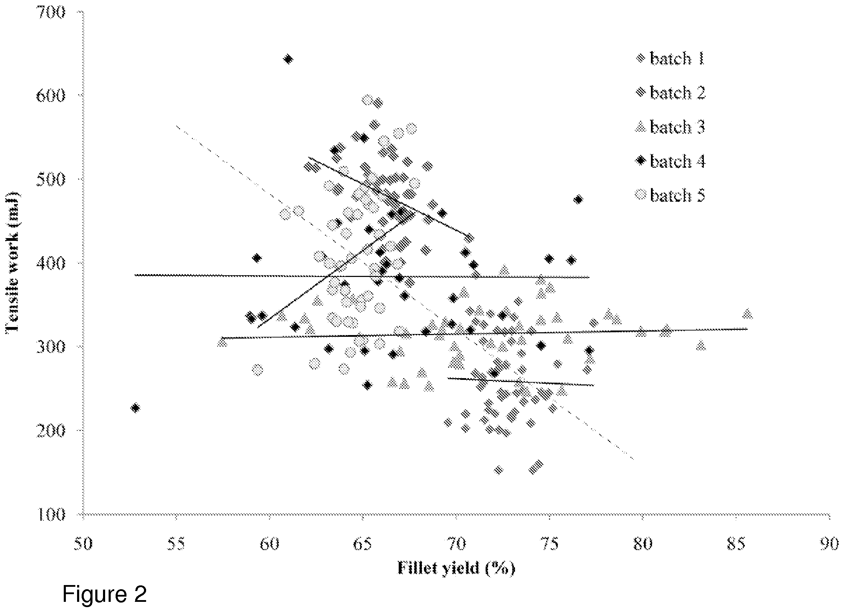

Figure 2: Relationships between significantly correlated traits. The chart shows tensile work against fillet yield where overall R = -0.496, P < 0.001. When resolved at batch level, the data shows no clear trend of relationship between the traits. Dashed red line indicates overall trendline and solid black lines are batch trendlines.

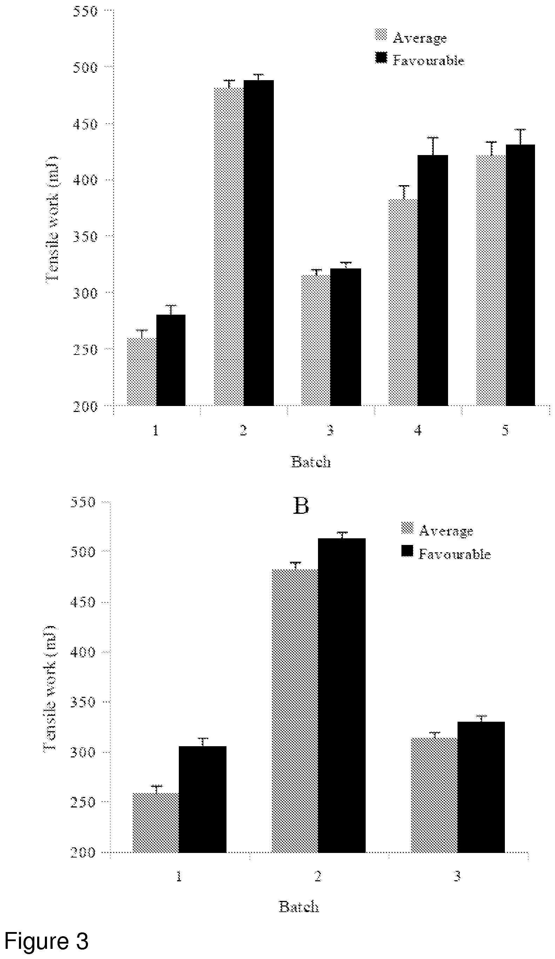

Figure 3: Effect of multi-locus genotypes on tensile work (mJ). A = combined favourable genotypes of CAPN1a (CC & AA) and MYOD1b (GG) compared to the batch averages. The combined favourable genotype occurred with 25.8% frequency (n = 57). B = combined favourable genotypes of CAPN1a, MYOD1b and MYF5 (GA). Batches 4 and 5 are not included as there were no occurrences of the favourable genotype in these batches. Combined favourable genotype occurred with 6% frequency overall (n = 15) Error bars = standard error.

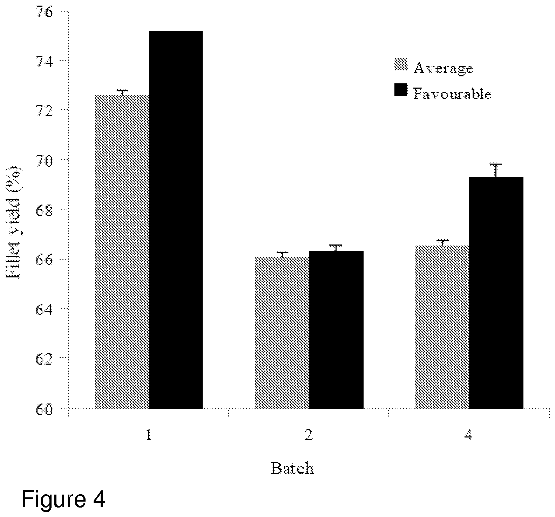

Figure 4: Effect of multi-locus genotype on fillet yield (%). Combined favourable genotype of MYOD1b (AA) and CAPN1a (CC) compared to batch averages. Favourable combined genotype occurred with 9.4% frequency (n = 12). Error bars = standard error.

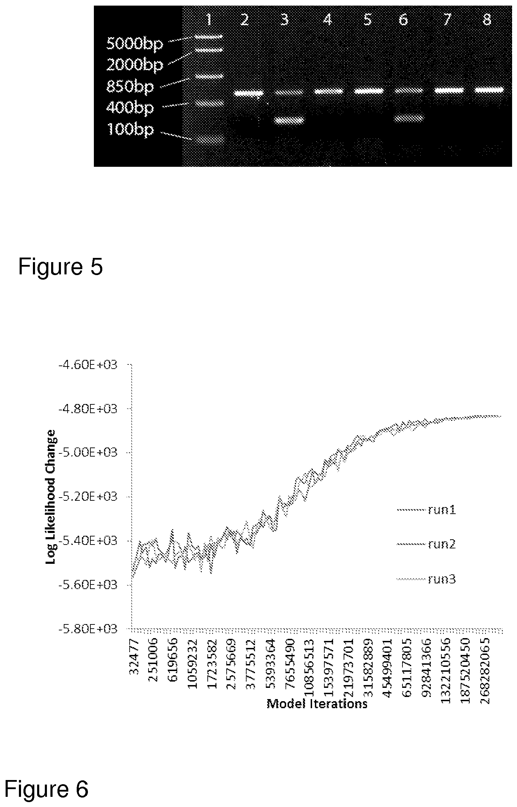

Figure 5: Visualisation of sex determination duplex PCR. In all cases the 550bp control band is present, whereas the 220bp sdY band is only present in males. Lane 1 = size marker, lanes 2, 4, 5, 7 & 8 = females, lanes 3 & 6 = males.
Figure 6: Comparison of log likelihood change over successive model iterations during three replicate runs of COLONY to infer siblings and paternal half siblings in 8 batches of Atlantic salmon.

### Materials and Methods

### Identification of candidate genes

Sequences of candidate genes with potential influence over flesh texture and yield were retrieved from publicly available fish genomes on Ensembl Genome Browser (http://www.ensembl.org/). These sequences were then BLAST searched against the nucleotide and EST databases of Atlantic salmon on GenBank, using tBLASTn. Table 1 shows the selected sequences that yielded interesting polymorphisms.

**Table 1.Details of polymorphic loci significantly associated with fillet traits. Sequencing primers = primers used to amplify the candidate gene for sequencing. cDNA PCR product size given in basepairs length. Primer melting temperature (Tm) given in °C. SNP (bp) = basepair substitution, SNP (aa) = amino acid substitution. MAF = minimum allele frequency across the 8 tested batches. Primer sequences in italics are for preliminary pre amplification, prior to Taqman assay.**

| Sequence | Type | Genbank | Source | Sequencing primers | T m | Product (cDNA) | SNP (bp) | SNP (aa) | MAF | Taqman primers | Taqman probes | |
|---|---|---|---|---|---|---|---|---|---|---|---|---|
| CAST2 | Protein coding | JX489501 | Solexa | aagttggttacggctgttgctg | 57 | 950bp | 134 G>T | 45 Gly>Val | 0.21 | *GCCTCCAATTAGAGACAACGAC* | | |
| | | | | ctagctcttgccacccttatttttg | 57 | | | | | *CTTTACCTTGATGGGAGCGG* | | |
| | | | | | | | | | | GAGGATTTGGCTGCCATTGATG | vic | TGCTGGCTTTTCC |
| | | | | | | | | | | GGTGGAGGTGGAGCAAAGTT | fam | CTGCTGTCTTTTCC |
| CAPN1a | Protein coding | BT059271 | Sanger | actacggccaggtgagaatg | 60 | 1028bp | 561 C>T | 39 Ser>Leu | 0.48 | AGAACCACAACGCAGTCAAGTT | vic | CCCTCAGCGACTCA |
| | | | | caggaagtcgctgaatgaca | 60 | | | | | GCGGCTCTGGAGGCA | fam | CCCTCAGCAACTCA |
| CAPN11 | 3'UTR | CX355265 | Solexa | ctgcagacttggaagatgagg | 60 | 700bp | 611 C>A | n/a | 0.23 | CTTGTCCCAGGAGACAGAACAG | vic | TGCCGGGCACTTG |
| | | | | caggcagaggacaataaggc | 60 | | | | | CAATAAGGCTAGGAAGTTCTTCATGGT | fam | CTGCCGGTCACTTG |
| MYF5 | Protein Coding | DQ452070 | | atggatgtcttctcccagtcc | 60 | 760bp | 578-9 AC>GT | 193 Asn>Ser | | GGTGCTGGTGCCTCCA | vic | |
| | | | | tcacaatacgtggtacacaggtc | 60 | | | | | GCGAGAAGGTAAGCATATCTCTGA | fam | |
| MYOD1b | Protein coding | AJ557149 | Sanger | cctgtcacctctgctgatga | 60 | 850bp | 829 G>A | 243 Cys>Tyr | 0.44 | CGCGTCCACTGTGTTATCAG | vic | TAGCCCCTGCTCTC |
| | | | | cttggtagatggggtcatgg | 60 | | | | | TCGCTCAGGATAGATCCCTCTTG | fam | TAGCCCCTACTCTC |
| CAPN3 | Protein coding | BT058958 | Solexa | acaagtcccatagctggacg | 59 | 1000bp | 1701 C>G | 541 Thr>Arg | 0.39 | TGTCTGTTCTCTTTCAGGGAAACC | vic | TTGGTCTGACGTAA |
| | | | | gagcaggactcctctgatgg | 59 | | | | | GGCTTTCCTTTCTTTGTCCTGTTTT | fam | TTGGTCTGACCTAA⁻ |
| Tag4083 | Unidentified | DY714774 | RAD | n/a | | n/a | 251 C>T | n/a | 0.40 | TGAGAGAGCAAACGGCATGTTAT | vic | |
| | | | | | | | | | | CATATTTTCCATGACATGTCAATGCTTCT | fay | |
| Tag49325 | Unidentified | AGKD01012360 | RAD | n/a | | n/a | 520 C>A | n/a | 0.28 | CCTTAATTTATAGCCCTGGCTGGTA | vic | ACACTGACGGCACT |
| | | | | | | | | | | AAACTAGTTTTGAATCCCACTCCTCTTC | fam | CACACTGACTGCAC |

Where no homologous sequence was returned, the BLAST search was performed against the NCBI EST database. Attempts were made to align ESTs and build longer contigs of gene sequence but occasionally this was not possible due to incomplete EST coverage and the existence of highly homologous paralogues of the gene. In this case ESTs were treated as separate gene sequences. Table 1 shows the selected gene sequences that yielded interesting polymorphisms and also indicates whether the sequences are known sequences, contigs built from EST coverage or single ESTs. A complete table of gene sequences studied and the primers used to amplify them is given in supplementary material. The method of sequencing (Sanger or Solexa) and Genbank accession numbers are also shown. Primer pairs for PCR were designed to amplify the selected sequences. The chosen oligonucleotides were between 18 and 25 bases in length and had melting temperature in the range of 57 - 61°C. NetPrimer software was used to assess potential primers for susceptibility to dimer and hairpin formation and specificity was checked against known sequences from Atlantic salmon using NCBI PrimerBlast. Where desired sequences were longer than 1.5kb, two pairs of primers were designed to amplify the target in fragments of around 1kb. In some cases separate primer pairs were also designed to amplify 3' UTR regions. In total 54 primer pairs were designed. These are given in Table 1 with oligonucleotide sequences, amplicon lengths and primer melting temperatures.

### Reference genetic material

DNA sequence polymorphisms were initially sought using a diverse pool of tissue samples sourced from 150 Atlantic salmon from Scottish farms. The majority of individuals came from numerous batches from five major salmon aquaculture companies (Lighthouse Caledonia, Scottish Seafarms, Mainstream, Marine Harvest) sampled at different time points during 2008. Fast muscle samples were dissected from fillets taken from the Macrae's processing factory production line (Fraserburgh, UK), after being stored at 0 - 4 °C for up to 4 days. Additional cDNA was obtained from approximately 200 individuals from Norwegian aquaculture sources and 140 individuals from Scottish aquaculture origin (Landcatch Natural Selection).

### Amplification of candidate genes

RNA was extracted using an RNeasy kit (Qiagen) according to the manufacturer's instructions. RNA was concentrated by ethanol precipitation and quantified using a

Nanodrop spectrophotometer. Only RNA with a ratio of absorbance at 260 nm - 280 nm between 1.8 and 2.1 and a ratio of absorbance at 260 nm - 230 nm above 2.0 was used for cDNA synthesis. The integrity of the RNA was confirmed by gel electrophoresis. Poor quality samples were abandoned and RNA extraction was repeated if possible. Genomic DNA was removed using the genomic DNA wipeout buffer included in the Quantitect reverse transcription kit (Qiagen). RNA was reverse transcribed into cDNA for 30 min at 42°C using a Quantitect reverse transcription kit according to the manufacturer's recommendations. PCR was performed using Taq DNA polymerase (Bioline) and the MgCl2 and 10x reaction buffer included with the Taq. The standard reaction was a 25µl volume and contained: 2.5µl 10x buffer, 1µl MgCl2, 0.5µl dNTPs (10mM), 18.9µl nuclease free water, 1µl 10µM forward + reverse primer mix, lul DNA template and 0.125µl Taq. The reaction was denatured at 95°C for 10 minutes before 35 cycles of 95°C denaturation 30s, variable annealing temperature 30s, 72°C extension. The extension time was calculated based on 1min for each 1kb of expected amplicon size. A final extension of 7mins at 72°C was used. The standard PCR conditions were adjusted empirically for each reaction, if necessary. PCR products were visualised by agarose gel electrophoresis and purified using a Qiaquick gel extraction kit (Qiagen).

### Sanger sequencing

The amplifications of each particular sequence from the various cDNA pools were combined, giving one sample for each sequence. The PCR products were cloned into a T/A pCR4-TOPO vector (Invitrogen) and transformed into chemically competent TOP10 *Escherichia coli* cells (Invitrogen). 96 colonies were picked for each sample and transferred into 96 well plates containing 200µl LB broth in each well, before incubation overnight at 37°C. Preliminary colony PCR was performed using 1µl overnight culture as a template and modified T3 and T7 primers. Limiting concentrations of dNTPs and primers were used, to prevent carryover into the sequencing reaction. Sequencing was performed using T3 and T7 sequencing primers with Big Dye terminator v3.1 sequencing chemistry (Applied Biosystems) by Genservice, University of Oxford. Sequences were quality checked, trimmed and aligned in CodonCode Aligner software (CodonCode Corp.). The resultant contigs were inspected manually for the existence of co-amplified paralogues, insertions/deletions, splice variants and SNPs.

### Trait database compilation

Sampling was carried out on one occasion at the The Seafood Company's Marsden Road factory (Grimsby, UK) and on four occasions at the Macrae factory (Fraserburgh, UK) during 2009-2010. Each sampling consisted of 50-54 fish from the same harvest batch (Table 2) and were therefore as similar as possible with respect to any commercial production variables (e.g. harvest stress, size, quality grade etc.). Batches 1-4 were normal aquaculture products, taken from the daily supply through the processing plants. Batch 5 was processed exclusively for this project and consisted of the F1 progeny of wild net-caught wild salmon of Scottish origin. Batch 5 was produced and farmed by North Uist Fisheries, North Uist, UK. Fillet yield was measured by subtracting the untrimmed fillet mass after automatic processing from the whole gutted carcass weight. The fillets were then trimmed of excess bone, fat, connective tissue and fins, according to the industry standard of preparation for downstream processing, before being weighed again. The resulting dataset contained a whole weight, a crude split weight and a trimmed saleable fillet weight. The whole process was performed by a team of trained and experienced filleting staff, using common tools and techniques, so as to best represent the actual fillet yield achievable in a factory environment. The instrumental texture analysis procedure consisted of firmness measurements by Warner Bratzler probe and tensile strength measurements, both using a mechanical texture analyser (TA.XTplus, Stable Microsystems). The texture analysis protocols were as previously described (Ashton *et al.* 2010). Small tissue samples for genomic DNA extraction were taken at the time of texture analysis (5 days post slaughter).

**Table 2. Details of sampling batches and trait details of 8 batches of farmed Atlantic salmon. Tensile strength, firmness and fillet yield are given with standard deviation in parentheses. Dams and Sires = number of individual female and male parents contributing to the batch. Full and Half sibs = the number of pairs of individuals within each batch determined to be full or half siblings. Batches 6-8 share a common ova source so are presented as a single batch.**

| Batch | Harvest | date *n* | Supplier | Egg source | Tensile (mJ) | Firmness (mJ) | Fillet yield (%) | Males (%) | Dams | Sires | Full sibs | Half sibs |
|---|---|---|---|---|---|---|---|---|---|---|---|---|
| 1 | 11/04/09 | 54 | Marine Harvest | Aquagen | 258.8(52.9) | 267.5(31.0) | 72.8(1.6) | 51 | 37 | 11 | 11 | 132 |
| 2 | 21/01/10 | 50 | Lighthouse Caledonian | Fanad | 489.6(61.1) | 482.4(99.1) | 66.1(1.7) | 32 | 31 | 9 | 20 | 97 |
| 3 | 04/03/10 | 50 | Sea Products | Lakeland | 317.5(41.6) | 389.3(74.1) | 71.5(5.8) | 42 | 37 | 15 | 21 | 38 |
| 4 | 26/03/10 | 50 | Coast Seafoods | Erfjord | 423.1(132.7) | 338.6(122.9) | 66.3(4.9) | 36 | 29 | 7 | 19 | 208 |
| 5 | 05/05/10 | 50 | N. Uist Fisheries | Wild salmon | 306.5(61.1) | 199.4(24.0) | 64.6(1.6) | 56 | 14 | 6 | 253 | 163 |
| 6 | 16/01/12 | 96 | TSSC | SalmoBreed | - | - | 69.4(1.3) | 75 | | | | |
| 7 | 23/01/12 | 96 | TSSC | SalmoBreed | - | - | 70.4(1.2) | 58 | 150 | 40 | 183 | 1019 |
| 8 | 27/01/12 | 76 | TSSC | SalmoBreed | - | - | 68.2(3.3) | 64 | | | | |

### SNP genotyping by Taqman assay

Polymorphic loci discovered by sequencing were investigated using BioEdit (Hall 1999) and DNA MAN (Lynnon Biosoft) software to manipulate reference sequences to determine whether SNPs were located within coding regions or untranslated regions (UTRs) and whether they were responsible for amino acid changes in the transcribed proteins (non-synonymous SNPs). Coding region SNPs that made no difference to the amino acid sequence (synonymous SNPs) were disregarded from further analysis. A minor allele occurrence of 8% was set as a requirement for inclusion of a particular SNP for further study. The frequency of an allele in the sequenced reads was not taken as a definitive indication of the actual frequency within the population due to the possibility of differential amplification and cloning efficiency of different transcripts or of the genetic material from individuals. Taqman genotyping assays were designed for those loci selected for further study. Prior to assay design, orthologous sequences were retrieved from Ensembl for all available fish species. Alignments were made of these sequences, including their intron/exon boundaries. This was used to predict the intron sites in the salmon genes of interest. Wherever possible, it was considered favourable to have at least one of the Taqman primer sites situated in an intron sequence. This served two purposes; firstly, it greatly reduced the likelihood of the primers annealing to an alternative paralogue and secondly, it reduced the risk of contamination from previously extracted plasmids, since the entirety of the sequence region involved in the assay would never have been cloned in a single plasmid. In the case of CAST2, the assay location was not close to any introns (>100bp) so a preliminary PCR reaction was designed, whereby primers were designed to span the assay region and bind in an intron sequence. The product of this PCR was then diluted 1:10,000 and used as the template for the taqman assay, again reducing the risk of poor paralogue specificity and plasmid contamination. Primers were designed and PCR performed as described in 3.3 and PCR products were cloned in a T/A pCR4-TOPO vector (Invitrogen) and transformed into chemically competent TOP10 *E. coli* cells (Invitrogen). Plasmids were extracted using a Qiaprep plasmid extraction kit (Qiagen) and sent for Sanger sequencing at the DNA Sequencing and Services laboratory at Dundee University. Taqman assays were ordered according to the primer and probe sequences specified in Table 1.

### Trait database compilation

Sampling was carried out on one occasion at the The Seafood Company's Marsden Road factory (Grimsby, UK) and on seven occasions at the Macrae factory (Livingston, UK) during 2009-2012 (Table 2). Each sampling consisted of fish from the same harvest batch and were therefore as similar as possible with respect to any commercial production variables (e.g. harvest stress, size, quality grade etc.). Fish were mechanically filleted on the automated production line before being hand-trimmed of excess bone, fat, connective tissue and fins, according to the industry standard of preparation for downstream processing. The resulting dataset contained whole weights and trimmed fillet weights. The process was performed by a team of trained and experienced filleting staff, using common commercial tools and techniques. One fillet from each fish in batches 1-5 was labelled and sent on ice to St Andrews University for texture analysis. The instrumental texture analysis procedure consisted of firmness measurements by Warner Bratzler probe and tensile strength measurements, both using a mechanical texture analyser (TA.XTplus, Stable Microsystems). The texture analysis protocols were as previously described (Ashton *et al.* 2010). All texture analysis was performed at 5 days post slaughter. Genomic DNA was extracted using a DNeasy Blood and tissue kit (Qiagen) from all fish sampled.

### RAD library sequencing

A pool of high tensile strength individuals was created by selecting the 10 fish from each of batches 1-5 that possessed the highest tensile work (mJ) values. A low tensile strength pool was created in the same way. DNA from the fish in each pool was combined after standardization to 5ng/µl, resulting in two pooled samples. Restriction amplified DNA (RAD) libraries were produced following the protocol described by Baird et al. (2008) and used for bulk segregant analysis of tensile-strength pools. The adapter-ligated libraries were sequenced using a Solexa Genome Analyser (GA2X, Illumina) at The Genepool laboratory, University of Edinburgh, Edinburgh, UK. Reads were aligned and filtered with a quality requirement of 20 (<1% error rate). Tags identified as containing SNPs were filtered with a minimum coverage of 400 reads and minimum of 200 reads in each pool. Tags that passed these criteria were aligned by BLAST to the draft Atlantic salmon genome assembly ASM23337vl (Gen bank id GCA_000233375.1, Davidson et al., 2010) and the nr and EST databases of known sequences.

### Sex determination

Morphological determination of sex of fish used in this study was not possible, since all were obtained in gutted form, and were sexually immature. Sex was determined following the PCR method described by Yano et al. (2012) in rainbow trout, *Onchorhynchus mykiss.* The oligonucleotide primers for the rainbow trout sex determining locus, sdY, were used in duplex with primers designed to amplify a 550bp portion of the coding region of an insulin-like growth factor (IGF1) paralogue in Atlantic salmon (forward TTGCGGATGCTGCCTTGTC, reverse GGAAGGTAGATCGTGAAGCAGG). These primers were used as an internal control and were selected principally on the basis of their product size, annealing temperature and predicted compatibility with the sdY primers, based on analysis of dimer formation using NetPrimer software (Premier Biosoft). The optimised reaction was tested using purified DNA from 20 individuals of known sex from a breeding programme (10 male and 10 female). In all cases the males displayed both the 220bp sdY band and the 550bp IGF1 band, whereas all females displayed only the 550bp IGF1 band

### Relatedness

Microsatellite markers were used to infer frequencies of full and half siblings and to predict parent identity. Seven microsatellite sequences from Atlantic salmon were chosen based on their high degree of polymorphism (King *et al.* 2001; Norris *et al.* 1999; O'Reilly *et al.* 1996). Fluorescent labelled primers were designed to amplify the sequences. PCR amplicons were run through a capillary sequencer after addition of a GS500 size standard at Source Bioscience, Oxford.

Genotypes were called using Peak Scanner v1.0 software (Applied Biosystems) and raw fragment data was binned into allele sizes using SPSS statistics 19 (SPSS Inc.). COLONY software was used to infer sibling and half-sibling dyads and to calculate parentage within the whole population (Wang 2012). The model was set to consider females as monogamous, and was informed of all impossible sibling pairs, i.e. fish from different hatchery batches. The maximum likelihood model was run 3 times with a different random number seed and each proceeded to over 300million iterations. The real-time log-likelihood of each model was recorded as the run progressed, and the three runs were then compared. The sufficiency of the marker data and of the run length was assessed empirically by the convergence of the log likelihood curves and the replication of results between the runs.

### Statistical analysis

Initially, the Genepop 4.0.11 software package was used to perform population comparisons of genotypes. The Hardy Weinberg principle is an expected pattern of genotypes produced by an observed frequency of alleles in a opulation (Edwards 2008). Exact tests for deviations from Hardy Weinberg (HW) expectations were calculated for all loci at all populations. Bonferroni correction for multiple comparisons was applied to the resultant *P* values (α = 0.007). Examination of the trait data revealed that a small number of individuals displayed anomalously high or low values for one or more of the traits. These outliers are likely due to experimental error, such as leaving a pinbone in place, or miscommunication of a number during the yield measurements in the factory. Samples were also observed having abnormally high quantities of localised connective tissue. For these reasons it was decided to remove anomalous values from the datasets using the criteria of 3 standard deviations from the mean to identify outliers. Data were tested for normality using the Shapiro Wilk statistic in SPSS (SPSS Inc.), to test the null hypothesis of a non-normal distribution. All traits from all five batches were shown to be normal. Univariate ANOVA and Pearson correlations were performed with the trait datasets, also using SPSS. A univariate General Linear Model (GLM) was performed for each trait in SPSS. Tensile work and Warner Bratzler shear work were continuous variables containing values based on mJ needed to perform one complete run of the texture analyser. Yield was a continuous variable containing values based on the trimmed fillet weight as a percentage of the whole gutted weight. Each model included batch, all five genotypes and a full factorial combination of all loci. The mean rank values of each trait at each genotype were tabulated and visually examined to detect genotypes with apparently distinct trait values. In order to remove the effects of the many likely between batch differences and to consider only the relative variation within the batches, all trait data-points were converted to rank values within the respective batch. The datasets were subsequently combined and treated as a single batch.

Data was re-assessed using SPSS statistics 19 (SPSS Inc.) - this data is presented in Example 2. Trait data were tested for normality using the Shapiro Wilk statistic and for homogeneity of variance using Levene's statistic. Firmness, tensile strength and yield all significantly deviated from normal distribution (P<0.001) and also from homogeneous variance between groups (P<0.001). Non-parametric statistical tests were therefore used for all further analyses. An independent samples Krusakal Wallis test was used to compare trait means across batches, Spearman correlations were performed pairwise between the trait datasets. A univariate Generalized Linear Model was performed for each trait. Tensile work and Warner Bratzler shear work (mJ) were continuous variables, and yield was a continuous variable containing values based on the trimmed fillet weight as a percentage of the whole gutted weight. Each model included batch identity as a random factor to account for potential differences between slaughter dates, husbandry technique, diet, cage location and other aquaculture variables. Egg source identity was included as a random factor to account for background strain genetics of the ova sources, and dam and sire identity were included as random factors to account for population stratification caused by presence of siblings and half-siblings within the sample set. Sex was included as a fixed factor to account for potential differences in the interaction of genotypes and traits between the sexes. Whole weight was included in all models as a covariate factor. The eight SNPs were tested as individual effects and two-way interactions.

### Results

### Example 1

### SNP identification

Initial data revealed a total of ten non-synonymous polymorphisms in coding regions and nine in 3'UTR sequences (Table 3). Hereafter, polymorphisms may be named using the short HGNC (http://www.genenames.org/) gene name followed by the amino acid position number and the two alternative amino acids. SNPs located in UTRs may be named using the cDNA sequence position in the relevant genbank entry (see Table 1) and the two alternative bases.

**Table 3: Details of single nucleotide polymorphisms identified by Sanger and Solexa sequencing. Min.Allele Freq. refers to the frequency of occurrence of the minor, or less common allele in the analysed sequencing reads during polymorphism discovery.**

| **Gene** | **Region** | **Base change** | **AA change** | **Min Allele Freq.** |
|---|---|---|---|---|
| MYF5 | Coding | 578-9 AC>GT | Ser193Asn | Asn 20% |
| MYOD1b | Coding | 829G>A | Cys243Tyr | Tyr 17% |
| CAST2 | Coding | 134G>T | Gly45Va1 | Val 41% |
| CAPN1a | Coding | 561C>T | Ser39Leu | Leu 25% |
| CAPN11 EST CX355265. 1 | 3' UTR | 611C>A | | A 16% |

### Example 2

The data provided in example 1 was supplemented with new data and re-analysed.

### SNPs discovered

A total of 16 SNPs were identified within the coding regions and the UTRs of the 37 candidate genes examined (Table 4). Of these 16, only 6 had a minimum allele frequency (MAF) >0.2 in the fish sampled for texture and fillet yield. Five were non-synonymous polymorphisms in protein-coding regions and one SNP was found in a 3'UTR sequence (Table 1). After coverage filtering, Solexa sequencing produced 89 RAD tags containing segregating SNPs. BLAST analysis of these tags revealed only two that were genuine alleles. The rest were apparently falsely called as SNPs due to repeat regions or highly homologous paralogues differing by a single base in the 50bp downstream of the SbfI cut site. The two remaining SNPs were named tag4083 and tag49325 after the identification numbers of the RAD tags in which they were found.

**Table 4 details of all candidate genes examined by Solexa and Sanger sequencing. The genes described below were amplified by PCR from cDNA and sequenced in order to identify sequence polymorphisms**

| Sequence | Gene | Type | San/Sol | Genbank | primers | | Size | Tm |
|---|---|---|---|---|---|---|---|---|
| CAST 1 | Calpastatin 1 | Known | Solexa | BT059251 | f | atgtccctggatgctctcagtgctc | 907 | 58 |
| | | | | | r | ctagctcttgccacccttatttttg | | 58 |
| CAST 1 Dom L | Calpastatin 1 | Known | Sanger | BT059251 | f | gagcggccaaagtgatgat | 1000 | 60 |
| | | | | | r | cagccagagtgtctcccaga | | 60 |
| CAST 2 | Calpastatin 2 | Novel | Solexa | | f | aagttggttacggctgttgctg | 950 | 57 |
| | | | | | r | ctagctcttgccacccttatttttg | | 57 |
| CTSB1 | Cathepsin B | Known | Solexa | BT060214 | f | cacgtctgacagcagataaccg | 1340 | 61 |
| | | | | | r | atgcccagttccaagtcaacc | | 61 |
| CTSB1 3'UTR | Cathepsin B | Known | Sanger | BT060214 | f | gaccactgtggcatcgagtct | 497 | 59 |
| | | | | | r | cctttcacccatcctgcatt | | 59 |
| CTSB2 | Cathepsin B | Known | Solexa | BT046013 | f | tcggtacacctgacaagcagac | 1060 | 60 |
| | | | | | r | cgcctctctcatcacatcctg | | 60 |
| CTSB2 3'UTR | Cathepsin D | Known | Sanger | BT046013 | f | gctggcatccctttgtaacc | 706 | 59 |
| | | | | | r | tccacatttcaatgatgatccaa | | 59 |
| CT SL1 | Cathepsin L | Known | Solexa | BT057383 | f | gcagtgttggtgctctgtgtg | 1100 | 59.5 |
| | | | | | r | gcagcactgtcgtcatcattg | | 59.5 |
| CT SD1 | Cathepsin D | Known | Solexa | BT059125 | f | tgtgtcttttcgcggcact | 1500 | 57 |
| | | | | | r | agtggagaatggggacgtagc | | 57 |
| CTSD1 3'UTR | Cathepsin D | Known | Sanger | BT059125 | f | gggcttcgctaagtccaagt | 532 | 59 |
| | | | | | r | ggtccattgcctcataaaacg | | 59 |
| CTSD2 | Cathepsin D | Known | Solexa | BT045103 | f | cggtttggacgtcctagattc | 1500 | 57 |
| | | | | | r | agtggctaggggctgttttt | | 57 |
| CTSD2 3'UTR | Cathepsin D | Known | Sanger | BT045103 | f | gtgtttgacagggacgcaga | 673 | 59 |
| | | | | | r | aacagaagcattgtggtcttgg | | 59 |
| CAPNs | Calpain small subunit | Known | Solexa | BT048580 | f | caatatcactggcacctcca | 762 | 59.5 |
| | | | | | r | cagcgccatgtagaaagaca | | 60 |
| CAPNs 3'UTR | Calpain small subunit | Known | Sanger | BT048580 | f | aggtcaacgttcaggatgg | 600 | 57 |
| | | | | | r | atggcatctgttggcttcat | | 57 |
| CAPN1a 1^{st} 1kb | Calpain 1 | Known | Solexa | BT059271 | f | actacggccaggtgagaatg | 1028 | 60 |
| | | | | | r | caggaagtcgctgaatgaca | | 60 |
| CAPN1a 2^{nd} 1kb | Calpain 1 | Known | Solexa | BT059271 | f | aacttccctgccacattctg | 1003 | 60 |
| | | | | | r | tgcaataacatcagtgagtcca | | 59 |
| CAPN1a 3'UTR | Calpain 1 | Known | Sanger | BT059271 | f | cttccagtggatcaccctgac | 1218 | 59 |
| | | | | | r | tgtaaacaacagtcgtgattgca | | 59 |
| CAPN1b 1^{st} 1kb | Calpain 1 | Known | Solexa | BT059312 | f | cagccgtctgctgtaggtaa | 1009 | 59 |
| | | | | | r | gaactctccgtcctcactgc | | 60 |
| CAPN1b 2^{nd} 1kb | Calpain 1 | Known | Solexa | BT059312 | f | actccagagagtgggagggt | 1042 | 60 |
| | | | | | r | cgcagtatgatcagctggaa | | 60 |
| CAPN1b 3'UTR | Calpain 1 | Known | Sanger | BT059312 | f | ggatcagcctgaccatgtttg | 1000 | 59 |
| | | | | | r | ttggcgttcatctacacagga | | 59 |
| CAPN2 1^{st} 1kb | Calpain 2 | EST contig | Solexa | | f | acaaggacaaggacaatggc | 1000 | 59 |
| | | | | | r | attgttcctgcatcctccag | | 59 |
| CAPN2 2^{nd} 1kb | Calpain 2 | EST contig | Solexa | | f | tctggagctctgcactctga | 1000 | 59 |
| | | | | | r | tttgggaggactggtctttg | | 59 |
| CAPN3 1^{st} 1kb | Calpain 3 | Known | Solexa | BT058958 | f | gacacgcataaggggaaaaa | 1000 | 58.5 |
| | | | | | r | ccagctatgggacttgtcgt | | 58.5 |
| CAPN3 2^{nd} 1kb | Calpain 3 | Known | Solexa | BT058958 | f | acaagtcccatagctggacg | 1000 | 59 |
| | | | | | r | gagcaggactcctctgatgg | | 59 |
| CAPN3 3'UTR | Calpain 3 | Known | Sanger | BT058958 | f | tggctccagttgaccatgta | 1060 | 57 |
| | | | | | r | tccaacaatccagtccaaca | | 57 |
| CAPN11 EST 1 | Calpain 11 | EST | Solexa | GE782145 | f | ttggctttactcagatgacatga | 700 | 60 |
| | | | | | r | gtaaaactctgccacgccac | | 60 |
| CAPN11 EST2 | Calpain 11 | EST | Solexa | CX355265 | f | ctgcagacttggaagatgagg | 700 | 60 |
| | | | | | r | caggcagaggacaataaggc | | 60 |
| FSTN | Follistatin | Known | Sanger | DQ186633 | f | cccgatacctcgttcacttg | 1020 | 60 |
| | | | | | r | caggagtgttcccagagagc | | 60 |
| MSTN1a | Myostatin | Known | Sanger | AJ344158 | f | actctgtagtccgccttcacg | 1140 | 59 |
| | | | | | r | tgctctttgcggttgaagtag | | 59 |
| MSTN1a 3'UTR | Myostatin | Known | Sanger | AJ344158 | f | gatgctcgtgagcgagagct | 1000 | 60 |
| | | | | | r | tttgccatagtgcaaactgtgtt | | 60 |
| MSTN1b | Myostatin | Known | Sanger | AJ297267 | f | actctgtagtccgccttcacg | 1140 | 59 |
| | | | | | r | tgctctttgcggttgaagtag | | 59 |
| MSTN1b 3'UTR | Myostatin | Known | Sanger | AJ297267 | f | accctggactttgggacaga | 1000 | 59 |
| | | | | | r | gaaaggcaactgctaatggaaa | | 59 |
| MYOG | Myogenin | Known | Sanger | DQ294029 | f | ctagcgtcgaccagtatggag | 800 | 60 |
| | | | | | r | ctctgggtttatttgggaatg | | 60 |
| MYOD1a | MyoD | Known | Sanger | AJ557148 | f | gccggatattcctttcccta | 850 | 60 |
| | | | | | r | gctccgttctcactcgctat | | 60 |
| MYOD1b | MyoD | Known | Sanger | AJ557149 | f | cctgtcacctctgctgatga | 850 | 60 |
| | | | | | r | cttggtagatggggtcatgg | | 60 |
| MYOD1c | MyoD | Known | Sanger | DQ366709 | f | atggagttgtcggatatttcg | 850 | 60 |
| | | | | | r | tcatagcacttggtagatggggtc | | 60 |
| MRF4a | MRF4 | Known | Sanger | BT047543 | f | atgatggacctttttgagacc | 800 | 60 |
| | | | | | r | ggaaatacgaaatgctgacg | | 60 |
| MRF4b | MRF4 | Known | Sanger | BT049875 | f | atgatggacctttttgagacc | 800 | 60 |
| | | | | | r | ggaaatacgaaatgctgacg | | 60 |
| MYF5 | MYF5 | Known | Sanger | DQ452070 | f | atggatgtcttctcccagtcc | 760 | 60 |
| | | | | | r | tcacaatacgtggtacacaggtc | | 60 |
| CEE | Cee | Known | Sanger | EF036472 | f | atgtcggagcaggaggctctg | 1000 | 57 |
| | | | | | r | tcagtccagctcaatggggc | | 57 |
| SDHD | Succunate dehydrogenase D | Known | Sanger | BT071922 | f | tggtaggggtcagggggac | 870 | 58 |
| | | | | | r | ggccatcgtcaggataaattc | | 58 |
| HSP90a 1^{st} kb | Heatshock protein 90a | Known | Sanger | BT059272 | f | ttccacaacaagatgccgg | 1000 | 59 |
| | | | | | r | tgacagccaggtgttcttcc | | 59 |
| HSP90a 2^{nd} kb | Heatshock protein 90a | Known | Sanger | BT059272 | f | ggaagaacacctggctgtca | 1200 | 58 |
| | | | | | r | tcctggatgtgtcctcgtct | | 58 |
| LOX | Lysyl oxidase | Known | Solexa | DQ167828 | f | agcatcaggcatgtccatc | 819 | 58 |
| | | | | | r | gccagtatagcggacctcac | | 58 |
| IGF-1 | Insulin-like growth factor | Known | Sanger | EF432852 | f | acgagcctgcgcaatggaaca | 833 | 57 |
| | | | | | r | ccccagcactttaactctcagcttt | | 57 |
| IGF-2 | Insulin-like growth factor | Known | Sanger | EF432854 | f | cgggggtcgcctggtctttg | 801 | 55 |
| | | | | | r | gggggagttggggtgctggt | | 55 |
| IGFBP-1 | IGF binding protein | Known | Sanger | EF432856 | f | ccctcctgacctctctggccc | 601 | 55 |
| | | | | | r | ctggcagggggtcccacca | | 55 |
| IGFBP-2 | IGF binding protein | Known | Sanger | EF432858 | f | tgtcggtggctttcgtgggc | 804 | 57 |
| | | | | | r | tctgggctgaggcgagggtg | | 57 |
| IGFBP-3 | IGF binding protein | Known | Sanger | EF432860 | f | agctgcggtttgcttctgct | 769 | 57 |
| | | | | | r | actggctgcagttggggtcc | | 57 |
| IGFBP-4 | IGF binding protein | Known | Sanger | EU861007 | f | gctatagcgccgccctgctg | 670 | 57 |
| | | | | | r | accagcactttccccgctgc | | 57 |
| IGFBP-5 | IGF binding protein | Known | Sanger | EF432862 | f | ggctgaccgggtcctttggc | 709 | 57 |
| | | | | | r | ccgggaacctgggagccgta | | 57 |
| IGFBP-5.2 | IGF binding protein | Known | Sanger | EU861009 | f | agcgagtcgggttgtttgggc | 750 | 57 |
| | | | | | r | gctctccgggtctttgcactgg | | 57 |
| IGFBP-6 | IGF binding protein | Known | Sanger | BT045207 | f | ccggctaagcaccaagcaaagc | 862 | 57 |
| | | | | | r | tccctggtttctcctgtcgaggc | | 57 |
| IGFBP-rP1 | IGFBP receptor protein | Known | Sanger | BT059616 | f | atgttagtgtttttcgctgtggtc | 795 | 58 |
| | | | | | r | gaattcacagctcatcatccttggccac | | 58 |

### Trait values

Fillet yield, tensile work and shear work were significantly different between the eight batches (all P<0.001). The average yield ranged from 64.6% in batch 5 to 72.8% in batch 1. The lowest average tensile and firmness values were seen in batch 1 (258.8mJ and 267.5mJ respectively), while batch 2 had the highest, (482.4mJ tensile and 489.6mJ firmness). The total sex ratio across the eight batches was 55% male and 45% female. There was considerable variation between batches, ranging from 32% male in batch 2 to 75% male in batch 6 (2). The numbers of full and half sibling dyads are also given in Table 2, along with numbers of individual male and female parents inferred to have contributed to each batch.

### Genotype/trait association

Four SNPs showed significant individual effects on fillet yield. Cast2 possessed a rare allele, the homozygous genotype of which was found in 1.7% of the tested fish but was associated with a yield gain 4.71% above average. The homozygous common allele of Myodlb was associated with a yield increase of 0.96% and was considerably more common, occurring in 35% of tested animals. Capn11 and Capn3 both appeared to have anallele which was associated with a small increase in yield, either when homozygous or heterozygous. In combination, Cast2 and Myod had a significant effect on yield (*P*=0.001). The combined favourable homozygous genotypes at these loci were associated with a predicted 6% increase in yield, but this was found in only 1.6% of the sampled individuals. The combined effect of Capn3 and Capn11 on yield was also significant (*P*=0.002), with three genotype combinations being associated with predicted yield increases greater than 1%. All significant SNP effects on fillet yield are described in Table 5.

Three SNPs were individually associated with tensile strength. The strongest association was found with tag49325 (*P*=0.012), which had an estimated tensile strength gain of 10.05% associated with the CC genotype, present in 54% of the population. was associated with an 8.77% tensile strength gain (*P*=0.022). The favourable TT homozygote was found in 11% of the tested animals. Additionally, the combination of tag4083 and tag49325 was significantly associated with yield (*P*=0.028) and the combined favourable genotype, TTCC, was associated with an estimated tensile strength increase of 20.7%, and was found in 4.5% of individuals. The combination of Capn1 and tag4083 SNPs was also significantly associated with tensile strength (*P*<0.001). The combined favourable genotype, GGTT, was found in 2% of animals tested and estimated to be associated with a tensile strength increase of 26.9%. Two SNPs were significantly associated with firmness, as measured by Warner Bratzler shear force. The most notable being tag49325 (*P*<0.001), the CC genotype of which was associated with an estimated firmness increase of 17.2%. Additionally, Capn3 was associated with firmness, but the GG genotype was favourable (10.74% gain), which is in contrast to the yield analysis where the alternative CC homozygote was favourable. The combined effect of tag49325 and Capn3 was also highly significant (*P*<0.001), the combined favourable genotype (GGCC) being associated with an estimated 19.63% increase in fillet firmness.

**Table 5. Significant associations between SNP genotypes and fillet traits. P = significance of the locus/loci as a factor in the linear model. Favourable genotypes are those associated with a trait mean greater than average. Frequency = the percentage occurrence of the genotype across all tested individuals. Gain = estimated genotype trait mean - estimated total trait mean. % gain = gain expressed as a percentage of the total trait mean.**

| **Trait** | **SNP(s)** | ***P*** | **Favourable genotype** | **Frequency (%)** | **Gain** | **% gain** |
|---|---|---|---|---|---|---|
| **Yield** (%) | Cast2 | 0.006 | TT | 1.7 | 3.34 | 4.71 |
| | Myodlb | 0.004 | GG | 35.4 | 0.66 | 0.96 |
| | Capn11 | 0.015 | CC | 74.0 | 0.5 | 0.73 |
| | | | CA | 15.8 | 0.5 | 0.73 |
| | Capn3 | 0.002 | CC | 9.4 | 0.35 | 0.51 |
| | | | CG | 48.2 | 0.55 | 0.79 |
| | Cast2*Myod1b | 0.001 | TTGG | 1.6 | 4.23 | 6.00 |
| | Cast2*Capn11 | <0.001 | TTCC | 1.8 | 4.25 | 6.08 |
| | Myod1b*Capn11 | <0.001 | GGCC | 29.1 | 1 | 1.46 |
| | Cap3*Capn11 | 0.002 | CGCC | 35.1 | 1 | 1.45 |
| | | | CCCC | 6.7 | 0.7 | 1.02 |
| | | | CCCA | 0.8 | 0.7 | 1.02 |
| | Myod1b*Capn3 | <0.001 | GGCG | 35.1 | 1.07 | 1.55 |
| **Tensile (mJ)** | Capn1 | 0.024 | GG | 33.6 | 7.7 | 2.11 |
| | | | AA | 13.1 | 15 | 4.12 |
| | tag4083 | 0.022 | AA | 11.0 | 32.6 | 8.77 |
| | tag49325 | 0.012 | CC | 54.9 | 33.6 | 10.05 |
| | Capn1*4083 | <0.001 | GGAA | 2.0 | 104.22 | 26.88 |
| | 49325*4083 | 0.028 | CCAA | 4.5 | 71.54 | 20.74 |
| **Firmness (mJ)** | Capn3 | 0.023 | GG | 57.5 | 34.7 | 10.27 |
| | tag49325 | <0.001 | CC | 54.9 | 54.38 | 17.24 |
| | tag49325*Capn3 | <0.001 | CCGG | 22.0 | 63 | 19.63 |

### Discussion

### Relationship of traits to genotypes

Described herein are seven individual SNPs and eight multi-locus genotypes with the power to segregate a small percentage of individual Atlantic salmon with significantly improved tensile strength, firmness or fillet yield relative to the unselected population. The majority of these markers were discovered using the candidate gene approach. The genetics of salmonids is more complex than that of mammals due to whole genome duplication events at the base of the teleost radiation (3R) (Jaillon et al. 2004) and in the lineage leading to modern salmonids (4R) (Allendorf and Thorgaard, 1984). Atlantic salmon are considered autotetraploid and in the process of reverting to a diploid state (Allendorf and Thorgaard, 1984). In theory, 4 gene family members are possible relative to each single mammalian orthologue, although in practice paralogue retention rates are in the range 30-70% (Koop and Davidson, 2008). Extensive sequence characterisation, phylogenetic and synteny analysis is required to attribute retained paralogues to 3R or 4R and devise a correct nomenclature across gene family members (Macqueen et al. 2010a). 4R paralogues often show 97% sequence identity, but yet markedly distinct expression patterns in different physiological contexts due to complex patterns of regulatory evolution (Macqueen et al. 2010a; 2012). The calpain-calpastatin system which is involved in protein turnover, myogenesis and the post-mortem degradation of myofibrillar proteins in fish (Bahuaud et al. 2010; Geesink et al. 2000) and mammals (Casas et al. 2006; Gill et al. 2009; Schenkel et al. 2006; Ciobanu et al. 2004) was the source of 50% of the SNPs that had significant associations with the traits of interest (Table 4). Calpains represent a superfamily of Ca²⁺-regulated proteases comprising 14 distinct genes in the human genome including the ubiquitously expressed calpnl and calpn2 and calpn3 which is expressed much more strongly in skeletal muscle than in other tissues (Goll et al. 2003). Teleosts possess another ubiquitous calpain (calpn11) which forms a monophyletic clade ancestral to calpnl and calpn2, which is restricted to testis in eutherian mammals (Macqueen et al. 2010b).

Myoblast Determination Factor (myoD) is one of four master transcription factors with specific and redundant functions in myogenic lineage determination and muscle differentiation (Hinitis et al. 2009). Two paralogues of myoD (myoD1 and myoD2) are conserved in some teleosts from the 3R duplication event, although myoD2 has subsequently been lost from salmonid genomes (Macqueen et al. 2008). The three myoD1 genes present in Atlantic salmon (myoD1a, 1b and lc) resulted from the lineage-specific WGD event in salmonids and a local duplication (Macqueen et al. 2008). *In vitro,* myoDlb and 1c expression was strongly associated with myoblast proliferation whereas myoD1a expression peaked during terminal differentiation concomitant with myotube formation and myofibrillargenesis (Bower and Johnston, 2010). SNPs within Myod genes have previously been shown to influence carcass traits in cattle (Bhuiyan et al. 2009) and pigs (Verner et al. 2007)

The sequencing of RAD-tagged libraries represents a very cost effective method for SNP discovery and genotyping (Baird et al. 2008). In the present study we obtained 25million 50bp reads corresponding to 82,164 RAD-tags containing 569 potential SNPs segregating between the high and low trait pools. After filtering on quality, depth and uniformity of coverage only 89 SNPs remained with the relatively high false positive rate ('pseudo SNPs') likely due to homologous paralogues and repeated regions. Only 2 of the SNPs in RAD-tags showed significant association with the traits of interest with tag4083 and tag49325 correlating with tensile strength and tag4935 with fillet firmness (Table 4). However, the effect of these SNPs on trait values exceeded that found in any of the candidate genes confirming the utility of such genome-wide discovery methods.

### Benefits to breeders

Marker assisted selection for increased fillet yield and/or firmer flesh may be used in conjunction with family selection programs for high growth rate and disease resistance. The commercial value of increasing fillet yield is relatively straightforward to quantify. The typical slaughter weight of Atlantic salmon is 4.5 kg of which in a modern processing plant 65-75% is recoverable muscle fillet depending on batch origin. Farm gate prices of Atlantic salmon in the European Union averaged around €5/kg in 2011 (REF), equivalent to €7.7/g or €34.3/fish for a 65% yield. Thus 1% and 6% increases in fillet yield are worth 35 cents to €2 per salmon more to the processor than unselected fish. The potential economic value of texture markers may be much harder to assess since the quantitative relationships between fillet firmness and tensile strength and downgrading losses during secondary processing are unknown. Tensile strength showed a good correlation with the total length of gapes in per salmon fillet (R2=0.6, X degrees of freedom) which suggests that eliminating fish with low tensile strength values from the population would decrease the incidence of downgrading and increase process yield.

### Additive effects

The present results demonstrate the possibility of combining SNP markers to create an additive effect on the trait of interest. Cast2, Capn11 and Myodlb were all significantly associated with fillet yield individually. All two-locus combinations of these genes were also significantly associated with yield. This suggests their effect is additive and that all three loci could be used to create a three-locus genotype of even greater influence. Capnla, tag4083 and tag49325 also appear to have an additive association with tensile strength and seem likely to be able to exert a greater combined influence over the trait. With a larger trait and DNA database, it should be possible to explore more complex multi-locus genotypes to take advantage of the summation of these less strong genetic influences. Future work may examine the creation of a large panel of markers with additive effects on commercial quality and profitability. These may be used to improve salmon breeding programmes as has been implemented in the cattle industry with commercial success (Weaber and Lusk 2010).

### Trade-offs and co-selection

In beef cattle, increased tenderness has been shown to be associated with increased muscle yield as segregated by genetic polymorphisms within the calpain system (Gill *et al.* 2009). Since tenderness is not desired in salmon, it was considered possible that selection for firmer flesh would be associated with poorer yield. The only example of a marker having such an effect was Capn3, which appeared to have a dominant C allele that favoured fillet yield either in homozygous or heterozygous state, while the homozygous G allele favoured firmness. Since the effect of Capn3 on yield is not strong (<1% above average) one of skill may opt not to used it as a (brood)stock selection marker, except when one trait is selected at the expense of the other. In cases where co-selection of yield and firm texture is required, the skilled user may opt not to use capn3 as a marker. All other markers significantly associated with increased fillet trait showed no conflicting influences, suggesting they may be used as stock selection markers without trade-offs in flesh texture.

### References

Anderson, S., 2000 Salmon Color and the Consumer. Proc. Int. Inst. of Fisheries Economics and Trade (IIFET) Conf. Corvallis, Oregon, USA.
Ashton, T. J., I. Michie and I. A. Johnston, 2010 A Novel Tensile Test Method to Assess Texture and Gaping in Salmon Fillets. journal of food science 75: S182-S190.
Bahuaud, D., M. Gaarder, E. Veiseth-Kent and M. Thomassen, 2010 Fillet texture and protease activities in different families of farmed Atlantic salmon (Salmo salar L.). Aquaculture 310: 213-220.
Baird, N. A., P. D. Etter, T. S. Atwood, M. C. Currey, A. L. Shiver et al., 2008 Rapid SNP Discovery and Genetic Mapping Using Sequenced RAD Markers. PLoS One 3: e3376.
Baranski, M., T. Moen and D. Vage, 2010 Mapping of quantitative trait loci for flesh colour and growth traits in Atlantic salmon (Salmo salar). Genetics Selection Evolution 42: 17.
Bhuiyan, M. S. A., N. K. Kim, Y. M. Cho, D. Yoon, K. S. Kim et al., 2009 Identification of SNPs in MYOD gene family and their associations with carcass traits in cattle. Livestock Science 126: 292-297.
Brix, K., A. Dunkhorst, K. Mayer and S. Jordans, 2008 Cysteine cathepsins: Cellular roadmap to different functions. Biochimie 90: 194-207.
Carlson, C. S., S. F. Aldred, P. K. Lee, R. P. Tracy, S. M. Schwartz et al., 2005 Polymorphisms within the C-Reactive Protein (CRP) Promoter Region Are Associated with Plasma CRP Levels. The American Journal of Human Genetics 77: 64-77.
Casas, E., S. N. White, T. L. Wheeler, S. D. Shackleford, M. Koohmaraie et al., 2006 Effects of calpastatin and u-calpain markers in beef cattle on tenderness traits. Journal of Animal Science 84: 520-525.
Cheret, R., C. Delbarre-Ladrat, M. d. Lamballerie-Anton and V. Verrez-Bagnis, 2007 Calpain and cathepsin activities in post mortem fish and meat muscles. Food Chemistry 101: 1474-1479.
Ciobanu, D. C., J. W. M. Bastiaansen, S. M. Lonergan, H. Thomsen, J. C. M. Dekkers et al., 2004 New alleles in calpastatin gene are associated with meat quality traits in pigs. Journal of Animal Science 82: 2829-2839.
Delbarre-Ladrat, C., V. Verrez-Bagnis, J. Noel and J. Fleurence, 2004 Relative contribution of calpain and cathepsins to protein degradation in muscle of sea bass (Dicentrarchus labrax L.). Food Chemistry 88: 389-395.
Einen, O., B. Waagan and M. Thomassen, 1998 Starvation prior to slaughter in Atlantic salmon (Salmo salar) I. Effects on weight loss, body shape, slaughter- and fillet-yield, proximate and fatty acid composition. Aquaculture 166: 85-104.
Geesink, G. H., J. D. Morton, M. P. Kent and R. Bickerstaffe, 2000 Partial purification and characterization of chinook salmon (Oncorhynchus tshawytscha) calpains and an evaluation of their role in postmortem proteolysis. Journal of food science 65: 1318-1324.
Gill, J., S. Bishop, C. McCorquodale, J. Williams and P. Wiener, 2009 Association of selected SNP with carcass and taste panel assessed meat quality traits in a commercial population of Aberdeen Angus-sired beef cattle. Genetics Selection Evolution 41: 36.
Gjedrem, T., and M. Baranski, 2009 The Success of Selective Breeding in Aquaculture, pp. 13-23 in Selective Breeding in Aquaculture. Springer Netherlands.
Gjedrem, T., H. M. Gjøen and B. Gjerde, 1991 Genetic origin of Norwegian farmed Atlantic salmon. Aquaculture 98: 41-50.
Godiksen, H., M. Morzel, G Hyldig and F. Jessen, 2009 Contribution of cathepsins B, L and D to muscle protein profiles correlated with texture in rainbow trout (Oncorhynchus mykiss). Food Chemistry 113: 889-896.
Goll, D. E., V. F. Thompson, H. Li, W. Wei and J. Cong, 2003 The Calpain System. Physiol. Rev. 83: 731-801.
Haard, N. F., 1992 Control of chemical composition and food quality attributes of cultured fish. Food Research International 25: 289-307.
Hall, T. A., 1999 BioEdit: a user-friendly biological sequence alignment editor and analysis program for Windows 95/98/NT. Nucl. Acids. Symp. Ser. 41: 95-98.
Houston, R., C. Haley, A. Hamilton, D. Guy, A. Tinch et al., 2008 Major quantitative trait loci affect resistance to infectious pancreatic necrosis in Atlantic salmon (Salmo salar). genetics 178: 1109 - 1115.
Lavéty, J., O. A. Afolabi and R. M. Love, 1988 The connective tissues of fish. International Journal of Food Science and Technology 23: 23-30.
Leong, J., S. Jantzen, K. von Schalburg, G. Cooper, A. Messmer et al., 2010 Salmo salar and Esox lucius full-length cDNA sequences reveal changes in evolutionary pressures on a post-tetraploidization genome. BMC Genomics 11: 279.Michie, I., 2001 Causes of Downgrading in the Salmon Farming Industry, pp. 129-136 in Farmed Fish Quality, edited by S. C. Kestin and P. D. Warriss. Fishing News Books.
Miller, M. R., J. P. Dunham, A. Amores, W. A. Cresko and E. A. Johnson, 2007 Rapid and cost-effective polymorphism identification and genotyping using restriction site associated DNA (RAD) markers. Genome Research 17: 240-248.
Moen, T., M. Baranski, A. Sonesson and S. Kjoglum, 2009 Confirmation and fine-mapping of a major QTL for resistance to infectious pancreatic necrosis in Atlantic salmon (Salmo salar): population-level associations between markers and trait. BMC Genomics 10: 368.
Ng, S., C. Artieri, I. Bosdet, R. Chiu, R. Danzmann et al., 2005 A physical map of the genome of Atlantic salmon, Salmo salar. Genomics 86: 396-404.
Robb, D. H. F., S. C. Kestin, P. D. Warriss and G. R. Nute, 2002 Muscle lipid content determines the eating quality of smoked and cooked Atlantic salmon (Salmo salar). Aquaculture 205: 345-358.
Ryynanen, H., and C. Primmer, 2006 Single nucleotide polymorphism (SNP) discovery in duplicated genomes: intron-primed exon-crossing (IPEC) as a strategy for avoiding amplification of duplicated loci in Atlantic salmon (Salmo salar) and other salmonid fishes. BMC Genomics 7: 192.
Sargent, J. R., J. G. Bell, F. McGhee, J. McEvoy and J. L. Webster, 2001 The nutritional value of fish, pp. 3-12 in Farmed Fish Quality, edited by S. C. Kestin and P. D. Warriss. Fishing News Books, Oxford.
Schenkel, F., S. Miller, Z. Jiang, I. Mandell, X. Ye et al., 2006 Association of a single nucleotide polymorphism in the calpastatin gene with carcass and meat quality traits of beef cattle. J Anim Sci 84: 291 - 299.
Verner, J., P. Humpolí 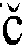 ek and A. Knoll, 2007 Impact of MYOD family genes on pork traits in Large White and Landrace pigs. Journal of Animal Breeding and Genetics 124: 81-85.
Wang, J., M. Pitarque and M. Ingelman-Sundberg, 2006 3'-UTR polymorphism in the human CYP2A6 gene affects mRNA stability and enzyme expression. Biochemical and Biophysical Research Communications 340: 491-497.
Weaber, R. L., and J. L. Lusk, 2010 The Economic Value of Improvements in Beef Tenderness by Genetic Marker Selection. American Journal of Agricultural Economics 92: 1456-1471.
Weinberg, E. S., M. L. Allende, C. S. Kelly, A. Abdelhamid, T. Murakami et al., 1996 Developmental regulation of zebrafish MyoD in wild-type, no tail and spadetail embryos. Development 122: 271-280.
Yun, K., and B. Wold, 1996 Skeletal muscle determination and differentiation: story of a core regulatory network and its context. Current Opinion in Cell Biology 8: 877-889.

## Claims

1. A method of selecting fish from species within the family *Salmonidae* with one or more improved traits, said method comprising the step of identifying in a sample, the presence of sequence variations within the nucleic acid or amino acid sequences encoding one or more of the proteins/sequences selected from the group consisting of:
(i) CAST2 (SEQ ID NO: 7/8);
(ii) cAPN1a (SEQ ID NO: 1/2);
(iii) MYOD1b (SEQ ID NO: 3/4);
(iv) MYF5 (SEQ ID NO: 5/6);
(v) CAPN11 (SEQ ID NO: 9);
(vi) CAPN3 (SEQ ID NO: 10/11);
(vii) DY714774 (SEQ ID NO: 12); and
(viii) AGKD01012360 (SEQ ID NO: 13)
wherein the presence of variations in the nucleic acid or amino acid sequences encoding any of (i)-(viii) above, are associated with improved traits, optionally wherein, (a) the improved trait is assessed relative to the corresponding trait in a control fish or flesh sample or by reference to the corresponding trait in an unselected population of fish; and
(b) the sequence variation is determined relative to the sequence of a reference sequence.

2. A method of selecting fish from species within the family *Salmonidae* having a greater yield of flesh, said method comprising the steps of identifying in a sample, sequence variations within the nucleic acid or amino acid sequences encoding one or more proteins selected from the group consisting of:
(a) CAST2;
(b) MYOD1b;
(c) CAPN11; and
(d) CAPN3.
wherein the presence of sequence variations in the nucleic acid or amino acid sequences encoding any of (a)-(d) above, are associated with a greater yield of flesh, optionally wherein, (a) the greater yield of flesh is assessed relative to the corresponding yield of flesh in a control fish or flesh sample or by reference to the corresponding flesh yield in an unselected population of fish; and
(b) the sequence variation is determined relative to the sequence of a reference sequence.

3. The method of claim 1, wherein the method comprises identifying the presence of sequence variations within the nucleic acid and/or amino acid sequence encoding CAST2 (SEQ ID NO: 7/8), optionally wherein, the method further comprises identifying the presence of sequence variations within the nucleic acid and/or amino acid sequences encoding one or more proteins selected from the group consisting of:
(i) cAPN1a (SEQ ID NO: 1/2);
(ii) MYOD1b (SEQ ID NO: 3/4);
(iii) MYF5 (SEQ ID NO: 5/6);
(iv) CAPN11 (SEQ ID NO: 9);
(v) CAPN3 (SEQ ID NO: 10/11);
(vi) DY714774 (SEQ ID NO: 12); and
(vii) AGKD01012360 (SEQ ID NO: 13)

4. The method of claims 1 or 3 wherein the improved traits are one or more selected from the group consisting of:
(i) improved fillet yield;
(ii) improved flesh texture;
(iii) improved flesh nutrition;
(iv) improved reduced gaping;
(vi) improved flesh tensile strength; and
(vii) improved flesh firmness.

5. A method of selecting fish from species within the family *Salmonidae* having flesh exhibiting improved tensile strength, said method comprising the steps of identifying in a sample, sequence variations within the nucleic acid or amino acid sequences encoding one or more proteins selected from the group consisting of:
(a) CAPN1;
(b) DY714774; and
(c) AGKD01012360.
wherein the presence of sequence variations in the nucleic acid or amino acid sequences encoding any of (a)-(c) above, are associated with flesh exhibiting improved tensile strength.

6. A method of selecting fish from species within the family *Salmonidae* having flesh exhibiting improved firmness, said method comprising the steps of identifying in a sample sequence variations within the nucleic acid or amino acid sequences encoding one or more proteins selected from the group consisting of:
(a) CAPN3;
(b) DY714774; and
(c) AGKD01012360.
wherein the presence of sequence variations in the nucleic acid or amino acid sequences encoding any of (a)-(c) above, are associated with flesh exhibiting improved firmness.

7. The method of any preceding claim, wherein the sequence variation comprises one or more polymorphisms, optionally wherein the sequence variations are selected from the group consisting of:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a: Ser39Leu;
(ix) CAPN11 611 C>A;
(x) CAPN3: 1701 C>G;
(xi) CAPN3: Thr541Arg;
(xii) DY714774 251 C>T; and
(xiii) AGKD01012360 520 C>A.

8. A method of selecting fish from species within the family *Salmonidae* having a greater yield of flesh, said method comprising the steps of identifying in a sample sequence one or more of the sequence variations given as (i), (ii), (iii), (iv) and (v) below:
(i)
(a) CAST2 234 G>T; or
(b) CAST2 Gly45Val
and
(a) MYOD1b: 829 G>A; or
(b) MYOD1b: Cys243Tyr;
(ii) s
(a) CAST2 234 G>T; or
(b) CAST2 Gly45Val
and
(a) CAPN11 611 C>A
(iii) (a) MYOD1b: 829 G>A; or
(b) MYOD1b: Cys243Tyr;
and
(a) CAPN11 611 C>A
(iv)
(a) CAPN3 1701 C>G; or
(b) CAPN3: Thr541Arg
and
(a) CAPN11 611 C>A
(v)
(a) MYOD1b: 829 G>A; or
(b) MYOD1b: Cys243Tyr;
and
(a) CAPN3 1701 C>G; or
(b) CAPN3 Thr541Arg.
wherein detection of one or more of sequence variations (i), (ii), (iii), (iv) or and/or (v) in a sample, indicates that the sample was provided by or obtained from a fish having a greater yield of flesh, optionally wherein the greater yield of flesh is assessed relative to a fish lacking one or more of sequence variations (i), (ii), (iii), (iv) or (v).

9. A method of selecting fish from species within the family *Salmonidae* having flesh exhibiting improved tensile strength, said method comprising the steps of identifying in a sample, one or more of the sequence variations given as (i) and (ii):
(i)
(a) cAPN11 611 C>A
and
(a) DY714774 (TAG4083) 251 C>T
(ii)
(a) AGKD01012360 (TAG49325) 520 C>A
and
(a) DY714774 (TAG4083) 251 C>T
wherein detection of sequence variations (i) and/or (ii) in a sample, indicates that the sample was provided by or obtained from, a fish with flesh exhibiting improved tensile strength.

10. A method of selecting a fish from species within the family *Salmonidae* with flesh exhibiting improved firmness, said method comprising the steps of identifying in a sample, the sequence variations:
(a) AGKD01012360 (TAG49325) 520 C>A
and
(a) CAPN3 1701 C>G
(b) CAPN3 Thr541Arg
wherein detection of these sequence variations in a sample, indicates that the sample was provided by or obtained from, a fish with flesh exhibiting improved firmness.

11. A method for identifying broodstock fish from species within the family *Salmonidae,* said method comprising the steps of providing a sample from a fish and identifying in the presence of sequence variations within the nucleic acid or amino acid sequences encoding one or more of the proteins selected from the group consisting of:
(i) CAST2 (SEQ ID NO: 7/8);
(ii) cAPN1a (SEQ ID NO: 1/2);
(iii) MYOD1B (SEQ ID NO: 3/4);
(iv) MYF5 (SEQ ID NO: 5/6);
(vi) CAPN11 (SEQ ID NO: 9);
(vi) CAPN3 (SEQ ID NO: 10/11);
(vii) DY714774 (SEQ ID NO: 12); and
(viii) AGKD01012360 (SEQ ID NO: 13)
wherein fish identified as possessing one or variations in the nucleic and/or amino acid sequences encoding one or more of the proteins/sequences identified as (i)-(viii) above, are suitable for selection as broodstock.

12. The method of any preceding claim, wherein the fish are wild or farmed fish, optionally, wherein the species is
*Salmo solar* (the Atlantic salmon).

13. The method of any preceding claim wherein the sample is provided by or obtained from a fish to be tested and comprises nucleic acid and/or peptides and/or proteins optionally, wherein the sample comprises one or more selected from the group consisting of:
(i) a tissue biopsy (comprising cells and other material),
(ii) a scraping or swab;
(iii) a biological fluid;
(iv) a tissue and/or glandular secretion; and
(v) embryonic material

14. A method of selecting fish from species within the family *Salmonidae* with improved traits, said method comprising the step of identifying in a sample, a level of expression, function and/or activity of one or more of the proteins selected from the group consisting of:
(i) CAPN1a;
(ii) MYOD1b;
(iii) MYF5;
(iv) CAST2; and
(v) CAPN11
(vi) CAPN3
(vii) DY714774
(viii) AGKD01012360
wherein any modulation in the level of protein expression, function and/or activity may indicate that the sample was provided by a fish exhibiting improved traits.

15. A kit for identifying and/or determining whether or not a variant flesh quality associated gene/protein is present or absent in, a nucleic acid sample provided by a fish species from within the family *Salmonidae,* said kit comprising one or more pairs of oligonucleotide primers useful for amplifying a nucleotide sequence of interest and one or more components selected from the group consisting of:
(i) a nucleic acid polymerizing agent;
(ii) chain elongating nucleotides;
(iii) reaction and/or storage buffers;
(iv) vials or other storage/reaction vessels; and
(v) instructions for use.
wherein the nucleotide sequence of interest comprises a variant CAST2, MYF5, MYOD1b, CAPN1a, CAPN11, CAPN3, DY714774 and/or AGKD01012360 sequence, optionally wherein the one or more pairs of oligonucleotide primers are for amplifying a variant sequence selected from the group consisting of:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a Ser39Leu;
(ix) CAPN11 611 C>A;
(x) CAPN3: 1701 C>G;
(xi) CAPN3: Thr541Arg;
(xii) DY714774 251 C>T; and
(xiii) AGKD01012360 520 C>A.

## Patentansprüche

1. Verfahren für die Selektion von Fisch aus Spezies in der Familie der *Salmonidae* mit einem oder mehreren verbesserten Merkmalen, wobei das Verfahren in einer Probe den Schritt des Identifizierens des Vorhandenseins von Sequenzvariationen innerhalb der Nucleinsäure- oder Aminosäuresequenzen umfasst, die ein oder mehrere der Proteine/Sequenzen codieren, die ausgewählt sind aus der Gruppe, bestehend aus:
(i) CAST2 (SEQ ID NO: 7/8);
(ii) CAPN1a (SEQ ID NO: 1/2);
(iii) MYOD1b (SEQ ID NO: 3/4);
(iv) MYF5 (SEQ ID NO: 5/6);
(v) CAPN11 (SEQ ID NO: 9);
(vi) CAPN3 (SEQ ID NO: 10/11);
(vii) DY714774 (SEQ ID NO: 12); und
(viii) AGKD01012360 (SEQ ID NO: 13);
wobei das Vorhandensein von Variationen in den Nucleinsäure- oder Aminosäuresequenzen, die eine beliebige der vorgenannten (i) bis (viii) codieren, im Zusammenhang mit verbesserten Merkmalen steht, wahlweise wobei (a) das verbesserte Merkmal relativ zu dem entsprechenden Merkmal in einer Kontrollprobe von Fisch oder Fleisch oder durch Bezug auf das entsprechende Merkmal in einer nichtselektierten Population von Fisch bewertet wird; und
(b) die Sequenzvariation relativ zu der Sequenz einer Referenzsequenz bestimmt wird.

2. Verfahren für die Selektion von Fisch aus Spezies in der Familie der *Salmonidae* mit einer größeren Ausbeute an Fleisch, wobei das Verfahren in einer Probe die Schritte des Identifizierens von Sequenzvariationen innerhalb der Nucleinsäure- oder Aminosäuresequenzen umfasst, die ein oder mehrere Proteine/Sequenzen codieren, die ausgewählt sind aus der Gruppe, bestehend aus:
(a) CAST2;
(b) MYOD1b;
(c) CAPN11; und
(d) CAPN3;
wobei das Vorhandensein von Sequenzvariationen in den Nucleinsäure- oder Aminosäuresequenzen, die eine der vorgenannten (a) bis (d) codieren, im Zusammenhang mit einer größeren Ausbeute an Fleisch steht, wahlweise wobei (a) die größere Ausbeute an Fleisch relativ zu der entsprechenden Ausbeute an Fleisch in einer Kontrollprobe von Fleisch oder Fisch oder durch Bezugnahme auf die entsprechende Ausbeute an Fleisch in einer nichtselektierten Population von Fisch bewertet wird; und
(b) die Sequenzvariation relativ zu der Sequenz einer Referenzsequenz bestimmt wird.

3. Verfahren nach Anspruch 1, wobei das Verfahren das Identifizieren des Vorhandenseins von Sequenzvariationen innerhalb der Nucleinsäure- und/oder Aminosäuresequenz umfasst, die CAST2 (SEQ ID NO: 7/8) codieren, wobei das Verfahren wahlweise ferner das Identifizieren des Vorhandenseins von Sequenzvariationen innerhalb der Nucleinsäure- und/oder Aminosäuresequenz umfasst, die ein oder mehrere Proteine codieren, die ausgewählt sind aus der Gruppe, bestehend aus:
(i) CAPN1a (SEQ ID NO: 1/2);
(ii) MYOD1b (SEQ ID NO: 3/4);
(iii) MYF5 (SEQ ID NO: 5/6);
(iv) CAPN11 (SEQ ID NO: 9);
(v) CAPN3 (SEQ ID NO: 10/11);
(vi) DY714774 (SEQ ID NO: 12); und
(vii) AGKD01012360 (SEQ ID NO: 13).

4. Verfahren nach Anspruch 1 oder 3, wobei die verbesserten Merkmale eines oder mehrere sind, die ausgewählt sind aus der Gruppe, bestehend aus:
(i) verbesserter Filet-Ausbeute;
(ii) verbesserter Fleischtextur;
(iii) verbessertem Fleischnährwert;
(iv) verbessertem reduzierten Gaping;
(vi) verbesserter Fleisch-Zugfestigkeit; und
(vii) verbesserter Fleisch-Festigkeit.

5. Verfahren für die Selektion von Fisch aus Spezies in der Familie der *Salmonidae* mit Fleisch, das verbesserte Zugfestigkeit zeigt, wobei das Verfahren in einer Probe die Schritte zum Identifizieren von Sequenzvariationen innerhalb der Nucleinsäure- oder Aminosäuresequenzen umfasst, die ein oder mehrere Proteine codieren, die ausgewählt sind aus der Gruppe, bestehend aus:
(a) CAPN1;
(b) DY714774; und
(c) AGKD01012360;
wobei das Vorhandensein von Sequenzvariationen in den Nucleinsäure- oder Aminosäuresequenzen, die eine beliebige der vorgenannten (a) bis (c) codieren, im Zusammenhang mit Fleisch steht, das verbesserte Zugfestigkeit zeigt.

6. Verfahren für die Selektion von Fisch aus Spezies in der Familie der *Salmonidae* mit Fleisch, das verbesserte Festigkeit zeigt, wobei das Verfahren in einer Probe die Schritte zum Identifizieren von Sequenzvariationen innerhalb der Nucleinsäure- oder Aminosäuresequenzen umfasst, die ein oder mehrere Proteine codieren, die ausgewählt sind aus der Gruppe, bestehend aus:
(a) CAPN3;
(b) DY714774; und
(c) AGKD01012360,
wobei das Vorhandensein von Sequenzvariationen in den Nucleinsäure- oder Aminosäuresequenzen, die eine beliebige der vorgenannten (a) bis (c) codieren, im Zusammenhang mit Fleisch steht, das verbesserte Festigkeit zeigt.

7. Verfahren nach einem der vorgenannten Ansprüche, wobei die Sequenzvariation einen oder mehrere Polymorphismen umfasst, wahlweise wobei die Sequenzvariationen ausgewählt sind aus der Gruppe, bestehend aus:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a: Ser39Leu;
(ix) CAPN11 611 C>A;
(x) CAPN3: 1701 C>G;
(xi) CAPN3: Thr541Arg;
(xii) DY714774 251 C>T; und
(xiii) AGKD01012360 520 C>A.

8. Verfahren für die Selektion von Fisch aus Spezies in der Familie der *Salmonidae* mit einer größeren Ausbeute an Fleisch, wobei das Verfahren in einer Probesequenz die Schritte des Identifizierens von einem oder mehreren der nachfolgend als (i), (ii), (iii), (iv) und (v) gegebenen Sequenzvariationen umfasst:
(i)
(a) CAST2 234 G>T; oder
(b) CAST2 Gly45Val;
und
(a) MYOD1b: 829 G>A; oder
(b) MYOD1b: Cys243Tyr;
(ii)
(a) CAST2 234 G>T; oder
(b) CAST2 Gly45Val;
und
(a) CAPN11 611 C>A;
(iii)
(a) MYOD1b: 829 G>A; oder
(b) MYOD1b: Cys243Tyr;
und
(a) CAPN11 611 C>A;
(iv)
(a) CAPN3 1701 C>G; oder
(b) CAPN3: Thr541Arg;
und
(a) CAPN11 611 C>A;
(v)
(a) MYOD1b: 829 G>A; oder
(b) MYOD1b: Cys243Tyr;
und
(a) CAPN3 1701 C>G; oder
(b) CAPN3 Thr541Arg;
wobei Detektion von einer oder mehreren Sequenzvariationen (i), (ii), (iii), (iv) oder, und/oder (v) in einer Probe, anzeigt, dass die Probe bereitgestellt wurde durch oder erhalten wurde aus einem Fisch mit einer größeren Ausbeute an Fleisch, wobei wahlweise die größere Ausbeute an Fleisch relativ zu einem Fisch bewertet wird, dem eine oder mehrere von Sequenzvariationen (i) (ii), (iii), (iv) oder (v) fehlen.

9. Verfahren für die Selektion von Fisch aus Spezies in der Familie der *Salmonidae* mit Fleisch, das verbesserte Zugfestigkeit zeigt, wobei das Verfahren in einer Probe die Schritte des Identifizierens einer oder mehreren der als (i) und (ii) gegebenen Sequenzvariationen umfasst:
(i)
(a) CAPN11 611 C>A
und
(a) DY714774 (TAG4083) 251 C>T
(ii)
(a) AGKD01012360 (TAG49325) 520 C>A
und
(a) DY714774 (TAG4083) 251 C>T,
wobei Detektion der Sequenzvariationen (i) und/oder (ii) in einer Probe anzeigt, dass die Probe bereitgestellt wurde durch oder erhalten wurde aus einem Fisch mit Fleisch, das verbesserte Zugfestigkeit zeigt.

10. Verfahren für die Selektion von Fisch aus Spezies in der Familie der *Salmonidae* mit Fleisch, das verbesserte Festigkeit zeigt, wobei das Verfahren in einer Probe die Schritte des Identifizierens einer oder mehrerer Sequenzvariationen umfasst:
(i)
(a) AGKD01012360 (TAG49325) 520 C>A;
und
(a) CAPN3 1701 C>G;
(b) CAPN3 Thr541Arg;
wobei Detektion der Sequenzvariationen in einer Probe anzeigt, dass die Probe bereitgestellt wurde durch oder erhalten wurde aus einem Fisch mit Fleisch, das verbesserte Festigkeit zeigt.

11. Verfahren zum Identifizieren von Zuchtfisch aus Spezies in der Familie der *Salmonidae,* wobei das Verfahren die Schritte des Bereitstellens einer Probe von einem Fisch und Identifizierens des Vorhandenseins von Sequenzvariationen innerhalb der Nucleinsäure- oder Aminosäuresequenzen umfasst, die ein oder mehrere Proteine codieren, die ausgewählt sind aus der Gruppe, bestehend aus:
(i) CAST2 (SEQ ID NO: 7/8);
(ii) CAPN1a (SEQ ID NO: 1/2);
(iii) MYOD1b (SEQ ID NO: 3/4);
(iv) MYF5 (SEQ ID NO: 5/6);
(vi) CAPN11 (SEQ ID NO: 9);
(vi) CAPN3 (SEQ ID NO: 10/11;
(vii) DY714774 (SEQ ID NO: 12); und
(viii) AGKD01012360 (SEQ ID NO: 13);
wobei der Fisch, von dem nachgewiesen wurde, dass er ein oder mehrere Variationen in den Nucleinsäure- und/oder Aminosäuresequenzen besitzt, die eine oder mehrere der vorstehend als (i) bis (viii) identifizierten Proteine/Sequenzen codieren, für die Selektion als Zuchtmaterial geeignet ist.

12. Verfahren nach einem der vorgenannten Ansprüche, wobei der Fisch Wildfisch oder Zuchtfisch ist, wahlweise wobei die Spezies
*Salmo salar* (der Atlantische Lachs) ist.

13. Verfahren nach einem der vorgenannten Ansprüche, wobei die Probe bereitgestellt wird durch oder erhalten wird aus einem zu testenden Fisch und Nucleinsäure und/oder Peptide und/oder Proteine aufweist, wahlweise wobei die Probe eines oder mehrere aufweist, die ausgewählt sind aus der Gruppe, bestehend aus:
(i) einer Gewebebiopsie (mit Zellen und anderem Material);
(ii) einem Abstrich oder Tupfer;
(iii) biologischem Fluid;
(iv) einem Gewebe- und/oder Drüsensekret; und
(v) embryonalem Material.

14. Verfahren für die Selektion von Fisch aus Spezies in der Familie der *Salmonidae* mit verbesserten Merkmalen, wobei das Verfahren in einer Probe den Schritt des Identifizierens eines Pegels der Expression, Funktion und/oder Aktivität von einem oder mehreren Proteinen umfasst, die ausgewählt sind aus der Gruppe, bestehend aus:
(i) CAPN1a;
(ii) MYOD1b;
(iii) MYF5;
(iv) CAST2; und
(v) CAPN11;
(vi) CAPN3;
(vii) DY7I4774;
(viii) AGKD01012360;
wobei jede beliebige Modulation in dem Expressions-, Funktions- und/oder Aktivitätspegel eines Proteins anzeigen kann, dass die Probe von einem Fisch bereitgestellt wurde, welcher verbesserte Merkmale zeigt.

15. Kit zum Identifizieren und/oder zum Bestimmen, ob oder nicht ein mit varianter Fleischqualität assoziiertes Gen/Protein in einer Nucleinsäure-Probe vorhanden ist oder fehlt, die von einer Fischspezies aus innerhalb der Familie der *Salmonidae* bereitgestellt wurde, wobei das Kit ein oder mehrere Paare von Oligonucleotid-Primern aufweist, die für das Amplifizieren einer Nucleotidsequenz von Interesse nutzbar sind, sowie eine oder mehrere Komponenten, die ausgewählt sind aus der Gruppe, bestehend aus:
(i) ein Nucleinsäure polymerisierendes Mittel;
(ii) Kette verlängernde Nucleotide;
(iii) Reaktions- und/oder Speicherpuffer;
(iv) Ampullen oder andere Aufbewahrungs- oder Reaktionsbehälter; und
(v) Gebrauchsanweisungen;
wobei die Nucleotidsequenz, die von Interesse ist, eine variante CAST2-, MYF5,-MYOD1b-, CAPN1a-, CAPN11-, CAPN3-, DY714774- und/oder AGKD01012360-Sequenz umfasst, wobei wahlweise das eine oder die mehreren Paare von Oligonucleotid-Primern zum Amplifizieren einer varianten Sequenz dienen, die ausgewählt ist aus der Gruppe, bestehend aus:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a: Ser39Leu;
(ix) CAPN11 611 C>A;
(x) CAPN3: 1701 C>G;
(xi) CAPN3: Thr541Arg;
(xii) DY714774 251 C>T; und
(xiii) AGKD01012360 520 C>A.

## Revendications

1. Méthode pour la sélection de poissons à partir d'une espèce dans la famille *Salmonidae* avec un ou plusieurs traits améliorés, ladite méthode comprenant l'étape d'identification dans un échantillon de la présence de variations de séquences dans les séquences d'acide nucléique ou d'acides aminés codant pour une ou plusieurs des protéines/séquences choisies dans le groupe constitué de:
(i) CAST2 (SEQ ID NO: 7/8);
(ii) CAPN1a (SEQ ID NO: 1/2);
(iii) MYOD1b (SEQ ID NO: 3/4);
(iv) MYF5 (SEQ ID NO: 5/6);
(v) CAPN11 (SEQ ID NO: 9);
(vi) CAPN3 (SEQ ID NO: 10/11);
(vii) DY714774 (SEQ ID NO: 12); et
(viii) AGKD01012360 (SEQ ID NO: 13);
dans laquelle la présence de variations dans les séquences d'acide nucléique ou d'acides aminés, codant pour l'une quelconque des (i)-(viii) ci-dessus, est associée à des traits améliorés, optionnellement dans laquelle (a) le trait amélioré est évalué relativement au trait correspondant dans un échantillon de poisson ou de chair témoin ou en référence au trait correspondant dans une population de poissons non sélectionnée; et
(b) la variation de séquence est déterminée relativement à la séquence d'une séquence de référence.

2. Méthode pour la sélection de poissons à partir d'une espèce dans la famille *Salmonidae* possédant un rendement de chair supérieur, ladite méthode comprenant les étapes d'identification dans un échantillon de variations de séquences dans les séquences d'acide nucléique ou d'acides aminés codant pour une ou plusieurs protéines choisies dans le groupe constitué de:
(a) CAST2;
(b) MYOD1b;
(c) CAPN11; et
(d) CAPN3;
dans laquelle la présence de variations de séquences dans les séquences d'acide nucléique ou d'acides aminés, codant pour l'une quelconque des (a)-(d) ci-dessus, est associée à un rendement de chair supérieur, optionnellement dans laquelle (a) le rendement de chair supérieur est évalué relativement au rendement de chair correspondant dans un échantillon de poisson ou de chair témoin ou en référence au rendement de chair correspondant dans une population de poissons non sélectionnée; et
(b) la variation de séquence est déterminée relativement à la séquence d'une séquence de référence.

3. Méthode selon la revendication 1, où la méthode comprend l'identification de la présence de variations de séquences dans la séquence d'acide nucléique et/ou d'acides aminés codant pour CAST2 (SEQ ID NO: 7/8), optionnellement où la méthode comprend en outre l'identification de la présence de variations de séquences dans les séquences d'acide nucléique et/ou d'acides aminés codant pour une ou plusieurs protéines choisies dans le groupe constitué de:
(i) CAPN1a (SEQ ID NO: 1/2);
(ii) MYOD1b (SEQ ID NO: 3/4);
(iii) MYF5 (SEQ ID NO: 5/6);
(iv) CAPN11 (SEQ ID NO: 9);
(v) CAPN3 (SEQ ID NO: 10/11);
(vi) DY714774 (SEQ ID NO: 12); et
(vii) AGKD01012360 (SEQ ID NO: 13).

4. Méthode selon les revendications 1 à 3, dans laquelle les traits améliorés sont un ou plusieurs choisis dans le groupe constitué de:
(i) rendement de filet amélioré;
(ii) texture de chair améliorée;
(iii) nutrition de chair améliorée;
(iv) émiettement réduit amélioré;
(vi) résistance à la traction de chair améliorée; et
(vii) fermeté de chair améliorée.

5. Méthode pour la sélection de poissons à partir d'une espèce dans la famille *Salmonidae* possédant une chair affichant une résistance à la traction améliorée, ladite méthode comprenant les étapes d'identification dans un échantillon de variations de séquences dans les séquences d'acide nucléique ou d'acides aminés codant pour une ou plusieurs protéines choisies dans le groupe constitué de:
(a) CAPN1;
(b) DY714774; et
(c) AGKD01012360;
dans laquelle la présence de variations de séquences dans les séquences d'acide nucléique ou d'acides aminés, codant pour l'une quelconque des (a)-(c) ci-dessus, est associée à une chair affichant une résistance à la traction améliorée.

6. Méthode pour la sélection de poissons à partir d'une espèce dans la famille *Salmonidae* possédant une chair affichant une fermeté améliorée, ladite méthode comprenant les étapes d'identification dans un échantillon de variations de séquences dans les séquences d'acide nucléique ou d'acides aminés codant pour une ou plusieurs protéines choisies dans le groupe constitué de:
(a) CAPN3;
(b) DY714774; et
(c) AGKD01012360;
dans laquelle la présence de variations de séquences dans les séquences d'acide nucléique ou d'acides aminés, codant pour l'une quelconque des (a)-(c) ci-dessus, est associée à une chair affichant une fermeté améliorée.

7. Méthode selon l'une quelconque des revendications précédentes, dans laquelle la variation de séquence comprend un ou plusieurs polymorphismes, optionnellement dans laquelle les variations de séquences sont choisies dans le groupe constitué de:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a: Ser39Leu;
(ix) CAPN11 611 C>A;
(x) CAPN3: 1701 C>G;
(xi) CAPN3: Thr541Arg;
(xii) DY714774 251 C>T; et
(xiii) AGKD01012360 520 C>A.

8. Méthode pour la sélection de poissons à partir d'une espèce dans la famille *Salmonidae* possédant un rendement de chair supérieur, ladite méthode comprenant les étapes d'identification dans une séquence d'échantillon d'une ou de plusieurs des variations de séquences données comme (i), (ii), (iii), (iv) et (v) ci-dessous:
(i)
(a) CAST2 234 G>T; ou
(b) CAST2 Gly45Val;
et
(a) MYOD1b: 829 G>A; ou
(b) MYOD1b: Cys243Tyr;
(ii)
(a) CAST2 234 G>T; ou
(b) CAST2 Gly45Val;
et
(a) CAPN11 611 C>A;
(iii)
(a) MYOD1b: 829 G>A; ou
(b) MYOD1b: Cys243Tyr;
et
(a) CAPN11 611 C>A;
(iv)
(a) CAPN3 1701 C>G; ou
(b) CAPN3: Thr541Arg;
et
(a) CAPN11 611 C>A;
(v)
(a) MYOD1b: 829 G>A; ou
(b) MYOD1b: Cys243Tyr;
et
(a) CAPN3 1701 C>G; ou
(b) CAPN3 Thr541Arg;
dans laquelle la détection d'une ou de plusieurs variations de séquences (i), (ii), (iii), (iv) ou et/ou (v) dans un échantillon indique que l'échantillon a été fourni par ou obtenu à partir d'un poisson possédant un rendement de chair supérieur, optionnellement dans laquelle le rendement de chair supérieur est évalué relativement à un poisson dénué d'une ou de plusieurs variations de séquences (i), (ii), (iii), (iv) ou (v).

9. Méthode pour la sélection de poissons à partir d'une espèce dans la famille *Salmonidae* possédant une chair affichant une résistance à la traction améliorée, ladite méthode comprenant les étapes d'identification dans un échantillon d'une ou de plusieurs des variations de séquences données comme (i) et (ii):
(i)
(a) CAPN11 611 C>A;
et
(a) DY714774 (TAG4083) 251 C>T;
(ii)
(a) AGKD01012360 (TAG49325) 520 C>A;
et
(a) DY714774 (TAG4083) 251 C>T;
dans laquelle la détection de variations de séquences (i) et/ou (ii) dans un échantillon indique que l'échantillon a été fourni par ou obtenu à partir d'un poisson avec une chair affichant une résistance à la traction améliorée.

10. Méthode pour la sélection de poissons à partir d'une espèce dans la famille *Salmonidae* avec une chair affichant une fermeté améliorée, ladite méthode comprenant les étapes d'identification dans un échantillon des variations de séquences:
(i)
(a) AGKD01012360 (TAG49325) 520 C>A;
et
(a) CAPN3 1701 C>G;
(b) CAPN3 Thr541Arg;
dans laquelle la détection de ces variations de séquences dans un échantillon indique que l'échantillon a été fourni par ou obtenu à partir d'un poisson avec une chair affichant une fermeté améliorée.

11. Méthode pour l'identification de poissons reproducteurs à partir d'une espèce dans la famille *Salmonidae,* ladite méthode comprenant les étapes de fourniture d'un échantillon provenant d'un poisson et d'identification de la présence de variations de séquences dans les séquences d'acide nucléique ou d'acides aminés codant pour une ou plusieurs des protéines choisies dans le groupe constitué de:
(i) CAST2 (SEQ ID NO: 7/8);
(ii) CAPN1a (SEQ ID NO: 1/2);
(iii) MYOD1b (SEQ ID NO: 3/4);
(iv) MYF5 (SEQ ID NO: 5/6);
(v) CAPN11 (SEQ ID NO: 9);
(vi) CAPN3 (SEQ ID NO: 10/11);
(vii) DY714774 (SEQ ID NO: 12); et
(viii) AGKD01012360 (SEQ ID NO: 13);
dans laquelle les poissons identifiés comme possédant une ou plusieurs variations dans les séquences d'acide nucléique et/ou d'acides aminés, codant pour une ou plusieurs des protéines/séquences identifiées comme (i)-(viii) ci-dessus, sont appropriés pour une sélection en tant que reproducteurs.

12. Méthode selon l'une quelconque des revendications précédentes, dans laquelle les poissons sont des poissons sauvages ou d'élevage, optionnellement dans laquelle l'espèce est:
*Salmo salar* (le saumon de l'Atlantique).

13. Méthode selon l'une quelconque des revendications précédentes, dans laquelle l'échantillon est fourni par ou obtenu à partir d'un poisson à tester et comprend un acide nucléique et/ou des peptides et/ou des protéines, optionnellement dans laquelle l'échantillon comprend un ou plusieurs choisis dans le groupe constitué de:
(i) une biopsie de tissu (comprenant des cellules et un autre matériau),
(ii) un grattage ou un écouvillon;
(iii) un fluide biologique;
(iv) une sécrétion tissulaire et/ou glandulaire; et
(v) un matériau embryonnaire.

14. Méthode pour la sélection de poissons à partir d'une espèce dans la famille *Salmonidae* avec des traits améliorés, ladite méthode comprenant l'étape d'identification dans un échantillon d'un niveau d'expression, d'une fonction et/ou d'une activité d'une ou de plusieurs des protéines choisies dans le groupe constitué de:
(i) CAPN1a;
(ii) MYOD1b;
(iii) MYF5;
(iv) CAST2; et
(v) CAPN11;
(vi) CAPN3;
(vii) DY714774;
(viii) AGKD01012360;
dans laquelle toute modulation dans le niveau d'expression, la fonction et/ou l'activité d'une protéine peut indiquer que l'échantillon a été fourni par un poisson affichant des traits améliorés.

15. Kit pour l'identification et/ou la détermination si oui ou non un gène/protéine associé(e) à une qualité de chair variante est présente ou absente dans un échantillon d'acide nucléique fourni par une espèce de poisson dans la famille *Salmonidae,* ledit kit comprenant une ou plusieurs paires d'amorces oligonucléotidiques utiles pour l'amplification d'une séquence de nucléotides intéressante et un ou plusieurs composants choisis dans le groupe constitué de:
(i) un agent de polymérisation d'acide nucléique;
(ii) des nucléotides d'allongement de chaîne;
(iii) des tampons de réaction et/ou de stockage;
(iv) des flacons ou d'autres récipients de stockage/réaction; et
(v) des instructions pour l'utilisation;
dans lequel la séquence de nucléotides intéressante comprend une séquence CAST2, MYF5, MYOD1b, CAPN1a, CAPN11, CAPN3, DY714774 et/ou AGKD01012360 variante, optionnellement dans lequel les une ou plusieurs paires d'amorces oligonucléotidiques sont pour l'amplification d'une séquence variante choisie dans le groupe constitué de:
(i) CAST2: 134 G>T;
(ii) CAST2: Gly45Val;
(iii) MYF5 578-9 AC>GT;
(iv) MYF5 Asn193Ser;
(v) MYOD1b: 829 G>A;
(vi) MYOD1b: Cys243Tyr;
(vii) CAPN1a: 561 C>T;
(viii) CAPN1a: Ser39Leu;
(ix) CAPN11 611 C>A;
(x) CAPN3: 1701 C>G;
(xi) CAPN3: Thr541Arg;
(xii) DY714774 251 C>T; et
(xiii) AGKD01012360 520 C>A.


### SEQUENCE LISTING

<110> University Court of The University of St Andrews
   Johnston, Ian A
   Ashton, Thomas J
<120> Fish Selection
<130> PG443193WO
<160> 170
<170> PatentIn version 3.3
<210> 1
   <211> 3738
   <212> DNA
   <213> Salmo salar
<400> 1
   acaaaacaac acattgaacc gctaaacaac caccaaaagg ggctttggtg ggacaacaag      60
   gtacagccaa ctcgctgagt ctcacctcat tctattggct tctgagccct gtctgctaaa     120
   ggtacagcca gccaatcagt gaactgttgc cgtgactctg agatctgcct ggtgctgtga     180
   agaagacgtt ttccttctga gcccatcctc cagtccccac tctaccactt tttacatgac     240
   taggactgtc actgatctcc attaagcctg tcacagccta ttatcactac ctcatctaca     300
   attctaacct cataattggc tgttgctgtt ctatgtctga ttcctaattg aacgtcagcc     360
   attaacctcc tccctgactg gctccctgga ttagctttga actttgacct ctgccacagc     420
   caaatacaac tacggccagg tgagaatgga gcaggtgtat gcgtcgggga tggccgccaa     480
   gttgcgcagc cagtgggacc gggacgaggg tctgggccag aaccacaacg cagtcaagtt     540
   ccagggccag gactttgagt cgctgagggc ccggtgcctc cagagccgca gcctgtttga     600
   ggacagcctg ttcatctgcg cctcctcctc tctggggttc aatgagttgg gaccacgctc     660
   ctccaagacc cagggagtac gctggatgag acctacggag atctgtaccc gtccccagtt     720
   catagtagat ggagccactc gcactgatat ctgccaggga gctttaggtg actgctggtt     780
   gctggcagcc atagcctcgt tgactctgaa cgacaacctc ctccacagag tggtgcctca     840
   tggacaggac ttcggaggcc agtacgctgg catcttccac ttccagttct ggcagtatgg     900
   ggagtgggtg gaggtggtga tcgatgacag gctgccggtg aaggatggga agctactctt     960
   cgtccactct gccgagggag gagaattctg gagcgccctg ctggagaaag cctacgccaa    1020
   gctgcatggc tgttacgagg ccctgtcggg aggcagcacg tcagagggtt ttgaggactt    1080
   cacaggaggg gtaacagaga tgtatgagct gaggaaagcg ccctctgacc tctacagtat    1140
   catcagtcgg gctgtggaga gaggatcgct gctgggctgc tccatagaca tcaccggtag    1200
   tcaggacatg gaggctgtga cctttaagaa gctggtgaaa ggtcacgcct actcagtgac    1260
   cggggttgac gaggtggtgt acagggggaa tatgaccaaa ctggtccgca tccgcaaccc     1320
   ctggggagag atagagtgga ctggtgcctg gagtgacaac tccagagagt gggagagcgt   1380
   ggaccggtca gtcagagggc gactccagaa ccgcagtgag gatggagagt tctggatgtc   1440
   attcagcgac ttcctgcgtg agtttacgcg tctggagatc tgtaacctca cagcagacgc   1500
   cctggaggcc aaccagcaga agaagtggag ctcagccgtc taccaggggg agtggaggag   1560
   gggcagcacg gccgggggct gcaggaactt ccctgccaca ttctggatca acccacaatt   1620
   caagatcggc ttgcagcacc ccgacacagc cggccaatca gattgcagct tcctggtggc   1680
   gctgatgcag aaggaccgga gaaagaaacg caaggagggc aaggacatgg agaccatcgg   1740
   attcgccatt tacgaggttc ctaatgagta tgtgggtaga tcaggcatcc acctgaagag   1800
   agatttcttc ctgacgcacg gttccagtgc ccgctctgag ctcttcatca acctgagaga   1860
   ggtgagctct cgcttccagc tgcccgccgg ggagtacatc atcgtccctt ccaccttcga   1920
   accccagaag gagggagact ttgtcctcag agtcttctct gagaaaccag ctaactcgga   1980
   ggagctggat gatgacgtca cagctgaact tcctgcagag tctcagctgg aggagagcca   2040
   gattgatgct ggcttcaaga gtctgttcag acagctggct ggagaggaca tggagatcag   2100
   tgccacagag ctgcagacca tactcaacag gatcataagc aagcacaaag atctgaagac   2160
   agatggattt ggaccagaag cttgtcgcac tatgatcaac ctgatggata ctgatggtag   2220
   tggtaaactg ggactggcag agttccacgt gctgtgggag aaaattaaac gatacctgac   2280
   tgtattccgt cagtttgacc tggacaagtc aggcaccatg agttcctatg agatgaggat   2340
   ggccctggag gcggcaggtt tcaagctgac gaaccacctg ttccagctga taattctgcg   2400
   ctacactgag gctgacctga cagttgactt tgacaacttt gttacctgcc tggttaggct   2460
   ggagaccatg ttcaaaacct tcaagacgat ggacacagat tctgatggag tgatagaact   2520
   caacttcttc cagtggatca ccctgaccat gtttgcctaa agatgatgga ctcactgatg   2580
   ttattgcacc agactacact aatgtaattt cagaagtgcc tttctctggc tcttcctctc   2640
   tgcactgcag agatcaaaaa aaaaacttga gtaatgttta caacatttaa tatggggatc   2700
   atgtgccaaa gcattgcctg ttaccttaga aacagaggga gacaactcta ctcacatggc   2760
   gccacaacat agaggaagac ggaagggcat tttgatactc cctccaattg gataggaagg   2820
   aaacttccta aagtttttgt tttacatctt gtgccaattt tcacaattgc cttgtcatat   2880
   tttcaacgtt ccttttggaa gccattttgg ggcctgtgtt tcaggaatag gcaatggggg   2940
   tgttggtatg gagtttgggt caaaaggatt ttctctgctg agaaagaagg caaagtgctg   3000
   ttgtaatttg gaagtacttc atcatcctta gaggatcgaa tcaccggcat gacaattttt   3060
   atttagaagt cgtatatgtc agtacttttc agtgggaaaa cccccacaat atccttgtaa   3120
   tgccgcatac tcaaaacttg atatcctcat tttgaatgga tgccaaagac acttcaagga   3180
   tatcccagct ttttatgtta attttttttg tgtgttccac ggggaacatg aaactaccca 3240
   ctgggcattg acgtcaattc cacgttggtt caacgtaatt tcattgaaat gacatggaaa 3300
   caacattgat ccaaccagtg tgtgcccagt ggatatttta tttttatttt agtatttgtg 3360
   atgtaggtta taagtgatga cagatttttg attggaatag caggcatcaa cctgatcgca 3420
   tgtaatgtca gttgtattca tggatgaagg aaggtaatgt gcagttggag acattttgcc 3480
   aaacagactg agggaaccag aaacagtatg acactcttat tgccatgaca ttcaattcta 3540
   tagttgccat ttttctgtgt agattgataa taaaatgtct tatatgcatt atgcaatcac 3600
   gactgttgtt tacagtgtac tctggaattg tgttatgctc tctcttatta tggaattatg 3660
   taccttttcc atttctatct atacaaaact tcaataaact ttttctgaac acaaaaaaaa 3720
   aaaaaaaaaa aaaaaaga 3738
<210> 2
   <211> 703
   <212> PRT
   <213> Salmo salar
<400> 2
   Met Glu Gln Val Tyr Ala Ser Gly Met Ala Ala Lys Leu Arg Ser Gln
   1 5 10 15
   Trp Asp Arg Asp Glu Gly Leu Gly Gln Asn His Asn Ala Val Lys Phe
   20 25 30
   Gln Gly Gln Asp Phe Glu Ser Leu Arg Ala Arg Cys Leu Gln Ser Arg
   35 40 45
   Ser Leu Phe Glu Asp Ser Leu Phe Ile Cys Ala Ser Ser Ser Leu Gly
   50 55 60
   Phe Asn Glu Leu Gly Pro Arg Ser Ser Lys Thr Gln Gly Val Arg Trp
   65 70 75 80
   Met Arg Pro Thr Glu Ile Cys Thr Arg Pro Gln Phe Ile Val Asp Gly
   85 90 95
   Ala Thr Arg Thr Asp Ile Cys Gln Gly Ala Leu Gly Asp Cys Trp Leu
   100 105 110
   Leu Ala Ala Ile Ala Ser Leu Thr Leu Asn Asp Asn Leu Leu His Arg
   115 120 125
   Val Val Pro His Gly Gln Asp Phe Gly Gly Gln Tyr Ala Gly Ile Phe
   130 135 140
   His Phe Gln Phe Trp Gln Tyr Gly Glu Trp Val Glu Val Val Ile Asp
   145 150 155 160
   Asp Arg Leu Pro Val Lys Asp Gly Lys Leu Leu Phe Val His Ser Ala
   165 170 175
   Glu Gly Gly Glu Phe Trp Ser Ala Leu Leu Glu Lys Ala Tyr Ala Lys
   180 185 190
   Leu His Gly Cys Tyr Glu Ala Leu Ser Gly Gly Ser Thr Ser Glu Gly
   195 200 205
   Phe Glu Asp Phe Thr Gly Gly Val Thr Glu Met Tyr Glu Leu Arg Lys
   210 215 220
   Ala Pro Ser Asp Leu Tyr Ser Ile Ile Ser Arg Ala Val Glu Arg Gly
   225 230 235 240
   Ser Leu Leu Gly Cys Ser Ile Asp Ile Thr Gly Ser Gln Asp Met Glu
   245 250 255
   Ala Val Thr Phe Lys Lys Leu Val Lys Gly His Ala Tyr Ser Val Thr
   260 265 270
   Gly Val Asp Glu Val Tyr Arg Gly Asn Met Thr Lys Leu Val Arg Ile
   275 280 285
   Arg Asn Pro Trp Gly Glu Ile Glu Trp Thr Gly Ala Trp Ser Asp Asn
   290 295 300
   Ser Arg Glu Trp Glu Ser Val Asp Arg Ser Val Arg Gly Arg Leu Gln
   305 310 315 320
   Asn Arg Ser Glu Asp Gly Glu Phe Trp Met Ser Phe Ser Asp Phe Leu
   325 330 335
   Arg Glu Phe Thr Arg Leu Glu Ile Cys Asn Leu Thr Ala Asp Ala Leu
   340 345 350
   Glu Ala Asn Gln Gln Lys Lys Trp Ser Ser Ala Val Tyr Gln Gly Glu
   355 360 365
   Trp Arg Arg Gly Ser Thr Ala Gly Gly Cys Arg Asn Phe Pro Ala Thr
   370 375 380
   Phe Trp Ile Asn Pro Gln Phe Lys Ile Gly Leu Gln His Pro Asp Thr
   385 390 395 400
   Ala Gly Gln Ser Asp Cys Ser Phe Leu Val Ala Leu Met Gln Lys Asp
                   405                 410                 415
   Arg Arg Lys Lys Arg Lys Glu Gly Lys Asp Met Glu Thr Ile Gly Phe
               420                 425                 430
   Ala Ile Tyr Glu Val Pro Asn Glu Tyr Val Gly Arg Ser Gly Ile His
           435                 440                 445
   Leu Lys Arg Asp Phe Phe Leu Thr His Gly Ser Ser Ala Arg Ser Glu
       450                 455                 460
   Leu Phe Ile Asn Leu Arg Glu Val Ser Ser Arg Phe Gln Leu Pro Ala
   465                 470                 475                 480
   Gly Glu Tyr Ile Ile Val Pro Ser Thr Phe Glu Pro Gln Lys Glu Gly
                   485                 490                 495
   Asp Phe Val Leu Arg Val Phe Ser Glu Lys Pro Ala Asn Ser Glu Glu
               500                 505                 510
   Leu Asp Asp Asp Val Thr Ala Glu Leu Pro Ala Glu Ser Gln Leu Glu
           515                 520                 525
   Glu Ser Gln Ile Asp Ala Gly Phe Lys Ser Leu Phe Arg Gln Leu Ala
       530                 535                 540
   Gly Glu Asp Met Glu Ile Ser Ala Thr Glu Leu Gln Thr Ile Leu Asn
   545                 550                 555                 560
   Arg Ile Ile Ser Lys His Lys Asp Leu Lys Thr Asp Gly Phe Gly Pro
                   565                 570                 575
   Glu Ala Cys Arg Thr Met Ile Asn Leu Met Asp Thr Asp Gly Ser Gly
               580                 585                 590
   Lys Leu Gly Leu Ala Glu Phe His Val Leu Trp Glu Lys Ile Lys Arg
           595                 600                 605
   Tyr Leu Thr Val Phe Arg Gln Phe Asp Leu Asp Lys Ser Gly Thr Met
       610                 615                 620
   Ser Ser Tyr Glu Met Arg Met Ala Leu Glu Ala Ala Gly Phe Lys Leu
   625                 630                 635                 640
   Thr Asn His Leu Phe Gln Leu Ile Ile Leu Arg Tyr Thr Glu Ala Asp
   645 650 655
   Leu Thr Val Asp Phe Asp Asn Phe Val Thr Cys Leu Val Arg Leu Glu
   660 665 670
   Thr Met Phe Lys Thr Phe Lys Thr Met Asp Thr Asp Ser Asp Gly Val
   675 680 685
   Ile Glu Leu Asn Phe Phe Gln Trp Ile Thr Leu Thr Met Phe Ala
   690 695 700
<210> 3
   <211> 1319
   <212> DNA
   <213> Salmo salar
<400> 3
   tctaacgttt gtgacaaata cagagccagg attacactcg ttacagggct ggtcttagaa     60
   tttgacagtg agatggagtt gtcggatatt tcgttccctg tcacctctgc tgatgacttt    120
   tatgacgacc cgtgcttcaa caccagcgac atgcatttct tcgaggacct ggacccccgg    180
   ttagtccacg tgggactcct caagccagac gaccaccatt acaacgaaga cgaacacatc    240
   agggcaccga gcgggcacca tcaagccggc aggtgcctcc tctgggcatg taaagcttgc    300
   aagaggaaaa ccacaaactc tgaccggagg aaggctgcta ccatgcggga gaggaggagg    360
   ctgggcaagg tcaacgacgc cttcgagaac ctgaagagat gcacgtcgaa caaccccaat    420
   cagaggcttc ccaaggtgga gatcttgaga aatgccatca gctacatcga gtctctgcaa    480
   tctctgctca ggggccaggg cggcgagaac tactacccta tgttggaaca ctatagcggg    540
   gactctgacg catccagtcc acagtccaac tgctctgatg gaatgatgga ctataatacc    600
   ccaacgtgca cgtccgcaac acgaagcaac tattacagct cttatttcgc cgaaactcca    660
   aatgctggtg ccagaagcaa caagaacgca gcagtcatct ccagcttgga ttgtctttcc    720
   aacatcgtgg agagaatctt tacagacacg tccgcgtcca ctgtgttatc aggccaggag    780
   ggttccgagg gtagcccctg ctctccacaa gagggatcta tcctgagcga aacgggggca    840
   cccgtgccgt caccgaccaa ctgcccacag ccgtcccatg accccatcta ccaagtgcta    900
   tgaacacagt gatagtatgg ctacattcat gaaaacgaat cgattcctat aaatgaagaa    960
   aacaacaaac ggacagagtt gcagaatgtt gctgtagtag aagaatagga tggcaaagct   1020
   gtcattcctt ctaatagaaa ggtttcaatt ccttgaagat cagtcatttg cttcccattg   1080
   atcacgtttt atgagatata atttacgacg tatttatgta ggcctgagtg taatcgtata   1140
   tgaaatattt gactactttc ttaaacttgt gtgtgtgttg tcatcgtcat cttttgttct   1200
   ttccttgcca tttttattgt ttatgacaat agaggaccat ttttgtatat gtgtaaataa   1260
   gaggtgattt gttaaaaaaa agaagaaaaa aataacaaaa agtgttttat gtatttaat    1319
<210> 4
   <211> 276
   <212> PRT
   <213> Salmo salar
<400> 4
   Met Glu Leu Ser Asp Ile Ser Phe Pro Val Thr Ser Ala Asp Asp Phe
   1               5                   10                  15
   Tyr Asp Asp Pro Cys Phe Asn Thr Ser Asp Met His Phe Phe Glu Asp
               20                  25                  30
   Leu Asp Pro Arg Leu Val His Val Gly Leu Leu Lys Pro Asp Asp His
           35                  40                  45
   His Tyr Asn Glu Asp Glu His Ile Arg Ala Pro Ser Gly His His Gln
       50                  55                  60
   Ala Gly Arg Cys Leu Leu Trp Ala Cys Lys Ala Cys Lys Arg Lys Thr
   65                  70                  75                  80
   Thr Asn Ser Asp Arg Arg Lys Ala Ala Thr Met Arg Glu Arg Arg Arg
                   85                  90                  95
   Leu Gly Lys Val Asn Asp Ala Phe Glu Asn Leu Lys Arg Cys Thr Ser
               100                 105                 110
   Asn Asn Pro Asn Gln Arg Leu Pro Lys Val Glu Ile Leu Arg Asn Ala
           115                 120                 125
   Ile Ser Tyr Ile Glu Ser Leu Gln Ser Leu Leu Arg Gly Gln Gly Gly
       130                 135                 140
   Glu Asn Tyr Tyr Pro Met Leu Glu His Tyr Ser Gly Asp Ser Asp Ala
   145                 150                 155                 160
   Ser Ser Pro Gln Ser Asn Cys Ser Asp Gly Met Met Asp Tyr Asn Thr
                   165                 170                 175
   Pro Thr Cys Thr Ser Ala Thr Arg Ser Asn Tyr Tyr Ser Ser Tyr Phe
               180                 185                 190
   Ala Glu Thr Pro Asn Ala Gly Ala Arg Ser Asn Lys Asn Ala Ala Val
           195                 200                 205
   Ile Ser Ser Leu Asp Cys Leu Ser Asn Ile Val Glu Arg Ile Phe Thr
               210                 215                 220
   Asp Thr Ser Ala Ser Thr Val Leu Ser Gly Gln Glu Gly Ser Glu Gly
   225                 230                 235                 240
   Ser Pro Cys Ser Pro Gln Glu Gly Ser Ile Leu Ser Glu Thr Gly Ala
                   245                 250                 255
   Pro Val Pro Ser Pro Thr Asn Cys Pro Gln Pro Ser His Asp Pro Ile
               260                 265                 270
   Tyr Gln Val Leu
           275
<210> 5
   <211> 720
   <212> DNA
   <213> Salmo salar
<400> 5
   atggatgtct tctcccagtc ccagattttc tatgacagcg cctgtgcctc ctcgccagag     60
   gacctggact tcggccccgg ggaactggat ggctcagagg aggacgagca cgtccgggtc    120
   cctgggactc ctcaccaggc gggtcactgc cttcagtggg cctgcaaggc ctgcaagcgt    180
   aagtccagca cggtggaccg gcggcgggct gccaccatga gggaacgacg ccggctgaga    240
   aaggtgaacc acggcttcga ggctctgagg cgctgcacct cagccaacca cagccagagg    300
   ctgcctaagg tggagatcct gcgcaacgcc atccagtaca tcgagagcct ccaggagctg    360
   ctccatgagc atgtggagaa ctactacggc cttcctgggg agagcagctc agagcctggg    420
   agcccctcgt ccagccgctc cgacagcatg gttgactgta acattcctgt tgtgtggcct    480
   cagatgaaca caagctatgg caacaactac agttatacta agaatgtgag ctctggagag    540
   agaggtgctg gtgcctccag cctggcccgc ctgtctaaca tagtagatcg cctctcctcg    600
   gtggatgcca gtgccccagc agggctcaga gatatgctta ccttctcgcc ctccagcacc    660
   gactcccagc cttgcactac agaaagcccc gggaccagac ctgtgtacca cgtattgtga    720
<210> 6
   <211> 239
   <212> PRT
   <213> Salmo salar
<400> 6
   Met Asp Val Phe Ser Gln Ser Gln Ile Phe Tyr Asp Ser Ala Cys Ala
   1               5                   10                  15
   Ser Ser Pro Glu Asp Leu Asp Phe Gly Pro Gly Glu Leu Asp Gly Ser
               20                  25                  30
   Glu Glu Asp Glu His Val Arg Val Pro Gly Thr Pro His Gln Ala Gly
           35                  40                  45
   His Cys Leu Gln Trp Ala Cys Lys Ala Cys Lys Arg Lys Ser Ser Thr
       50                  55                  60
   Val Asp Arg Arg Arg Ala Ala Thr Met Arg Glu Arg Arg Arg Leu Arg
   65                  70                  75                  80
   Lys Val Asn His Gly Phe Glu Ala Leu Arg Arg Cys Thr Ser Ala Asn
                   85                  90                  95
   His Ser Gln Arg Leu Pro Lys Val Glu Ile Leu Arg Asn Ala Ile Gln
               100                 105                 110
   Tyr Ile Glu Ser Leu Gln Glu Leu Leu His Glu His Val Glu Asn Tyr
           115                 120                 125
   Tyr Gly Leu Pro Gly Glu Ser Ser Ser Glu Pro Gly Ser Pro Ser Ser
       130                 135                 140
   Ser Arg Ser Asp Ser Met Val Asp Cys Asn Ile Pro Val Val Trp Pro
   145                 150                 155                 160
   Gln Met Asn Thr Ser Tyr Gly Asn Asn Tyr Ser Tyr Thr Lys Asn Val
                   165                 170                 175
   Ser Ser Gly Glu Arg Gly Ala Gly Ala Ser Ser Leu Ala Arg Leu Ser
               180                 185                 190
   Asn Ile Val Asp Arg Leu Ser Ser Val Asp Ala Ser Ala Pro Ala Gly
           195                 200                 205
   Leu Arg Asp Met Leu Thr Phe Ser Pro Ser Ser Thr Asp Ser Gln Pro
       210                 215                 220
   Cys Thr Thr Glu Ser Pro Gly Thr Arg Pro Val Tyr His Val Leu
   225                 230                 235
<210> 7
   <211> 915
   <212> DNA
   <213> Salmo salar
<400> 7
   atggctaaaa aggatcctgc taagttggtt acggctgttg ctgcaccagc tgccgtggct      60
   ggagatgctt ctctgaaggg gccacagact aagaaagttg aggatttggc tgccattgat     120
   gcctcgtctg ctggcttttc caactttgct ccacctccac ccgctcccat caagaaagct     180
   gaagagttta agtctgcgcc tgtgaccaag tctcctgctc cccccattga caagaaagct     240
   aaggtggaaa agtttgctga tgacttctct ctgatgtcag gattggactc cccactggac     300
   accaagccca agacagctga gggtggctcc atgtccctgg atgctctcag cgctctggga     360
   gactctctgc ctgctccaga accagcacct caacctccca agatcagaca cgaggacata     420
   gtcatggagg gcaaactcac atcgaagaag ggagtgtttg tgggtgagcg agatgacaca     480
   ctcccaccaa agtacaggtt cacagaaaac aaagtcaaag acctgcctcc tctgaagcca     540
   gaggcaatgg cagaggccaa agtccaggct gtagttagac ccaagcagcc gtcgatggat     600
   gacagcgagg cccttgacct cttgtctaga gacttgtctt cctcagctgg tccagctgca     660
   gcctcagtag ccgtgacaac agagcagaga cagcctagac tggagcccat gtctgcccct     720
   gtcctggatg acctggcagg caccctgctc ccagacgccc ccgtgttcaa atctaaggga     780
   gacaagccaa agagcaagtc aaggtcaaag tcaaagaaac aacgggaaga ggatccgtca     840
   tccatagacc acctgtctgg acagctgagt tcagacgtag tgtcagcatc caaaaataag     900
   ggtggcaaga gctag                                                      915
<210> 8
   <211> 304
   <212> PRT
   <213> Salmo salar
<400> 8
   Met Ala Lys Lys Asp Pro Ala Lys Leu Val Thr Ala Val Ala Ala Pro
   1               5                   10                  15
   Ala Ala Val Ala Gly Asp Ala Ser Leu Lys Gly Pro Gln Thr Lys Lys
               20                  25                  30
   Val Glu Asp Leu Ala Ala Ile Asp Ala Ser Ser Ala Gly Phe Ser Asn
           35                  40                  45
   Phe Ala Pro Pro Pro Pro Ala Pro Ile Lys Lys Ala Glu Glu Phe Lys
       50                  55                  60
   Ser Ala Pro Val Thr Lys Ser Pro Ala Pro Pro Ile Asp Lys Lys Ala
   65                  70                  75                      80
   Lys Val Glu Lys Phe Ala Asp Asp Phe Ser Leu Met Ser Gly Leu Asp
                   85                  90                  95
   Ser Pro Leu Asp Thr Lys Pro Lys Thr Ala Glu Gly Gly Ser Met Ser
               100                 105                 110
   Leu Asp Ala Leu Ser Ala Leu Gly Asp Ser Leu Pro Ala Pro Glu Pro
           115                 120                 125
   Ala Pro Gln Pro Pro Lys Ile Arg His Glu Asp Ile Val Met Glu Gly
       130                 135                 140
   Lys Leu Thr Ser Lys Lys Gly Val Phe Val Gly Glu Arg Asp Asp Thr
   145                 150                 155                 160
   Leu Pro Pro Lys Tyr Arg Phe Thr Glu Asn Lys Val Lys Asp Leu Pro
                   165                 170                 175
   Pro Leu Lys Pro Glu Ala Met Ala Glu Ala Lys Val Gln Ala Val Val
               180                 185                 190
   Arg Pro Lys Gln Pro Ser Met Asp Asp Ser Glu Ala Leu Asp Leu Leu
           195                 200                 205
   Ser Arg Asp Leu Ser Ser Ser Ala Gly Pro Ala Ala Ala Ser Val Ala
       210                 215                 220
   Val Thr Thr Glu Gln Arg Gln Pro Arg Leu Glu Pro Met Ser Ala Pro
   225                 230                 235                 240
   Val Leu Asp Asp Leu Ala Gly Thr Leu Leu Pro Asp Ala Pro Val Phe
                   245                 250                 255
   Lys Ser Lys Gly Asp Lys Pro Lys Ser Lys Ser Arg Ser Lys Ser Lys
               260                 265                 270
   Lys Gln Arg Glu Glu Asp Pro Ser Ser Ile Asp His Leu Ser Gly Gln
           275                 280                 285
   Leu Ser Ser Asp Val Val Ser Ala Ser Lys Asn Lys Gly Gly Lys Ser
       290                 295                 300
<210> 9
   <211> 685
   <212> DNA
   <213> Salmo salar
<400> 9
   gatgatatga tatctgcaga cttggaagat gaggacgtca tcagtgaaga tgacgtcgac      60
   gactctttca aatccatgtt cgctcagctt gcaggagagg acatggagat atccgttcgt     120
   gaactcagga ccatcctgaa cagggtggtg actagacaca aagacctgaa gacagacggt     180
   ttcagtatgg agtcctgtag gaccatggtc aacctcatgg ataaagatgg cagtgcccat     240
   ctaggcctga tagagttcca gctcctctgg aataagatca ggaagtggct ggtcatctat    300
   agagaatatg acatggacaa gtcagggtcc atgagctcct atgagatgcg acttgctgtg    360
   gagtctgcag gttttaaact gaacaacaga ctgaatcagg tcctagtggc tcgctatgct    420
   gagaacgagg ccatagactt tgacaacttc atctgctgtc tggtcaaact ggaagccatg    480
   tttagatctt tccagcagct agacaaagag ggagaaggtg tggctgagat gaacatcact    540
   gagtggcttt atatgaccat gtgtggttga agaggaccag tcttgtccca ggagacagaa    600
   cagccaagtg cccggcagag tgcctcttcc ctgggaccat gaagaacttc ctagccttat    660
   tgtcctctgc ctgatcctga actat                                          685
<210> 10
   <211> 3360
   <212> DNA
   <213> Salmo salar
<400> 10
   actaacttac atactaaagg gggcagctca cactgatagg gggaacgagg acacgcataa     60
   ggggaaaaac atagccaaga tgtcccttgt tgaggaaact tcagtgaagg tggcgttgga    120
   aactgaagca tcacctgacg tgcctgaggg taagactgac taccccttca cggccagcaa    180
   cagtatctac actgccattt tgagcagaaa cgaggcagtc aaagatgcca agcgcctgaa    240
   gaacttcttg gagttgcgag acaaatacgt acacaagaaa gtcctgtttg aagaccctct    300
   gttcccaccc aatgattcct ctctttggta cagcagcaaa tttccaattg aatttgagtg    360
   gaagcgccca acggaaatct gtgacaaccc ccagttcatc atagacggag ctagtaggac    420
   agacatctgt caaggagagc tgggtgactg ctggctgctt gctgctattg cctgtctgac    480
   actgaatgac aagctgctgt acagagttat cccaccagac cagagtttca cagagaacta    540
   cgctggtatc ttccatttcc agttctggcg ttatggtgaa tgggtggacg tcgtggtgga    600
   cgaccgtatc cccacctcca ataaccagct ggtgttcacc aagtccttca ggaagaacga    660
   gttctggagc gctctgctgg agaaggctta cgccaagttg cacggttcct acgaggcctt    720
   gaaaggtggc aacaccctgg aggccatgga ggacttcact gggggtgtga cagagttctt    780
   tgagatgtcc gaggctccaa aagaactcta caagatcatg aagaaagccc tggagagagg    840
   ctctctgatg ggctgttcca tagacgcctt gctgccgagt cactgtgaga ctgaaactgc    900
   gaatggactg gtcagaggcc acgcctactc tatcactgca ctagaggagt gtgacaagct    960
   taaggttacc aaggacacca agattcgcct agttcaaatg cggaacccct gggggatggt   1020
   gctatggaaa gggccgtgga atgcaaagtc caaagaatgg tccaccatct ccagctcaga   1080
   caaggccagg ctgaagaaga agacagttga gacgagcgag acttggatat cctttgagga   1140
   tttcaaaaag aacttcacca agctggagat ctgtaacctg acccccgaca cacttctgga   1200
   cgacaagtcc catagctgga cggtggcggt caacgagggc cgctgggtac gaggcagctc 1260
   cgccgggggc tgcaggaact tcccagacac gttctggacc aacccgcagt accgcatgcg 1320
   tctgtacgag gaggacgacg accccgacga ccccgagcat gtggcgtgca ccgtcatggt 1380
   ggccctgatg cagaagggcc ggaggaaaga ccgcagcaag ggtgccaagc tttacaccat 1440
   cggattctcc atctacgagg tgcccaaaga gatgattggg agccagcagc acctgcagaa 1500
   agacttcttc ctttacaatg cctccaaggc caaatgcaag tcctacatca acctgcgtga 1560
   ggtgagcgag cgattctgcc tgccccccgg ggagtacgtg atcatccctt cgacctttga 1620
   cccccacgag gagggagagt ttatcctcag agtcttctcc gagaagaaca gcacctcaga 1680
   ggaaaccgaa aatacaatta cgtcagacca aatagagcaa ggcaaaaaca ggacaaagaa 1740
   aggaaagccc atcgtgtttg tatcagacag agcaaaggcc aacaaggaga ttgagcaaga 1800
   tgacatcgag gttcagaagg agaagaaaga gaaagtcaag ccaaaaccac agcaaccgga 1860
   tgaggagagc ggagaacaca aaatgttcag ggccatttac cagcagattg ctggggagga 1920
   catgcagatc tgtgccaacg agctcaaggt ggtcatgaaa agggtactgg aaaaacataa 1980
   tgaaatgaag acggacggct tcagcttaga gagttgccgg agcatgatcg ctttgatgga 2040
   cacggacggc acaggaaaac tgaacctgca ggagttcaaa cacctgtgga gtaagatcaa 2100
   agcatggcag ctgatcttca agaagtatga caaggacaaa tccagcacca tcagcagctt 2160
   tgagatgagg aacgcagtca atgacgcagg gttccaccta aacaaccagc tgtatgacat 2220
   catcacgatg cgctacgctg acgagcacct caacatcgac tttgacagct tcatctgctg 2280
   cttcgtcagg ctagaaggca tgttcaggac gttccatgcc tttgacaaaa acggagacgg 2340
   cacaatcaag ctcaacgtcc tcgagtggct ccagttgacc atgtatgctt agatcaccca 2400
   gccatcagag gagtcctgct ctctggtcta ccctcagcca tgcttctacc aacatcacat 2460
   agatcaccca gccatcagag aagtcctgct ctctggtcta ccctcagcca tgcttctacc 2520
   aacatcacat agatcaccca gccatcagag aagtcctgct ctctggtcta ccctcagcca 2580
   ctcttctacc aacatcgctt agatcaccca gccatcagag aagtcctgcc ctctggtcta 2640
   ccctcagcca tgcttctacc agcatcacct tcctctacct cagccaattc tccatgttct 2700
   ctctgacaag acattgccat tactctcatt ctagcataga aaacaaatta aatgcaatgg 2760
   tgatatttcc tgcagtcacg ctgcacaaat gatatatctc acaacgcgga gagaagtgtg 2820
   taatgtctca agaaagtaca tgaacacgat gtagcgatct tctaagacac acaggttggc 2880
   tggagagaaa gaccacctgg aatgtcaact ttgactggtg agaggtagca ccctggcagc 2940
   accagcagac agtacagcac cgagggactt gaatggggaa cccttgctcc agtacatttg 3000
   gctagtgctt taaactacaa gccttcaagt gttattgcct gtccttaaag gggaaggttt 3060
   tagaacgcca gctcactgta aataacatgt ttagtattag tggattgaat acatatcgtt   3120
   gcttagcttt acttcctgaa gtgtttccat tcccctttgt cattggtggt agctacatta   3180
   caaaatcaat gttgtgttcc tgtaagtctc tgtgataata tcatgtggat actcaggtct   3240
   cgagagacag tgatgatgct gttggactgg attgttggat tgtttcagaa gcatttttgt   3300
   cttttaaatt cattaaattc aaacttttaa aaaaaaaaaa aaaaaaaaaa aaaaaaaaga   3360
<210> 11
   <211> 770
   <212> PRT
   <213> Salmo salar
<400> 11
   Met Ser Leu Val Glu Glu Thr Ser Val Lys Val Ala Leu Glu Thr Glu
   1               5                   10                  15
   Ala Ser Pro Asp Val Pro Glu Gly Lys Thr Asp Tyr Pro Phe Thr Ala
               20                  25                  30
   Ser Asn Ser Ile Tyr Thr Ala Ile Leu Ser Arg Asn Glu Ala Val Lys
           35                  40                  45
   Asp Ala Lys Arg Leu Lys Asn Phe Leu Glu Leu Arg Asp Lys Tyr Val
       50                  55                  60
   His Lys Lys Val Leu Phe Glu Asp Pro Leu Phe Pro Pro Asn Asp Ser
   65                  70                  75                  80
   Ser Leu Trp Tyr Ser Ser Lys Phe Pro Ile Glu Phe Glu Trp Lys Arg
                   85                  90                  95
   Pro Thr Glu Ile Cys Asp Asn Pro Gln Phe Ile Ile Asp Gly Ala Ser
               100                 105                 110
   Arg Thr Asp Ile Cys Gln Gly Glu Leu Gly Asp Cys Trp Leu Leu Ala
           115                 120                 125
   Ala Ile Ala Cys Leu Thr Leu Asn Asp Lys Leu Leu Tyr Arg Val Ile
       130                 135                 140
   Pro Pro Asp Gln Ser Phe Thr Glu Asn Tyr Ala Gly Ile Phe His Phe
   145                 150                 155                 160
   Gln Phe Trp Arg Tyr Gly Glu Trp Val Asp Val Val Val Asp Asp Arg
                   165                 170                 175
   Ile Pro Thr Ser Asn Asn Gln Leu Val Phe Thr Lys Ser Phe Arg Lys
               180                 185                 190
   Asn Glu Phe Trp Ser Ala Leu Leu Glu Lys Ala Tyr Ala Lys Leu His
           195                 200                 205
   Gly Ser Tyr Glu Ala Leu Lys Gly Gly Asn Thr Leu Glu Ala Met Glu
       210                 215                 220
   Asp Phe Thr Gly Gly Val Thr Glu Phe Phe Glu Met Ser Glu Ala Pro
   225                 230                 235                 240
   Lys Glu Leu Tyr Lys Ile Met Lys Lys Ala Leu Glu Arg Gly Ser Leu
                   245                 250                 255
   Met Gly Cys Ser Ile Asp Ala Leu Leu Pro Ser His Cys Glu Thr Glu
               260                 265                 270
   Thr Ala Asn Gly Leu Val Arg Gly His Ala Tyr Ser Ile Thr Ala Leu
           275                 280                 285
   Glu Glu Cys Asp Lys Leu Lys Val Thr Lys Asp Thr Lys Ile Arg Leu
       290                 295                 300
   Val Gln Met Arg Asn Pro Trp Gly Met Val Leu Trp Lys Gly Pro Trp
   305                 310                 315                 320
   Asn Ala Lys Ser Lys Glu Trp Ser Thr Ile Ser Ser Ser Asp Lys Ala
                   325                 330                 335
   Arg Leu Lys Lys Lys Thr Val Glu Thr Ser Glu Thr Trp Ile Ser Phe
               340                 345                 350
   Glu Asp Phe Lys Lys Asn Phe Thr Lys Leu Glu Ile Cys Asn Leu Thr
           355                 360                 365
   Pro Asp Thr Leu Leu Asp Asp Lys Ser His Ser Trp Thr Val Ala Val
       370                 375                 380
   Asn Glu Gly Arg Trp Val Arg Gly Ser Ser Ala Gly Gly Cys Arg Asn
   385                 390                 395                 400
   Phe Pro Asp Thr Phe Trp Thr Asn Pro Gln Tyr Arg Met Arg Leu Tyr
                   405                 410                 415
   Glu Glu Asp Asp Asp Pro Asp Asp Pro Glu His Val Ala Cys Thr Val
               420                 425                 430
   Met Val Ala Leu Met Gln Lys Gly Arg Arg Lys Asp Arg Ser Lys Gly
           435                 440                 445
   Ala Lys Leu Tyr Thr Ile Gly Phe Ser Ile Tyr Glu Val Pro Lys Glu
       450                 455                 460
   Met Ile Gly Ser Gln Gln His Leu Gln Lys Asp Phe Phe Leu Tyr Asn
   465                 470                 475                 480
   Ala Ser Lys Ala Lys Cys Lys Ser Tyr Ile Asn Leu Arg Glu Val Ser
                   485                 490                 495
   Glu Arg Phe Cys Leu Pro Pro Gly Glu Tyr Val Ile Ile Pro Ser Thr
               500                 505                 510
   Phe Asp Pro His Glu Glu Gly Glu Phe Ile Leu Arg Val Phe Ser Glu
           515                 520                 525
   Lys Asn Ser Thr Ser Glu Glu Thr Glu Asn Thr Ile Thr Ser Asp Gln
       530                 535                 540
   Ile Glu Gln Gly Lys Asn Arg Thr Lys Lys Gly Lys Pro Ile Val Phe
   545                 550                 555                 560
   Val Ser Asp Arg Ala Lys Ala Asn Lys Glu Ile Glu Gln Asp Asp Ile
                   565                 570                 575
   Glu Val Gln Lys Glu Lys Lys Glu Lys Val Lys Pro Lys Pro Gln Gln
               580                 585                 590
   Pro Asp Glu Glu Ser Gly Glu His Lys Met Phe Arg Ala Ile Tyr Gln
           595                 600                 605
   Gln Ile Ala Gly Glu Asp Met Gln Ile Cys Ala Asn Glu Leu Lys Val
       610                 615                 620
   Val Met Lys Arg Val Leu Glu Lys His Asn Glu Met Lys Thr Asp Gly
   625                 630                 635                 640
   Phe Ser Leu Glu Ser Cys Arg Ser Met Ile Ala Leu Met Asp Thr Asp
                   645                 650                 655
   Gly Thr Gly Lys Leu Asn Leu Gln Glu Phe Lys His Leu Trp Ser Lys
               660                 665                 670
   Ile Lys Ala Trp Gln Leu Ile Phe Lys Lys Tyr Asp Lys Asp Lys Ser
           675                 680                 685
   Ser Thr Ile Ser Ser Phe Glu Met Arg Asn Ala Val Asn Asp Ala Gly
   690 695 700
   Phe His Leu Asn Asn Gln Leu Tyr Asp Ile Ile Thr Met Arg Tyr Ala
   705 710 715 720
   Asp Glu His Leu Asn Ile Asp Phe Asp Ser Phe Ile Cys Cys Phe Val
   725 730 735
   Arg Leu Glu Gly Met Phe Arg Thr Phe His Ala Phe Asp Lys Asn Gly
   740 745 750
   Asp Gly Thr Ile Lys Leu Asn Val Leu Glu Trp Leu Gln Leu Thr Met
   755 760 765
   Tyr Ala
   770
<210> 12
   <211> 758
   <212> DNA
   <213> Salmo salar
<400> 12
   tggctatttg aagaatctca aatatatttt gatttgtttt acttttttgg ttgctgcatg 60
   attccgtatg tgtgattcca tgttccacaa tgtggaaaat agtaagggtg acagggaact 120
   cttgaatgag taggtgttgt aaaagacctt tggaaagcta taagcagtat aacacctgac 180
   atcagcatta gtttagaaac ataaacatat tttccatgac atgtcaatgc ttctaaagac 240
   cttatgtata tccctataac atgccgtttg ctctctcact tcctgcaggc ctatgcttta 300
   ttatttcact atttgctttg gtagaacgtt cctttagccc ttcttttatt atttcatctc 360
   attcaaagaa ttgatccaga acttaacaat tcatataaag ggaaaatatg tccttccaca 420
   actctttata ttaccacaaa agtgtttctt tgaacaatgt atatatgtaa atataaacat 480
   gtctgtgtat atttgaattt agtaacttaa gtaatccttt tttattatag ttattctaaa 540
   gaataaggtt gtttatgttt tgttcgtttt ttatgatgtc attccgtttt attagtgtct 600
   gttgacacag gccaggtttt atacagattt atggacaata tccagggtcc cccttaatca 660
   caaaagcact gacagaaaca cattattcta agtgcttgtt tgttttgctt tctgatttga 720
   ttatgttcca acacttaaca ttttcagtaa gcacagat 758
<210> 13
   <211> 1010
   <212> DNA
   <213> Salmo salar
<400> 13
   accttgtgca tttcaggtaa aataacaact caatgtttat atccccggac aaattagcta     60
   gcaacagcaa gctatttaaa taggacaaat tagctagcaa gtgcaagcta gctacctaaa    120
   ttgccataca tgtttaatgc ttttcgacct gtccccaaat taatgtaatt tacagtctgt    180
   tgtgatcgcg tttggtgtgg ggggacaaaa tacatttatg cacgatggcg cacacgcgca    240
   gccggtttgg gttctgtgtt agcctactgc cgtgtgcaca ttgctgctct tataatgtga    300
   agaaataata gtttatcaat attttaagat aagccttctg atctgttgca tcagcctcat    360
   tgctttaaaa aaaatgtaat gatgtacagt gatttaggat ctatcatccc agaactgctc    420
   cagagtctgt tttaaaccgt cccagacata tgctttatcg ttccgggacg cccttaattt    480
   atagccctgg ctggtagcct ccgcctgcag gatgagtgca gtcagtgtgc cctcgagtgg    540
   gggaagagga gtgggattca aaactagttt gatgaggatt tcatttaaac aagagagaaa    600
   atagatcaaa agtgaaatag aaatgtcata ataaaaaaac accaccaatc tgaaagggca    660
   ctcattctct ctatctgtgc acgtacctgc tgatcaggtt gacaattatc ttctcaatgt    720
   atttgtgtgg ctaaaatgaa atttttagcg cacaacattt tctggtttgc agaaatgtat    780
   tgaaagagag aaatacaagt cctaccgagg tcgaaaagat ctaacttcta cttttggatg    840
   ctttctaact tctgatgcat gaatttgatc gatttattca gtgtattgtc gctgttatca    900
   aaataactaa tagtaagtgc atgcagccac taatcccact tgcttcagag cacgcattcc    960
   ctacatgaca ggaacgagca cgtagatctg atgcactaaa tggcaggtta              1010
<210> 14
   <211> 22
   <212> DNA
   <213> Artificial
<220>
   <223> Primer
<400> 14
   aagttggtta cggctgttgc tg 22
<210> 15
   <211> 25
   <212> DNA
   <213> Artificial
<220>
   <223> Primer
<400> 15
   ctagctcttg ccacccttat ttttg 25
<210> 16
   <211> 20
   <212> DNA
   <213> Artificial
<220>
   <223> Primer
<400> 16
   actacggcca ggtgagaatg 20
<210> 17
   <211> 20
   <212> DNA
   <213> Artificial
<220>
   <223> Primer
<400> 17
   caggaagtcg ctgaatgaca 20
<210> 18
   <211> 21
   <212> DNA
   <213> Artificial
<220>
   <223> Primer
<400> 18
   ctgcagactt ggaagatgag g 21
<210> 19
   <211> 20
   <212> DNA
   <213> Artificial
<220>
   <223> Primer
<400> 19
   caggcagagg acaataaggc 20
<210> 20
   <211> 21
   <212> DNA
   <213> Artificial
<220>
   <223> Primer
<400> 20
   atggatgtct tctcccagtc c 21
<210> 21
   <211> 23
   <212> DNA
   <213> Artificial
<220>
   <223> Primer
<400> 21
   tcacaatacg tggtacacag gtc 23
<210> 22
   <211> 20
   <212> DNA
   <213> Artificial
<220>
   <223> Primer
<400> 22
   cctgtcacct ctgctgatga 20
<210> 23
   <211> 20
   <212> DNA
   <213> Artificial
<220>
   <223> Primer
<400> 23
   cttggtagat ggggtcatgg 20
<210> 24
   <211> 20
   <212> DNA
   <213> Artificial
<220>
   <223> Primer
<400> 24
   acaagtccca tagctggacg 20
<210> 25
   <211> 20
   <212> DNA
   <213> Artificial
<220>
   <223> Primer
<400> 25
   gagcaggact cctctgatgg 20
<210> 26
   <211> 22
   <212> DNA
   <213> Artificial
<220>
   <223> Primer
<400> 26
   gcctccaatt agagacaacg ac 22
<210> 27
   <211> 20
   <212> DNA
   <213> Artificial
<220>
   <223> Primer
<400> 27
   ctttaccttg atgggagcgg 20
<210> 28
   <211> 22
   <212> DNA
   <213> Artificial
<220>
   <223> Primer
<400> 28
   gaggatttgg ctgccattga tg 22
<210> 29
   <211> 20
   <212> DNA
   <213> Artificial
<220>
   <223> Primer
<400> 29
   ggtggaggtg gagcaaagtt 20
<210> 30
   <211> 22
   <212> DNA
   <213> Artificial
<220>
   <223> Primer
<400> 30
   agaaccacaa cgcagtcaag tt 22
<210> 31
   <211> 15
   <212> DNA
   <213> Artificial
<220>
   <223> Primer
<400> 31
   gcggctctgg aggca 15
<210> 32
   <211> 22
   <212> DNA
   <213> Artificial
<220>
   <223> Primer
<400> 32
   cttgtcccag gagacagaac ag 22
<210> 33
   <211> 27
   <212> DNA
   <213> Artificial
<220>
   <223> Primer
<400> 33
   caataaggct aggaagttct tcatggt 27
<210> 34
   <211> 16
   <212> DNA
   <213> Artificial
<220>
   <223> Primer
<400> 34
   ggtgctggtg cctcca 16
<210> 35
   <211> 24
   <212> DNA
   <213> Artificial
<220>
   <223> Primer
<400> 35
   gcgagaaggt aagcatatct ctga 24
<210> 36
   <211> 20
   <212> DNA
   <213> Artificial
<220>
   <223> Primer
<400> 36
   cgcgtccact gtgttatcag 20
<210> 37
   <211> 23
   <212> DNA
   <213> Artificial
<220>
   <223> Primer
<400> 37
   tcgctcagga tagatccctc ttg 23
<210> 38
   <211> 24
   <212> DNA
   <213> Artificial
<220>
   <223> Primer
<400> 38
   tgtctgttct ctttcaggga aacc 24
<210> 39
   <211> 25
   <212> DNA
   <213> Artificial
<220>
   <223> Primer
<400> 39
   ggctttcctt tctttgtcct gtttt 25
<210> 40
   <211> 23
   <212> DNA
   <213> Artificial
<220>
   <223> Primer
<400> 40
   tgagagagca aacggcatgt tat 23
<210> 41
   <211> 29
   <212> DNA
   <213> Artificial
<220>
   <223> Primer
<400> 41
   catattttcc atgacatgtc aatgcttct 29
<210> 42
   <211> 25
   <212> DNA
   <213> Artificial
<220>
   <223> Primer
<400> 42
   ccttaattta tagccctggc tggta 25
<210> 43
   <211> 28
   <212> DNA
   <213> Artificial
<220>
   <223> Primer
<400> 43
   aaactagttt tgaatcccac tcctcttc 28
<210> 44
   <211> 13
   <212> DNA
   <213> Artificial
<220>
   <223> Primer
<400> 44
   tgctggcttt tcc 13
<210> 45
   <211> 14
   <212> DNA
   <213> Artificial
<220>
   <223> Primer
<400> 45
   ctgctgtctt ttcc 14
<210> 46
   <211> 15
   <212> DNA
   <213> Artificial
<220>
   <223> Primer
<400> 46
   ccctcagcga ctcaa 15
<210> 47
   <211> 15
   <212> DNA
   <213> Artificial
<220>
   <223> Primer
<400> 47
   ccctcagcaa ctcaa 15
<210> 48
   <211> 13
   <212> DNA
   <213> Artificial
<220>
   <223> Primer
<400> 48
   tgccgggcac ttg 13
<210> 49
   <211> 14
   <212> DNA
   <213> Artificial
<220>
   <223> Primer
<400> 49
   ctgccggtca cttg 14
<210> 50
   <211> 22
   <212> DNA
   <213> Artificial
<220>
   <223> Primer
<400> 50
   agaggcgatc tactatacta ga 22
<210> 51
   <211> 20
   <212> DNA
   <213> Artificial
<220>
   <223> Primer
<400> 51
   aggcgatcta ctatgttaga 20
<210> 52
   <211> 16
   <212> DNA
   <213> Artificial
<220>
   <223> Primer
<400> 52
   tagcccctgc tctcca 16
<210> 53
   <211> 16
   <212> DNA
   <213> Artificial
<220>
   <223> Primer
<400> 53
   tagcccctac tctcca 16
<210> 54
   <211> 17
   <212> DNA
   <213> Artificial
<220>
   <223> Primer
<400> 54
   ttggtctgac gtaattg 17
<210> 55
   <211> 17
   <212> DNA
   <213> Artificial
<220>
   <223> Primer
<400> 55
   ttggtctgac ctaattg 17
<210> 56
   <211> 21
   <212> DNA
   <213> Artificial
<220>
   <223> Primer
<400> 56
   aaagacctta tgtatatccc t 21
<210> 57
   <211> 21
   <212> DNA
   <213> Artificial
<220>
   <223> Primer
<400> 57
   aaagacctta tgtatacccc t 21
<210> 58
   <211> 16
   <212> DNA
   <213> Artificial
<220>
   <223> Primer
<400> 58
   acactgacgg cactca 16
<210> 59
   <211> 17
   <212> DNA
   <213> Artificial
<220>
   <223> Primer
<400> 59
   cacactgact gcactca 17
<210> 60
   <211> 19
   <212> DNA
   <213> Artificial
<220>
   <223> Primer
<400> 60
   ttgcggatgc tgccttgtc 19
<210> 61
   <211> 22
   <212> DNA
   <213> Artificial
<220>
   <223> Primer
<400> 61
   ggaaggtaga tcgtgaagca gg 22
<210> 62
   <211> 25
   <212> DNA
   <213> Artificial
<220>
   <223> Primer
<400> 62
   atgtccctgg atgctctcag tgctc 25
<210> 63
   <211> 25
   <212> DNA
   <213> Artificial
<220>
   <223> Primer
<400> 63
   ctagctcttg ccacccttat ttttg 25
<210> 64
   <211> 19
   <212> DNA
   <213> Artificial
<220>
   <223> Primer
<400> 64
   gagcggccaa agtgatgat 19
<210> 65
   <211> 20
   <212> DNA
   <213> Artificial
<220>
   <223> Primer
<400> 65
   cagccagagt gtctcccaga 20
<210> 66
   <211> 22
   <212> DNA
   <213> Artificial
<220>
   <223> Primer
<400> 66
   aagttggtta cggctgttgc tg 22
<210> 67
   <211> 25
   <212> DNA
   <213> Artificial
<220>
   <223> Primer
<400> 67
   ctagctcttg ccacccttat ttttg 25
<210> 68
   <211> 22
   <212> DNA
   <213> Artificial
<220>
   <223> Primer
<400> 68
   cacgtctgac agcagataac cg 22
<210> 69
   <211> 21
   <212> DNA
   <213> Artificial
<220>
   <223> Primer
<400> 69
   atgcccagtt ccaagtcaac c 21
<210> 70
   <211> 21
   <212> DNA
   <213> Artificial
<220>
   <223> Primer
<400> 70
   gaccactgtg gcatcgagtc t 21
<210> 71
   <211> 20
   <212> DNA
   <213> Artificial
<220>
   <223> Primer
<400> 71
   cctttcaccc atcctgcatt 20
<210> 72
   <211> 22
   <212> DNA
   <213> Artificial
<220>
   <223> Primer
<400> 72
   tcggtacacc tgacaagcag ac 22
<210> 73
   <211> 21
   <212> DNA
   <213> Artificial
<220>
   <223> Primer
<400> 73
   cgcctctctc atcacatcct g 21
<210> 74
   <211> 20
   <212> DNA
   <213> Artificial
<220>
   <223> Primer
<400> 74
   gctggcatcc ctttgtaacc 20
<210> 75
   <211> 23
   <212> DNA
   <213> Artificial
<220>
   <223> Primer
<400> 75
   tccacatttc aatgatgatc caa 23
<210> 76
   <211> 21
   <212> DNA
   <213> Artificial
<220>
   <223> Primer
<400> 76
   gcagtgttgg tgctctgtgt g 21
<210> 77
   <211> 21
   <212> DNA
   <213> Artificial
<220>
   <223> Primer
<400> 77
   gcagcactgt cgtcatcatt g 21
<210> 78
   <211> 19
   <212> DNA
   <213> Artificial
<220>
   <223> Primer
<400> 78
   tgtgtctttt cgcggcact 19
<210> 79
   <211> 21
   <212> DNA
   <213> Artificial
<220>
   <223> Primer
<400> 79
   agtggagaat ggggacgtag c 21
<210> 80
   <211> 21
   <212> DNA
   <213> Artificial
<220>
   <223> Primer
<400> 80
   agtggagaat ggggacgtag c 21
<210> 81
   <211> 20
   <212> DNA
   <213> Artificial
<220>
   <223> Primer
<400> 81
   gggcttcgct aagtccaagt 20
<210> 82
   <211> 21
   <212> DNA
   <213> Artificial
<220>
   <223> Primer
<400> 82
   ggtccattgc ctcataaaac g 21
<210> 83
   <211> 21
   <212> DNA
   <213> Artificial
<220>
   <223> Primer
<400> 83
   cggtttggac gtcctagatt c 21
<210> 84
   <211> 20
   <212> DNA
   <213> Artificial
<220>
   <223> Primer
<400> 84
   agtggctagg ggctgttttt 20
<210> 85
   <211> 20
   <212> DNA
   <213> Artificial
<220>
   <223> Primer
<400> 85
   gtgtttgaca gggacgcaga 20
<210> 86
   <211> 22
   <212> DNA
   <213> Artificial
<220>
   <223> Primer
<400> 86
   aacagaagca ttgtggtctt gg 22
<210> 87
   <211> 20
   <212> DNA
   <213> Artificial
<220>
   <223> Primer
<400> 87
   caatatcact ggcacctcca 20
<210> 88
   <211> 20
   <212> DNA
   <213> Artificial
<220>
   <223> Primer
<400> 88
   cagcgccatg tagaaagaca 20
<210> 89
   <211> 20
   <212> DNA
   <213> Artificial
<220>
   <223> Primer
<400> 89
   aggtcaacgt tcaggagtgg 20
<210> 90
   <211> 20
   <212> DNA
   <213> Artificial
<220>
   <223> Primer
<400> 90
   atggcatctg ttggcttcat 20
<210> 91
   <211> 20
   <212> DNA
   <213> Artificial
<220>
   <223> Primer
<400> 91
   actacggcca ggtgagaatg 20
<210> 92
   <211> 20
   <212> DNA
   <213> Artificial
<220>
   <223> Primer
<400> 92
   caggaagtcg ctgaatgaca 20
<210> 93
   <211> 20
   <212> DNA
   <213> Artificial
<220>
   <223> Primer
<400> 93
   aacttccctg ccacattctg 20
<210> 94
   <211> 22
   <212> DNA
   <213> Artificial
<220>
   <223> Primer
<400> 94
   tgcaataaca tcagrgagtc ca 22
<210> 95
   <211> 21
   <212> DNA
   <213> Artificial
<220>
   <223> Primer
<400> 95
   cttccagtgg atcaccctga c 21
<210> 96
   <211> 23
   <212> DNA
   <213> Artificial
<220>
   <223> Primer
<400> 96
   tgtaaacaac agtcgtgatt gca 23
<210> 97
   <211> 20
   <212> DNA
   <213> Artificial
<220>
   <223> Primer
<400> 97
   cagccgtctg ctgtaggtaa 20
<210> 98
   <211> 20
   <212> DNA
   <213> Artificial
<220>
   <223> Primer
<400> 98
   gaactctccg tcctcactgc 20
<210> 99
   <211> 20
   <212> DNA
   <213> Artificial
<220>
   <223> Primer
<400> 99
   actccagaga gtgggagggt 20
<210> 100
   <211> 20
   <212> DNA
   <213> Artificial
<220>
   <223> Primer
<400> 100
   cgcagtatga tcagctggaa 20
<210> 101
   <211> 21
   <212> DNA
   <213> Artificial
<220>
   <223> Primer
<400> 101
   ggatcagcct gaccatgttt g 21
<210> 102
   <211> 21
   <212> DNA
   <213> Artificial
<220>
   <223> Primer
<400> 102
   ttggcgttca tctacacagg a 21
<210> 103
   <211> 20
   <212> DNA
   <213> Artificial
<220>
   <223> Primer
<400> 103
   acaaggacaa ggacaatggc 20
<210> 104
   <211> 20
   <212> DNA
   <213> Artificial
<220>
   <223> Primer
<400> 104
   attgttcctg catcctccag 20
<210> 105
   <211> 20
   <212> DNA
   <213> Artificial
<220>
   <223> Primer
<400> 105
   tctggagctc tgcactctga 20
<210> 106
   <211> 20
   <212> DNA
   <213> Artificial
<220>
   <223> Primer
<400> 106
   tttgggagga ctggtctttg 20
<210> 107
   <211> 19
   <212> DNA
   <213> Artificial
<220>
   <223> Primer
<400> 107
   gacacgcata aggggaaaa 19
<210> 108
   <211> 20
   <212> DNA
   <213> Artificial
<220>
   <223> Primer
<400> 108
   ccagctatgg gacttgtcgt 20
<210> 109
   <211> 20
   <212> DNA
   <213> Artificial
<220>
   <223> Primer
<400> 109
   acaagtccca tagctggacg 20
<210> 110
   <211> 20
   <212> DNA
   <213> Artificial
<220>
   <223> Primer
<400> 110
   gagcaggact cctctgatgg 20
<210> 111
   <211> 20
   <212> DNA
   <213> Artificial
<220>
   <223> Primer
<400> 111
   tggctccagt tgaccatgta 20
<210> 112
   <211> 20
   <212> DNA
   <213> Artificial
<220>
   <223> Primer
<400> 112
   tccaacaatc cagtccaaca 20
<210> 113
   <211> 23
   <212> DNA
   <213> Artificial
<220>
   <223> Primer
<400> 113
   ttggctttac tcagatgaca tga 23
<210> 114
   <211> 20
   <212> DNA
   <213> Artificial
<220>
   <223> Primer
<400> 114
   gtaaaactct gccacgccac 20
<210> 115
   <211> 21
   <212> DNA
   <213> Artificial
<220>
   <223> Primer
<400> 115
   ctgcagactt ggaagatgag g 21
<210> 116
   <211> 20
   <212> DNA
   <213> Artificial
<220>
   <223> Primer
<400> 116
   caggcagagg acaataaggc 20
<210> 117
   <211> 20
   <212> DNA
   <213> Artificial
<220>
   <223> Primer
<400> 117
   cccgatacct cgttcacttg 20
<210> 118
   <211> 20
   <212> DNA
   <213> Artificial
<220>
   <223> Primer
<400> 118
   caggagtgtt cccagagagc 20
<210> 119
   <211> 21
   <212> DNA
   <213> Artificial
<220>
   <223> Primer
<400> 119
   actctgtagt ccgccttcac g 21
<210> 120
   <211> 21
   <212> DNA
   <213> Artificial
<220>
   <223> Primer
<400> 120
   tgctctttgc ggttgaagta g 21
<210> 121
   <211> 20
   <212> DNA
   <213> Artificial
<220>
   <223> Primer
<400> 121
   gatgctcgtg agcgagagct 20
<210> 122
   <211> 23
   <212> DNA
   <213> Artificial
<220>
   <223> Primer
<400> 122
   tttgccatag tgcaaactgt gtt 23
<210> 123
   <211> 21
   <212> DNA
   <213> Artificial
<220>
   <223> Primer
<400> 123
   actctgtagt ccgccttcac g 21
<210> 124
   <211> 21
   <212> DNA
   <213> Artificial
<220>
   <223> Primer
<400> 124
   tgctctttgc ggttgaagta g 21
<210> 125
   <211> 20
   <212> DNA
   <213> Artificial
<220>
   <223> Primer
<400> 125
   accctggact ttgggacaga 20
<210> 126
   <211> 22
   <212> DNA
   <213> Artificial
<220>
   <223> Primer
<400> 126
   gaaaggcaac tgctaatgga aa 22
<210> 127
   <211> 21
   <212> DNA
   <213> Artificial
<220>
   <223> Primer
<400> 127
   ctagcgtcga ccagtatgga g 21
<210> 128
   <211> 21
   <212> DNA
   <213> Artificial
<220>
   <223> Primer
<400> 128
   ctctgggttt atttgggaat g 21
<210> 129
   <211> 20
   <212> DNA
   <213> Artificial
<220>
   <223> Primer
<400> 129
   gccggatatt cctttcccta 20
<210> 130
   <211> 20
   <212> DNA
   <213> Artificial
<220>
   <223> Primer
<400> 130
   gctccgttct cactcgctat 20
<210> 131
   <211> 20
   <212> DNA
   <213> Artificial
<220>
   <223> Primer
<400> 131
   cctgtcacct ctgctgatga 20
<210> 132
   <211> 20
   <212> DNA
   <213> Artificial
<220>
   <223> Primer
<400> 132
   cttggtagat ggggtcatgg 20
<210> 133
   <211> 21
   <212> DNA
   <213> Artificial
<220>
   <223> Primer
<400> 133
   atggagttgt cggatatttc g 21
<210> 134
   <211> 24
   <212> DNA
   <213> Artificial
<220>
   <223> Primer
<400> 134
   tcatagcact tggtagatgg ggtc 24
<210> 135
   <211> 21
   <212> DNA
   <213> Artificial
<220>
   <223> Primer
<400> 135
   atgatggacc tttttgagac c 21
<210> 136
   <211> 20
   <212> DNA
   <213> Artificial
<220>
   <223> Primer
<400> 136
   ggaaatacga aatgctgacg 20
<210> 137
   <211> 21
   <212> DNA
   <213> Artificial
<220>
   <223> Primer
<400> 137
   atgatggacc tttttgagac c 21
<210> 138
   <211> 20
   <212> DNA
   <213> Artificial
<220>
   <223> Primer
<400> 138
   ggaaatacga aatgctgacg 20
<210> 139
   <211> 21
   <212> DNA
   <213> Artificial
<220>
   <223> Primer
<400> 139
   atggatgtct tctcccagtc c 21
<210> 140
   <211> 23
   <212> DNA
   <213> Artificial
<220>
   <223> Primer
<400> 140
   tcacaatacg tggtacacag gtc 23
<210> 141
   <211> 21
   <212> DNA
   <213> Artificial
<220>
   <223> Primer
<400> 141
   atgtcggagc aggaggctct g 21
<210> 142
   <211> 19
   <212> DNA
   <213> Artificial
<220>
   <223> Primer
<400> 142
   tcagtccagc taatggggc 19
<210> 143
   <211> 20
   <212> DNA
   <213> Artificial
<220>
   <223> Primer
<400> 143
   tggtaggggt caggggggac 20
<210> 144
   <211> 21
   <212> DNA
   <213> Artificial
<220>
   <223> Primer
<400> 144
   ggccatcgtc aggataaatt c 21
<210> 145
   <211> 19
   <212> DNA
   <213> Artificial
<220>
   <223> Primer
<400> 145
   ttccacaaca agatgccgg 19
<210> 146
   <211> 19
   <212> DNA
   <213> Artificial
<220>
   <223> Primer
<400> 146
   tgacagccag gtgtcttcc 19
<210> 147
   <211> 20
   <212> DNA
   <213> Artificial
<220>
   <223> Primer
<400> 147
   ggaagaacac ctggctgtca 20
<210> 148
   <211> 20
   <212> DNA
   <213> Artificial
<220>
   <223> Primer
<400> 148
   tcctggatgt gtcctcgtct 20
<210> 149
   <211> 19
   <212> DNA
   <213> Artificial
<220>
   <223> Primer
<400> 149
   agcatcaggc atgtccatc 19
<210> 150
   <211> 19
   <212> DNA
   <213> Artificial
<220>
   <223> Primer
<400> 150
   gccagtatag cggacctca 19
<210> 151
   <211> 21
   <212> DNA
   <213> Artificial
<220>
   <223> Primer
<400> 151
   acgagcctgc gcaatggaac a 21
<210> 152
   <211> 25
   <212> DNA
   <213> Artificial
<220>
   <223> Primer
<400> 152
   ccccagcact ttaactctca gcttt 25
<210> 153
   <211> 20
   <212> DNA
   <213> Artificial
<220>
   <223> Primer
<400> 153
   cgggggtcgc ctggtctttg 20
<210> 154
   <211> 20
   <212> DNA
   <213> Artificial
<220>
   <223> Primer
<400> 154
   gggggagttg gggtgctggt 20
<210> 155
   <211> 21
   <212> DNA
   <213> Artificial
<220>
   <223> Primer
<400> 155
   ccctcctgac ctctctggcc c 21
<210> 156
   <211> 19
   <212> DNA
   <213> Artificial
<220>
   <223> Primer
<400> 156
   ctggcagggg gtcccacca 19
<210> 157
   <211> 19
   <212> DNA
   <213> Artificial
<220>
   <223> Primer
<400> 157
   tgtcggtggc ttcgtgggc 19
<210> 158
   <211> 20
   <212> DNA
   <213> Artificial
<220>
   <223> Primer
<400> 158
   tctgggctga ggcgagggtg 20
<210> 159
   <211> 20
   <212> DNA
   <213> Artificial
<220>
   <223> Primer
<400> 159
   agctgcggtt tgcttctgct 20
<210> 160
   <211> 20
   <212> DNA
   <213> Artificial
<220>
   <223> Primer
<400> 160
   actggctgca gttggggtcc 20
<210> 161
   <211> 20
   <212> DNA
   <213> Artificial
<220>
   <223> Primer
<400> 161
   gctatagcgc cgccctgctg 20
<210> 162
   <211> 20
   <212> DNA
   <213> Artificial
<220>
   <223> Primer
<400> 162
   accagcactt tccccgctgc 20
<210> 163
   <211> 20
   <212> DNA
   <213> Artificial
<220>
   <223> Primer
<400> 163
   ggctgaccgg gtcctttggc 20
<210> 164
   <211> 20
   <212> DNA
   <213> Artificial
<220>
   <223> Primer
<400> 164
   ccgggaacct gggagccgta 20
<210> 165
   <211> 21
   <212> DNA
   <213> Artificial
<220>
   <223> Primer
<400> 165
   agcgagtcgg gttgtttgggc 21
<210> 166
   <211> 22
   <212> DNA
   <213> Artificial
<220>
   <223> Primer
<400> 166
   gctctccggg tctttgcact gg 22
<210> 167
   <211> 22
   <212> DNA
   <213> Artificial
<220>
   <223> Primer
<400> 167
   ccggctaagc accaagcaaa gc 22
<210> 168
   <211> 23
   <212> DNA
   <213> Artificial
<220>
   <223> Primer
<400> 168
   tccctggttt ctcctgtcga ggc 23
<210> 169
   <211> 24
   <212> DNA
   <213> Artificial
<220>
   <223> Primer
<400> 169
   atgttagtgt ttttcgctgt ggtc 24
<210> 170
   <211> 28
   <212> DNA
   <213> Artificial
<220>
   <223> Primer
<400> 170
   gaattcacag ctcatcatcc ttggccac 28